# EUROPEAN PATENT APPLICATION

(11) **EP 4 431 505 A1**
(43) Date of publication of application: **18.09.2024**
(21) Application number: 22893205.9
(22) Date of filing: 09.11.2022
(51) Int. Cl.: C07D 401/14, A61K 31/454, A61K 31/506, A61P 35/00, A61P 37/06, A61P 29/00, C07D 413/14, C07D 417/14, C07D 401/04

(54) **ISOINDOLINONE DERIVATIVE HAVING GLUTARIMIDE MOTHER NUCLEUS, AND USE THEREOF**

(30) Priority: 09.11.2021 KR 20210153439; 08.11.2022 KR 20220148244
(71) Applicant: Korea Research Institute of Chemical Technology, Daejeon 34114 (KR)
(72) Inventor: HWANG, Jong Yeon, Daejeon 34114 (KR); CHO, Sung Yun, Daejeon 34114 (KR); HA, Jae Du, Daejeon 34114 (KR); KIM, Pil Ho, Daejeon 34114 (KR); PARK, Chi Hoon, Daejeon 34114 (KR); KIM, Hyun Jin, Daejeon 34114 (KR); CHO, Yong Hee, Daejeon 34114 (KR); AKSHAY, Diw, Daejeon 34114 (KR)
(74) Representative: Kraus & Lederer PartGmbB
(86) International application number: PCT/KR2022/017592
(87) International publication number: WO 2023/085785

(57) **Abstract**

The present invention relates to an isoindolinone derivative compound having a glutarimide mother nucleus, and the application thereof and, more specifically, to an isoindolinone derivative compound having a glutarimide mother nucleus having a thalidomide analog. The compound of chemical formula 1 according to the present invention specifically binds to the CRBN protein and is involved in functions thereof. Thus, the compound of the present invention may be beneficially used in the prevention or treatment of leprosy, chronic graft versus host disease, inflammatory diseases, or cancer that are caused by actions of the CRBN protein.

## Description

### Technical Field

The present disclosure relates to an isoindolinone derivative with a glutarimide scaffold and a use thereof and, more specifically, to an isoindolinone derivative compound with a glutarimide scaffold, which exhibits preventive or therapeutic effects on leprosy, chronic graft-versus-host disease, inflammatory diseases, or cancer.

### Background Art

Thalidomide, sold under the brand name THALOMID^{®} and chemically known as α-(N-phthalimido)glutarimide or 2-(2,6-dioxo-3-piperidinyl)-1H-isoindol-1,3(2H)-dione, is a racemic compound originally developed to treat morning sickness but was discontinued due to teratogenic effects. Thalidomide has been approved in the United States for the treatment of erythema nodosum leprosum (Korean Patent No. 10-0671366).

Moreover, thalidomide has been reported to be used for patients with leprosy, chronic graft-versus-host disease, rheumatoid arthritis, sarcoidosis, certain inflammatory skin diseases, and inflammatory bowel diseases. It has also been reported that thalidomide can be combined with other drugs to treat cardiac and cerebral artery occlusion-related ischemia/reperfusion (U.S. Patent No. 05643915).

More recently, thalidomide has been used in the treatment of specific types of cancer, including refractory multiple myeloma, brain, melanoma, breast, colon, mesothelioma, and renal cell carcinoma. Additionally, it has been reported to prevent the manifestation of chronic cardiomyopathy induced by doxorubicin in rats. Other reports on the use of thalidomide in cancer treatment include its combination with carboplatin for treating glioblastoma multiforme. Thalidomide has also been reported as an analgesic in the treatment of astrocytoma (Costa, P. T. et al., Blood, 92 (Suppl 1, Pt 2), 235b, 1998; Marx, G. M. et al., Proc Am Soc Clin Oncol., 454a, 1999; Singhal, S. et al., N Engl J Med., 341(21), 1565-1571, 1999; Zwart, D., Arzneimittelforschung, 16(12), 1688-1689, 1966).

Furthermore, thalidomide has been diversely utilized for the prevention or treatment of lupus nephritis, fibromyalgia, schizophrenia, central nervous system disorders, diabetes, inflammatory diseases, etc. However, it was withdrawn from the market in late 1961 due to the fatal side effect of causing birth defects in pregnant women who took the drug.

Active research is underway to develop derivatives that retain the various physiological utilities of thalidomide while resolving the issue of serious side effects.

Protein degradation within cells primarily occurs through the ubiquitin-proteasome system (UPS). Proteins known as E1, E2, and E3 ligases transfer ubiquitin, consisting of 76 amino acids, to the substrate protein to be degraded, promoting polyubiquitination. This signals the 26S proteasome to recognize and degrade the protein.

Derivatives of thalidomide, known as immunomodulatory drugs (IMiDs), such as lenalidomide and pomalidomide, bind to an E3 ligase called cereblon (CRBN), inducing the degradation of the zinc finger transcription factors ikaros (IKZF1) and aiolos (IKZF3), and are used as treatments for multiple myeloma. Lenalidomide, in particular, degrades CK-1a, being also used as a treatment for 5q-del-MDS patients, and is one of the world's best-selling cancer drugs.

GSPT1, which functions as a small GTPase, forms a complex with eRF1 in a GTP-dependent manner to bind to the mRNA's stop codon. The change from GTP to GDP separates GSPT1 from eRF1, facilitating eRF1-mediated protein cleavage (Cell Reports, 2014, 8, 59-65).

Knockdown of GSPT1 reduces phosphorylation of 4E-BP1 and kinase S6K1, inhibiting mTOR activity and inducing G1 arrest (Molecular and cellular biology, 2007, 27, 5619).

GSPT1 was found to be overexpressed in colon cancer cell lines (HCT116) and involved in cell growth and migration. The knockdown of GAPT1 suppresses the expression of c-myc, survivin, and Bcl2L15, inducing cell apoptosis (Biomed. Pharmacother. 2015, 74, 138-144) .

Additionally, Celgene Corporation reported that the CC-885 compound, derived from lenalidomide, degrades the new protein GSPT1, demonstrating high cytotoxicity in various blood cancer cells. Optimization studies led to the development of CC-90009, which is currently undergoing phase 1 clinical trials for R,R-AML patients (Nature, 2016, 14, 252; Blood, 2021).

While retaining the physiological activities exhibited by thalidomide, new thalidomide derivatives based on piperidin-2,6-dione have been developed without the side effects associated with thalidomide. These compounds were found to promote the degradation of the GSPT1 protein and Aiolos. Moreover, the compounds exhibit significant cytotoxicity against cancer cells. Particularly, the urea derivative and triazine derivative compounds showed enhanced cytotoxic activity against cancer cells (Korean Patent No. 10-2020-0054046 A) .

Additionally, 5-substituted isoindoline compounds have been reported to exhibit anti-proliferative effects on various cancer cells, including prostate, colon, pancreatic, and breast tumors, in addition to inhibiting TNF-α and promoting IL-2 production (Korean Patent No. 10-2011-0019761 A). Isoindoline compounds have been reported to exhibit effects on various diseases, including cancer, through the regulation of angiogenesis, inhibition of specific cytokines such as TNF-α, and stimulation of certain cytokines like IL-10 production (Korean Patent No. 10-1696938).

A method for predicting and treating various diseases, including cancer, by administering compounds containing isoindolinone and glutarimide has also been reported (Korean Patent No. 10-2018-0095094 A). Compounds with an amino amide linker have been reported to act as modulators of various protein activities, including cytokines, TNF-α, GSPT1, etc., showing effectiveness in treating various diseases such as inflammatory diseases and cancer (Australian Patent No. 2019284608 A).

Leading to the present disclosure, thorough and intensive research conducted by the present inventors resulted in the finding that the compound, represented by Chemical Formula 1, bearing a glutarimide moiety and having an isoindolinone structure, show superior activity when an aryl substituent is present on the heteroaryl and particularly, the compounds exhibit outstanding biological activity against cancer cells when the heteroaryl is in a bicyclic form or when the monocyclic heteroaryl is substituted with an aryl group, compared to when the heteroaryl is in a monocyclic form.

### Disclosure of Invention

### Technical Problem

The present inventors have completed the present disclosure by developing isoindolinone derivative compounds with a gluarimide scaffold and evaluating the compounds for prophylactic or therapeutic effects on leprosy, chronic graft-versus-host disease, inflammatory diseases, or cancer.

Accordingly, the present disclosure is to provide a compound represented by the following Chemical Formula 1 and a pharmaceutically acceptable salt thereof.

### Solution to Problem

The present disclosure pertains to a compound represented by the following Chemical Formula 1, an optical isomer thereof, or a pharmaceutically acceptable salt thereof: 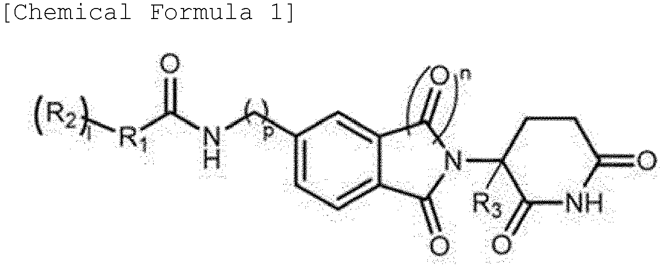 wherein,
l is an integer of 0 to 3;
n is an integer of 0 or 1;
p is an integer of 1 or 2;
R₁ is a C₅₋₁₃ heteroaryl, a C₆₋₁₀ aryl, a C₄₋₁₀ cycloalkyl, or a C₄₋₁₀ heterocycloalkyl;
R₂ is independently at least one substituent selected from the group consisting of a hydrogen atom, halogen, ketone, a C₁₋₅ alkyl, a C₁₋₃ alkoxy, a halo C₁₋₃ alkyl, a halo C₁₋₃ alkoxy, a C₃₋₆ cycloalkyl, a substituted or unsubstituted phenyl, and a substituted or unsubstituted benzyl,
the substituted phenyl or benzyl having as a substituent at least one selected from the group consisting of a halogen or a C₁₋₅ alkyl; and
R₃ is a hydrogen atom or a deuterium atom (D).

A particular embodiment of the present disclosure pertains to a compound, an optical isomer thereof, or a pharmaceutically acceptable salt thereof, the compound being represented by Chemical Formula 1 wherein,
l an integer of 0 to 3;
n is an integer of 0 or 1;
p is an integer of 1 or 2;
R₁ is a substituent selected from the group consisting of pyrrolyl, pyrazolyl, imidazolyl, isoxazolyl, triazolyl, pyridinyl, pyrazinyl, pyrimidinyl, indolyl, indazolyl, benzoimidazolyl, benzofuranyl, benzopyridinyl, benzopyranyl, benzopyridazinyl, benzoisoxazolyl, benzoxazolyl, benzothiazolyl, benzothiophenyl, and naphthofuranyl;
R₂ is independently at least one substituent selected from the group consisting of a hydrogen atom, halogen, ketone, a C₁₋₅ alkyl, a substituted or unsubstituted phenyl, a substituted or unsubstituted benzyl, C₁₋₃ alkoxy, a halo C₁₋₃ alkyl, and a halo C₁₋₃ alkoxy,
the substituent phenyl or benzyl having as a substituent at least one selected from the group consisting of halogen or a C₁₋₅ alkyl; and
R₃ is a hydrogen atom or a deuterium atom (D).

A particular embodiment of the present disclosure pertains to a compound, an optical isomer thereof, or a pharmaceutically acceptable salt thereof, the compound being represented by Chemical Formula 1 wherein,
l is an integer of 0 to 3;
n is an integer of 0 or 1;
p is an integer of 1 or 2;
R₁ is a C₅₋₁₀ heteroaryl;
R₂ is at least one substituent selected from the group consisting of a substituted or unsubstituted phenyl and a substituted or unsubstituted benzyl,
the substituted phenyl or benzyl having as a substituent at least one selected from the group consisting of halogen and a C₁₋₅ alkyl:
R₃ is a hydrogen atom or a deuterium atom (D).

Also, a particular embodiment of the present disclosure pertains to a compound, an optical isomer thereof, or a pharmaceutically acceptable salt thereof, the compound being represented by Chemical Formula 1 wherein,
l is an integer of 0 to 3;
n is an integer of 0 or 1;
p is an integer of 1 or 2;
R₁ is a substituent selected from the group consisting of pyrrolyl, pyrazolyl, imidazolyl, isoxazolyl, triazolyl, pyridinyl, pyrazinyl, pyrimidinyl, indolyl, indazolyl, benzoimidazolyl, benzofuranyl, benzopyridinyl, benzopyranyl, benzopyridazinyl, benzoisoxazolyl, benzoxazolyl, benzothiazolyl, benzothiophenyl, and naphthofuranyl:
R₂ is at least one substituent selected from the group consisting of a substituted or unsubstituted phenyl and a substituted or unsubstituted benzyl,
the substituted phenyl or benzyl having as a substituent at least one selected from the group consisting of halogen or a C₁₋₅ alkyl: and
R₃ is a hydrogen atom or a deuterium atom (D).

Concrete examples of the compound represented by Chemical Formula 1 include:
6-chloro-N-((2-(2,6-dioxopiperidin-3-yl)-1-oxoisoindolin-5-yl)methyl)-1H-indazole-3-carboxamide (Compound 1);
3-(4-chlorophenyl)-N-((2-(2,6-dioxopiperidin-3-yl)-1-oxoisoindolin-5-yl)methyl)-1H-pyrazole-5-carboxamide (Compound 2);
5-chloro-N-((2-(2,6-dioxopiperidin-3-yl)-1-oxoisoindolin-5-yl)methyl)-1H-benzo[d]imidazole-2-carboxamide (Compound 3);
5,6-dichloro-N-((2-(2,6-dioxopiperidin-3-yl)-1-oxoisoindolin-5-yl)methyl)-1H-indazole-3-carboxamide (Compound 4);
3-(3-chlorophenyl)-N-((2-(2,6-dioxopiperidin-3-yl)-1-oxoisoindolin-5-yl)methyl)-1H-pyrazole-5-carboxamide (Compound 5);
6-chloro-N-((2-(2,6-dioxopiperidin-3-yl)-1-oxoisoindolin-5-yl)methyl)-1H-indole-3-carboxamide (Compound 6);
5-chloro-N-((2-(2,6-dioxopiperidin-3-yl)-1-oxoisoindolin-5-yl)methyl)-1H-indole-3-carboxamide (Compound 7);
5-chloro-N-((2-(2,6-dioxopiperidin-3-yl)-1-oxoisoindolin-5-yl)methyl)-1H-indazole-3-carboxamide (Compound 8);
6-chloro-N-((2-(2,6-dioxopiperidin-3-yl)-1-oxoisoindolin-5-yl)methyl)-1H-indole-2-carboxamide (Compound 9);
6-chloro-N-((2-(2,6-dioxopiperidin-3-yl)-1-oxoisoindolin-5-yl)methyl)benzo[d]isoxazole-3-carboxamide (Compound 10);
5-(4-chlorophenyl)-N-((2-(2,6-dioxopiperidin-3-yl)-1-oxoisoindolin-5-yl)methyl)isoxazole-3-carboxamide (Compound 11);
N-((2-(2,6-dioxopiperidin-3-yl)-1-oxoisoindolin-5-yl)methyl)-4-phenylpicolinamide (Compound 12);
3-(3,4-dichlorophenyl)-N-((2-(2,6-dioxopiperidin-3-yl)-1-oxoisoindolin-5-yl)methyl)-1H-pyrazole-5-carboxamide (Compound 13);
4-(3-chlorophenyl)-N-((2-(2,6-dioxopiperidin-3-yl)-1-oxoisoindolin-5-yl)methyl)-1H-pyrrole-2-carboxamide (Compound 14);
1-(3-chlorophenyl)-N-((2-(2,6-dioxopiperidin-3-yl)-1-oxoisoindolin-5-yl)methyl)-1H-1,2,3-triazole-4-carboxamide (Compound 15);
5,6-dichloro-N-((2-(2,6-dioxopiperidin-3-yl)-1-oxoisoindolin-5-yl)methyl)-1H-benzo[d]imidazole-2-carboxamide (Compound 16);
N-((2-(2,6-dioxopiperidin-3-yl)-1-oxoisoindolin-5-yl)methyl)-4-methyl-5-phenyl-1H-pyrazole-3-carboxamide (Compound 17);
4-(4-chlorophenyl)-N-((2-(2,6-dioxopiperidin-3-yl)-1-oxoisoindolin-5-yl)methyl)picolinamide (Compound 18) ;
5-(3-chlorophenyl)-N-((2-(2,6-dioxopiperidin-3-yl)-1-oxoisoindolin-5-yl)methyl)isoxazole-3-carboxamide (Compound 19);
5-(3-chlorophenyl)-N-((2-(2,6-dioxopiperidin-3-yl)-1-oxoisoindolin-5-yl)methyl)pyrimidine-2-carboxamide (Compound 20);
1-benzyl-5-(3-chlorophenyl)-N-((2-(2, 6-dioxopiperidin-3-yl)-1-oxoisoindolin-5-yl)methyl)-4-methyl-1H-pyrazole-3-carboxamide (Compound 21);
4-(3-chlorophenyl)-N-((2-(2,6-dioxopiperidin-3-yl)-1-oxoisoindolin-5-yl)methyl)picolinamide (Compound 22);
N-((2-(2,6-dioxopiperidin-3-yl)-1-oxoisoindolin-5-yl)methyl)-1-methyl-5-phenyl-1H-pyrazole-3-carboxamide (Compound 23);
N-((2-(2,6-dioxopiperidin-3-yl)-1-oxoisoindolin-5-yl)methyl)-1-methyl-3-phenyl-1H-pyrazole-5-carboxamide (Compound 24) ;
5-(2,4-dichlorophenyl)-N-((2-(2,6-dioxopiperidin-3-yl)-1-oxoisoindolin-5-yl)methyl)-1H-pyrazole-3-carboxamide (Compound 25);
6-chloro-N-((2-(2,6-dioxopiperidin-3-yl)-1-oxoisoindolin-5-yl)methyl)benzo[d]thiazole-2-carboxamide (Compound 26);
4-bromo-3-(3-chlorophenyl)-N-((2-(2,6-dioxopiperidin-3-yl)-1-oxoisoindolin-5-yl)methyl)-1H-pyrazole-5-carboxamide (Compound 27);
5-(2,5-dichlorophenyl)-N-((2-(2,6-dioxopiperidin-3-yl)-1-oxoisoindolin-5-yl)methyl)-1H-pyrazole-3-carboxamide (Compound 28);
5-(2,4-dimethylphenyl)-N-((2-(2,6-dioxopiperidin-3-yl)-1-oxoisoindolin-5-yl)methyl)-1H-pyrazole-3-carboxamide (Compound 29);
N-((2-(2,6-dioxopiperidin-3-yl)-1-oxoisoindolin-5-yl)methyl)-[1,1'-biphenyl]-3-carboxamide (Compound 30);
N-((2-(2,6-dioxopiperidin-3-yl)-1-oxoisoindolin-5-yl)methyl)-4-methylpicolinamide (Compound 31);
N-((2-(2,6-dioxopiperidin-3-yl)-1-oxoisoindolin-5-yl)methyl)-4-phenylpicolinamide (Compound 32);
6-chloro-N-((2-(2,6-dioxopiperidin-3-yl)-1-oxoisoindolin-5-yl)methyl)benzo[d]oxazole-2-carboxamide (Compound 33);
N-((2-(2,6-dioxopiperidin-3-yl)-1-oxoisoindolin-5-yl)methyl)-1H-indazole-3-carboxamide (Compound 34);
3-bromo-N-((2-(2,6-dioxopiperidin-3-yl)-1-oxoisoindolin-5-yl)methyl)-1-methyl-1H-pyrazole-5-carboxamide (Compound 35);
N-((2-(2,6-dioxopiperidin-3-yl)-1-oxoisoindolin-5-yl)methyl)-1-methyl-1H-indazole-3-carboxamide (Compound 36);
4-bromo-N-((2-(2,6-dioxopiperidin-3-yl)-1-oxoisoindolin-5-yl)methyl)-1-methyl-1H-imidazole-2-carboxamide (Compound 37);
4-bromo-N-((2-(2,6-dioxopiperidin-3-yl)-1-oxoisoindolin-5-yl)methyl)-1-methyl-1H-pyrrole-2-carboxamide (Compound 38);
N-((2-(2,6-dioxopiperidin-3-yl)-1-oxoisoindolin-5-yl)methyl)-1-methyl-4-phenyl-1H-pyrrole-2-carboxamide (Compound 39);
4-(3-chlorophenyl)-N-((2-(2,6-dioxopiperidin-3-yl)-1-oxoisoindolin-5-yl)methyl)-1-methyl-1H-pyrrole-2-carboxamide (Compound 40);
5-(3-chlorophenyl)-N-((2-(2,6-dioxopiperidin-3-yl)-1-oxoisoindolin-5-yl)methyl)-1-isobutyl-4-methyl-1H-pyrazole-3-carboxamide (Compound 41);
5-(3-chlorophenyl)-N-((2-(2,6-dioxopiperidin-3-yl)-1-oxoisoindolin-5-yl)methyl)-4-methyl-1-phenyl-1H-pyrazole-3-carboxamide (Compound 42);
5-(3-chlorophenyl)-N-((2-(2,6-dioxopiperidin-3-yl)-1-oxoisoindolin-5-yl)methyl)-1-(4-fluorophenyl)-4-methyl-1H-pyrazole-3-carboxamide (Compound 43);
5-chloro-N-((2-(2,6-dioxopiperidin-3-yl)-1-oxoisoindolin-5-yl)methyl)benzo[d]thiazole-2-carboxamide (Compound 44);
N-((2-(2,6-dioxopiperidin-3-yl)-1-oxoisoindolin-5-yl)methyl)-5-methyl-4-phenylpicolinamide (Compound 45);
5-chloro-N-((2-(2,6-dioxopiperidin-3-yl)-1-oxoisoindolin-5-yl)methyl)-4-phenylpicolinamide (Compound 46);
N-((2-(2,6-dioxopiperidin-3-yl)-1-oxoisoindolin-5-yl)methyl)-6-phenylpyrazine-2-carboxamide (Compound 47);
3-(2-chloro-6-fluorophenyl)-N-((2-(2,6-dioxopiperidin-3-yl)-1-oxoisoindolin-5-yl)methyl)-5-methylisoxazole-4-carboxamide (Compound 48);
N-((2-(2,6-dioxopiperidin-3-yl)-1-oxoisoindolin-5-yl)methyl)-3-phenyl-1H-pyrazole-5-carboxamide (Compound 49);
N-((2-(2,6-dioxopiperidin-3-yl)-1-oxoisoindolin-5-yl)methyl)-1-phenyl-5-(trifluoromethyl)-1H-pyrazole-4-carboxamide (Compound 50);
N-((2-(2,6-dioxopiperidin-3-yl)-1-oxoisoindolin-5-yl)methyl)-[1,1'-biphenyl]-4-carboxamide (Compound 51);
N-((2-(2,6-dioxopiperidin-3-yl)-1-oxoisoindolin-5-yl)methyl)-[1,1'-biphenyl]-2-carboxamide (Compound 52);
1-(4-chlorophenyl)-N-((2-(2,6-dioxopiperidin-3-yl)-1-oxoisoindolin-5-yl)methyl)cyclopentane-1-carboxamide (Compound 53);
N-((2-(2,6-dioxopiperidin-3-yl)-1-oxoisoindolin-5-yl)methyl)-1-phenylcyclopentane-1-carboxamide (Compound 54);
N-((2-(2,6-dioxopiperidin-3-yl)-1-oxoisoindolin-5-yl)methyl)-2-oxo-1-phenylpyrrolidine-3-carboxamide (Compound 55);
N-((2-(2,6-dioxopiperidin-3-yl)-1-oxoisoindolin-5-yl)methyl)benzofuran-2-carboxamide (Compound 56);
N-((2-(2,6-dioxopiperidin-3-yl)-1-oxoisoindolin-5-yl)methyl)-5-methoxy-1H-indole-2-carboxamide (Compound 57);
N-((2-(2,6-dioxopiperidin-3-yl)-1-oxoisoindolin-5-yl)methyl)benzo[b]thiophene-2-carboxamide (Compound 58);
7-chloro-N-((2-(2,6-dioxopiperidin-3-yl)-1-oxoisoindolin-5-yl)methyl)-4-hydroxyquinoline-3-carboxamide (Compound 59);
N-((2-(2,6-dioxopiperidin-3-yl)-1-oxoisoindolin-5-yl)methyl)-2-oxo-2H-cromene-3-carboxamide (Compound 60);
N-((2-(2,6-dioxopiperidin-3-yl)-1-oxoisoindolin-5-yl)methyl)cinnoline-4-carboxamide (Compound 61);
5-(4-chlorophenyl)-N-((2-(2,6-dioxopiperidin-3-yl)-1-oxoisoindolin-5-yl)methyl)-1-isobutyl-4-methyl-1H-pyrazole-3 -carboxamide (Compound 62);
5-(3-chlorophenyl)-1-cyclopropyl-N-((2-(2,6-dioxopiperidin-3-yl)-1-oxoisoindolin-5-yl)methyl)-4-methyl-1H-pyrazole-3 -carboxamide (Compound 63);
5-(3-chlorophenyl)-N-((2-(2,6-dioxopiperidin-3-yl)-1-oxoisoindolin-5-yl)methyl)-4-methyl-1-(2,2,2-trifluoroethyl)-1H-pyrazole-3-carboxamide (Compound 64);
N-((2-(2,6-dioxopiperidin-3-yl)-1-oxoisoindolin-5-yl)methyl)-5-methoxybenzo[b]thiophene-2-carboxamide (Compound 65);
N-((2-(2,6-dioxopiperidin-3-yl)-1-oxoisoindolin-5-yl)methyl)-6-methylbenzo[b]thiophene-2-carboxamide (Compound 66);
3-chloro-N-((2-(2,6-dioxopiperidin-3-yl)-1-oxoisoindolin-5-yl)methyl)-6-methylbenzo[b]thiophene-2-carboxamide (Compound 67);
3-chloro-N-((2-(2,6-dioxopiperidin-3-yl)-1-oxoisoindolin-5-yl)methyl)-6-methoxybenzo[b]thiophene-2-carboxamide (Compound 68);
N-((2-(2,6-dioxopiperidin-3-yl)-1-oxoisoindolin-5-yl)methyl)-5,6-dimethoxybenzo[b]thiophene-2-carboxamide (Compound 69);
N-((2-(2,6-dioxopiperidin-3-yl)-1-oxoisoindolin-5-yl)methyl)-4-(trifluoromethyl)benzo[b]thiophene-2-carboxamide (Compound 70);
N-((2-(2,6-dioxopiperidin-3-yl)-1-oxoisoindolin-5-yl)methyl)-7-(trifluoromethyl)benzo[b]thiophene-2-carboxamide (Compound 71);
3-chloro-N-((2-(2,6-dioxopiperidin-3-yl)-1-oxoisoindolin-5-yl)methyl)-6-(trifluoromethyl)benzo[b]thiophene-2-carboxamide (Compound 72);
N-((2-(2,6-dioxopiperidin-3-yl)-1-oxoisoindolin-5-yl)methyl)-5-(trifluoromethyl)benzo[b]thiophene-2-carboxamide (Compound 73);
N-((2-(2,6-dioxopiperidin-3-yl)-1-oxoisoindolin-5-yl)methyl)-4-methoxybenzo[b]thiophene-2-carboxamide (Compound 74);
5-chloro-N-((2-(2,6-dioxopiperidin-3-yl)-1-oxoisoindolin-5-yl)methyl)-3-methylbenzo[b]thiophene-2-carboxamide (Compound 75);
N-((2-(2,6-dioxopiperidin-3-yl)-1-oxoisoindolin-5-yl)methyl)-3-methylbenzo[b]thiophene-2-carboxamide (Compound 76);
4-chloro-N-((2-(2,6-dioxopiperidin-3-yl)-1-oxoisoindolin-5-yl)methyl)-3-methylbenzo[b]thiophene-2-carboxamide (Compound 77);
7-chloro-N-((2-(2,6-dioxopiperidin-3-yl)-1-oxoisoindolin-5-yl)methyl)-3-methylbenzo[b]thiophene-2-carboxamide (Compound 78);
N-((2-(2,6-dioxopiperidin-3-yl)-1-oxoisoindolin-5-yl)methyl)-4-fluoro-3-methylbenzo[b]thiophene-2-carboxamide (Compound 79);
N-((2-(2,6-dioxopiperidin-3-yl)-1-oxoisoindolin-5-yl)methyl)-3-methylbenzofuran-2-carboxamide (Compound 80);
N-((2-(2,6-dioxopiperidin-3-yl)-1-oxoisoindolin-5-yl)methyl)-5-methoxybenzofuran-2-carboxamide (Compound 81);
N-((2-(2,6-dioxopiperidin-3-yl)-1-oxoisoindolin-5-yl)methyl)-7-methoxybenzofuran-2-carboxamide (Compound 82);
N-((2-(2,6-dioxopiperidin-3-yl)-1-oxoisoindolin-5-yl)methyl)-6-methoxybenzofuran-2-carboxamide (Compound 83);
N-((2-(2,6-dioxopiperidin-3-yl)-1-oxoisoindolin-5-yl)methyl)-3,6-dimethylbenzofuran-2-carboxamide (Compound 84);
5-bromo-N-((2-(2,6-dioxopiperidin-3-yl)-1-oxoisoindolin-5-yl)methyl)-3-methylbenzofuran-2-carboxamide (Compound 85); and
N-((2-(2,6-dioxopiperidin-3-yl)-1-oxoisoindolin-5-yl)methyl)naphtho[2,1-b]furan-2-carboxamide (Compound 86) .

Furthermore, the compound represented by Chemical Formula 1 is synthesized in the manner illustrated by the following Reaction Schemes 1 and 2:

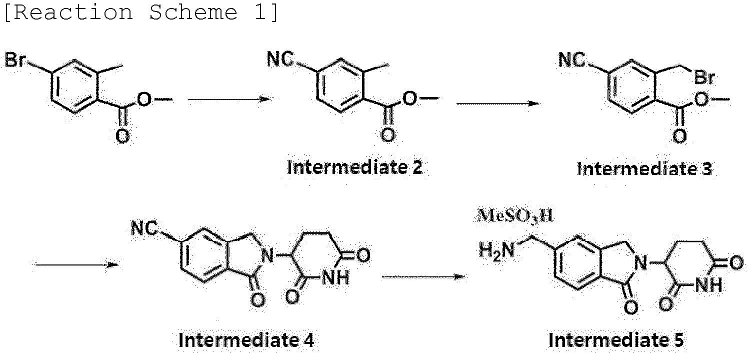

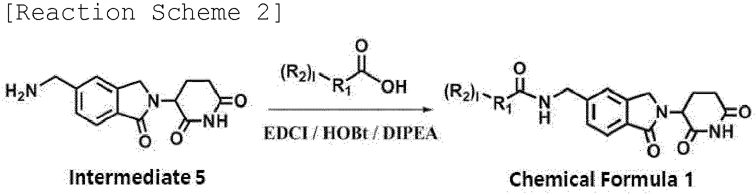

In Reaction Scheme 1, Intermediates 2 to 5 are prepared as follows.

### 1) Synthesis of methyl 4-cyano-2-methylbenzoate (Intermediate 2)

A solution of methyl 4-bromo-2-methylbenzoate (3 g, 13.10 mmol) in DMF (40 ml) was added with Pd₂(dba)₃ (0.480 g, 0.524 mmol), DPPF (0.436 g, 0.786 mmol), and zinc cyanide (1.692 g, 14.41 mmol) and stirred for 30 minutes in a nitrogen atmosphere. Then, the temperature was elevated to 130°C, followed by stirring for 3 hours. After completion of the reaction, the mixture was filtered through celite and washed many times with DMF. The filtrate was subjected to extraction with EtOAc (50 ml x 2) and washed with water and brine. The organic layer thus pooled was dried with magnesium sulfate anhydrous and concentrated by removing the solvent at a reduced pressure. The concentrate was purified by column chromatography (hexane: EtOAc = 9:1) to afford Intermediate 2 as a bright yellow solid at a yield of 85% (1.96 g, 11.19 mmol, 85%).

### 2) Synthesis of 2-(bromomethyl)-4-cyanobenzoate (Intermediate 3)

A solution of Intermediate 2 (1.96 g, 11.19 mmol) in DCE (30 ml) was added with N-bromosuccinimide (2.64 g, 22.38 mmol) and bezoyl peroxide (0.271 g, 1.119 mmol) and stirred at an elevated temperature for 8 hours under reflux. After completion of the reaction, the temperature was lowered to room temperature. The solvent was removed at a reduced pressure to concentrate the reaction mixture. Purification by column chromatography (hexane:EtOAc = 9:1) to afford Intermediate 3 as a yellow solid at a yield of 81% (2.29 g, 9.03 mmol, 81%).

### 3) Synthesis of -(2,6-dioxopiperidin-3-yl)-1-oxoisoindoline-5-carbonitrile (Intermediate 4)

A solution of Intermediate 3 (7.34 g, 28.9 mmol) in DMF (50 ml) was added with 3-aminopiperidine-2,6-dione hydrochloride (4.75 g, 28.9 mmol) and TEA (12.07 ml, 87 mmol) at room temperature and stirred overnight at 80°C. After completion of the reaction, the temperature was lowered to room temperature and concentration at a reduced pressure removed the solvent. Water was added to the concentrate and the solid thus formed was washed with an excess of water. Complete dehydration at reduced pressure afforded Intermediate 4 as a dark blue solid at a yield of 81% (6.28 g, 23.32 mmol, 81%).

### 4) Synthesis of 3-(5-(aminomethyl)-1-oxoisoindolin-2-yl)piperidine-2,6-dione (Intermediate 5)

A solution of Intermediate 4 (1.7 g, 6.31 mmol) in DMA (20 ml) was added with 10% Pd/C (2.32 g, 6.31 mmol) and methane sulfonate (0.451 ml, 6.94 mmol) and stirred overnight at 40°C in a hydrogen atmosphere. After completion of the reaction, the reaction mixture was filtered through celite and washed with 50 ml of DMA. The filtrate was concentrated at a reduced pressure and precipitated in a methanol/ethyl ether (5 ml/30 ml) mixed solvent. The precipitate thus formed was filtered to afford Intermediate 5 as a gray solid at a yield of 43% (0.99 g, 2.69 mmol, 43%).

¹H NMR (500 MHz, DMSO-*d*₆) δ 11.00 (s, 1H), 8.27 (s, 3H), 7.79 (d, *J* = 7.8 Hz, 1H), 7.70 (s, 1H), 7.60 (d, *J* = 7.9 Hz, 1H), 5.12 (dd, *J* = 13.3, 5.1 Hz, 1H), 4.49 (d, *J* = 17.4 Hz, 1H), 4.35 (d, *J* = 17.4 Hz, 1H), 4.17 (q, *J* = 5.9 Hz, 2H), 2.97-2.87 (m, 1H), 2.60 (d, *J* = 17.4 Hz, 1H), 2.45-2.38 (m, 1H), 2.34 (s, 6H), 2.06-1.97 (m, 1H).

In addition, Reaction Scheme 2 was carried out in the manner disclosed in Korean Patent No. 10-2011-0019761 A.

The preparation methods for the compound represented by Chemical Formula 1 according to the present disclosure should be understood as examples of a method for preparing the compound in the present disclosure. Any method capable of preparing the compound represented by Chemical Formula 1 is included in the present invention without limitation. Moreover, the method presented herein and preparation methods that could be easily altered or modified by a person skilled in the art are also considered to fall within the scope of the present disclosure, and would be obvious to those skilled in the relevant field.

The following terms have the following meanings unless otherwise indicated. Any undefined terms have their art recognized meanings.

As used herein, the term "halogen" or "halo" refers to fluorine (F), chlorine (Cl), bromine (Br), or iodine (I).

As used herein, the term "alkyl" refers to a monovalent, linear, or branched hydrocarbon radical. The term includes, by way of example, methyl, ethyl, propyl, and the like, but is not limited thereto.

As used herein, the term "alkoxy" refers to an oxygen bonded to a monovalent, linear or branched, saturated hydrocarbon. Alkoxy includes, by way of example, methoxy, ethoxy, propoxy, and the like, but is not limited thereto.

As used herein, the term "halogen alkyl" refers to a substituted alkyl radical where at least one hydrogen atom on the alkyl moiety is substituted by a halogen atom. Examples of the halogen alkyl include trifluoromethyl, difluoromethyl, trifluoroethyl, etc., but are not limited thereto.

As used herein, the term "halogen alkoxy" refers to alkyl-O- where at least one hydrogen atom on the alkyl moiety is substituted by a halogen atom, for example, trifluoromethoxy, etc., but with no limitations thereto.

As used herein, the term "heterocycloalkyl" refers to a monovalent cyclic saturated hydrocarbon radical bearing at least heteroatom such as N, O, or S as a ring member. According to the number and type of the heteroatoms and the number of carbon atoms contained in the ring, the heterocycloalkyl includes piperidinyl, morpholinyl, tetrahydrofuranyl, piperazinyl, etc., but is not limited thereto.

As used herein, the term "heteroaryl" refers to an aromatic ring compound bearing at least one heteroatom such as N, O, or S as a ring member. According to the number and type of the heteroatoms and the number of carbon atoms contained in the ring, the heteroaryl includes pyrrolyl, pyrazolyl, imidazolyl, isoxazolyl, triazolyl, pyridinyl, pyrazinyl, pyrimidinyl, indolyl, indazolyl, benzoimidazolyl, benzoisoxazolyl, benzoxazolyl, benzothiazolyl, benzothiophenyl, naphthofuranyl, quinolinyl, isoquinolinyl, quinoxalinyl, etc. but is not limited thereto.

The compounds represented by Chemical Formula 1 of the present disclosure may encompass not only pharmaceutically acceptable salts thereof, but also all salts, hydrates, solvates, and prodrugs thereof that can be prepared using common methods. In addition, the compound of the present disclosure may bear one or more asymmetry carbon atoms and may be present in racemic and optically active forms. All of these compounds and diastereomers fall within the scope of the present disclosure.

As used herein, the term "pharmaceutically acceptable salt" means a salt or complex of Chemical Formula 1 which retains the preferable biological activity. Examples of the salt includes acid addition salts formed by inorganic acids [e.g., hydrochloric acid, hydrobromic acid, sulfuric acid, phosphoric acid, nitric acid, and the like], and salts formed by organic acids such as acetic acid, oxalic acid, tartaric acid, succinic acid, malic acid, fumaric acid, maleic acid, ascorbic acid, benzoic acid, tannic acid, pamoic acid, alginic acid, polyglutamic acid, naphthalene sulfonic acid, naphthalene disulfonic acid, and poly-galacturonic acid, but are not limited thereto. The compound may be also administered as a pharmaceutically acceptable quaternary salt known to those skilled in the art, and in particular, it includes chloride, bromide, iodide, - O-alkyl, toluenesulfonate, methylsulfonate, sulfonate, phosphate, or carboxylate (e.g., benzoate, succinate, acetate, glycolate, maleate, malate, fumarate, citrate, tartrate, ascorbate, cinnamoate, mandeloate and diphenylacetate).

The acid addition salt according to the present disclosure may be prepared by a conventional method, and, for example, it may be prepared by dissolving the derivative of Chemical Formula 1 in an organic solvent, such as methanol, ethanol, acetone, methylene chloride, acetonitrile and the like, adding an organic acid or an inorganic acid, filtering the resulting precipitate, and drying the filtrate, or it may be prepared by distilling a solvent and an acid in excess amount under reduced pressure, then drying, and crystallizing in an organic solvent.

In addition, a pharmaceutically acceptable metal salt may be prepared by using a base. An alkali metal or alkaline earth metal salt is obtained, for example, by dissolving the compound in an alkali metal hydroxide or alkaline earth metal hydroxide solution in excess amount, filtering out the undissolved compound salt, evaporating the filtrate, and drying same. In this regard, a sodium, potassium, or calcium salt is pharmaceutically suitable for preparing a metal salt. In addition, the corresponding salt is obtained by reacting an alkali metal or alkaline earth metal salt with an appropriate silver salt (for example, silver nitrate).

Additionally, the present disclosure pertains to a pharmaceutical composition including the compound represented by Chemical Formula 1 or a pharmacologically acceptable salt thereof as an active ingredient for the prevention or treatment of leprosy, chronic graft-versus-host disease, inflammatory diseases, or cancer.

According to an experimental example of the present disclosure, the compound of the compound binds to cereblon (CRBN) protein to promote the degradation of Ikaros/Aiolos and GSPT1 proteins. The CRBN protein is known to be a type of E3 ubiquitin ligase, which, upon binding with thalidomide and its analogs such as pomalidomide and lenalidomide, attaches ubiquitin to substrate proteins such as Ikaros/Aiolos and GSPT1 proteins.

Examples of the cancer may include, but are not limited to, breast cancer, colon cancer, lung cancer, small cell lung cancer, stomach cancer, liver cancer, blood cancer, bone cancer, pancreatic cancer, skin cancer, head or neck cancer, cutaneous or intraocular melanoma, uterine cancer, ovarian cancer, rectal cancer, perianal cancer, colorectal cancer, breast cancer, fallopian tube carcinoma, endometrial carcinoma, cervical cancer, vaginal cancer, vulvar carcinoma, Hodgkin's disease, esophageal cancer, small intestine cancer, endocrine gland cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvis carcinoma, CNS tumors, primary CNS lymphoma, spinal tumors, brainstem gliomas, and pituitary adenomas.

The pharmaceutical composition according to the present disclosure may be formulated into a suitable form with a pharmaceutically acceptable carrier that is generally used. As used herein, the term "pharmaceutically acceptable" composition refers to a composition that is physiologically acceptable and does not cause gastric disorder, allergic reactions such as gastrointestinal disorder or vertigo, or similar reactions, when administered to humans. In addition, the composition may be formulated into oral dosage forms such as powder, granules, tablets, capsules, suspensions, emulsions, syrups, aerorols, etc., topical agents, suppositories, and sterile injections according to typical methods.

Examples of carriers, excipients, or diluents suitable for use in the composition include lactose, dextrose, sucrose, sorbitol, mannitol, xylitol, erythritol, maltitol, starch, Arabic gum, alginate, gelatin, calcium phosphate, calcium silicate, cellulose, methyl cellulose, microcrystalline cellulose, polyvinyl pyrrolidone, water, methyl paraoxybenzoate, propyl paraoxybenzoate, talc, magnesium stearate, and mineral oils, but are not limited thereto. When formulations are prepared, diluents or excipients such as filters, stabilizers, binders, disintegrants, surfactants, etc. may be employed. Solid formulations suitable for oral administration include tablets, pills, powders, granules, capsules, etc. and may be formulated with at least one excipient, for example, by mixing with starch, microcrystalline cellulose, sucrose, or lactose, low-substituted hydroxypropyl cellulose, hypromellose, etc. In addition to the simple excipient, a lubricant, such as magnesium stearate, talc, etc., may be used. Liquid formulations suitable for oral administration, exemplified by suspensions, peroral solutions, emulsions, syrup, etc., may comprise various excipients, such as wetting agents, sweetening agents, flavors, preservatives, as well as simple diluents such as water, liquid paraffin, etc. As for non-oral formulations, sterile aqueous solutions, non-aqueous solutions, suspensions, emulsions, lyophilized injections, suppositories, etc. may be exemplary. Non-aqueous solutions and suspensions may contain propylene glycol, polyethylene glycol, vegetable oil such as olive oil, and injectable esters such as ethylolate. As the base of the suppositories, witepsol, Macrogol, Tween 61, cacao butter, laurin butter, glycerogelatin and the like may be used. In order to prepare the formulation for parenteral administration, the compound represented by Chemical Formula 1, or a pharmaceutically acceptable salt thereof may be mixed with sterile/sterilized preservatives, stabilizers, wetting agents or emulsifying accelerators, auxiliary agents such as salts and/or buffers for regulating osmotic pressure and the like, and other therapeutically useful substance, to prepare a solution or suspension, and then, may be prepared in an ampoule or a vial unit dosage.

Provided is a pharmaceutical composition containing the compound of Chemical Formula 1 and an excipient. The compound may be preferably used in an amount of 0.001 wt% to 50 wt%, more preferably 0.001 wt% to 40 wt%, and most preferably 0.001 wt% to 30 wt%, based on the total weight of the composition.

The pharmaceutical composition including the compound of Chemical Formula 1 as an active ingredient may be administered to mammals such as rats, livestock, and humans via various routes. All modes of administration may be contemplated, e.g., by oral, rectal or intravenous, muscle, subcutaneous, intrauterine dura or intracerebral injection. The dosage may vary depending on the age, sex, weight of the subject to be treated, the specific disease or pathology to be treated, the severity of the disease or pathology, the time of administration, the route of administration, the absorption, distribution and excretion rate of the drug, the type of other drugs to be used, and the judgment of the prescriber. The dosage determined based on these factors are within the level of those skilled in the art, and the dosage may be, in general, in the range of 0.01 mg/kg/day to 2000 mg/kg/day. The dosage may be from 1 mg/kg/day to 500 mg/kg/day. The administration may be performed once or several times a day. The dosage does not limit the scope of the present disclosure in any way.

### Advantageous Effects of Invention

The compound of Chemical Formula 1 according to the present disclosure binds specifically to CRBN protein and is involved in the function thereof. Therefore, the compounds of the present disclosure can be advantageously used for preventing or treating CRBN protein-related diseases including leprosy, chronic graft versus host disease, inflammatory diseases, or cancer.

### Brief Description of Drawings

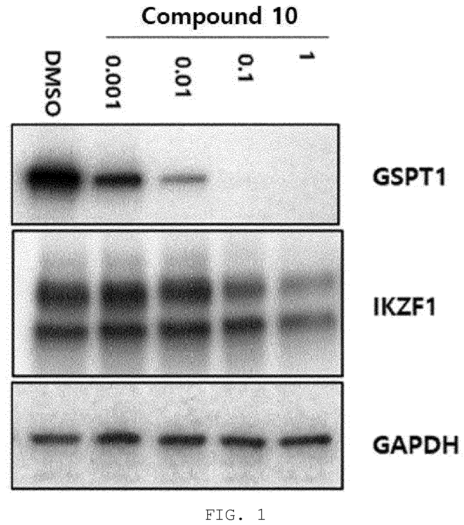

FIG. 1 is an image showing measurements of degradation activity on GSPT1, IKZF1, and GAPDH after treatment with Compound 10 for 6 hours.

### Best Mode for Carrying out the Invention

Hereinafter, preferable embodiments of the present disclosure. However, the present disclosure is not limited to the embodiments described herein but may be embodied in other forms. Rather, these embodiments are provided so that this disclosure will be thorough and complete, and will fully convey the scope of the invention to those skilled in the art.

### <EXAMPLE 1. Synthesis of Isoindolinone Derivative with Glutarimide Scaffold and Physicochemical Characterization thereof >

Compounds 1 to 86 of the present disclosure were synthesized with reference to the method disclosed in Korean Patent No. 10-2011-0019761 A and the physicochemical properties are as follows.

### Compound 1. 6-Chloro-N-((2-(2,6-dioxopiperidin-3-yl)-1-oxoisoindolin-5-yl)methyl)-1H-indazole-3-carboxamide

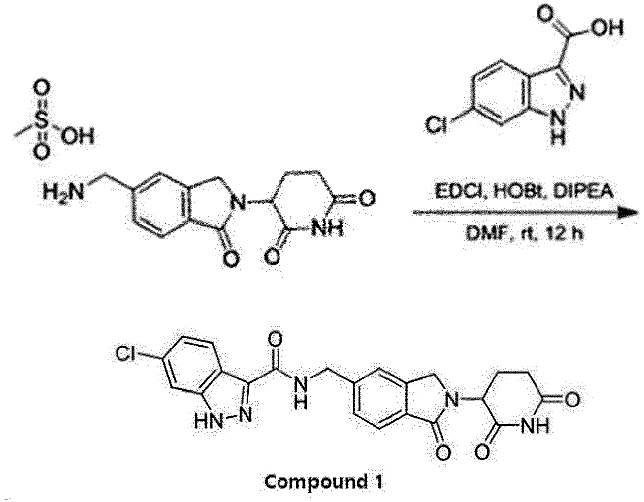

A solution of 3-(5-(aminomethyl)-1-oxoisoindolin-2-yl)piperidine-2,6-dione methanesulfonate (15 mg, 0.00406 mmol) in DMF (1 ml) was added with 6-chloro-1H-indazole-3-carboxylic acid (9.5 mg, 0.0487 mmol), EDCl·HCl (8.5 mg, 0.0446 mmol), HOBt·H₂O (6.0 mg, 0.0446 mmol), and DIPEA (28 *µℓ,* 0.162 mmol) and stirred at room temperature for 12 hours. The reaction mixture was diluted with water and subjected to extraction with ethyl acetate. The organic layer was washed brine, dried with sodium sulfate anhydrous, and filtered. The filtrate was concentrated at a reduced pressure, and isolation and purification by column chromatography afforded Compound 1 as a white solid (5.0 mg, 28%).

¹H NMR (500 MHz, DMSO-d₆) δ 13.76 (s, 1H), 10.98 (s, 1H), 9.18 (t, J = 6.3 Hz, 1H), 8.15 (d, J = 8.6 Hz, 1H), 7.72 (d, J = 1.8 Hz, 1H), 7.69 (d, J = 7.9 Hz, 1H), 7.56 (s, 1H), 7.50 (d, J = 8.1 Hz, 1H), 7.27 (dd, J = 8.7, 1.8 Hz, 1H), 5.10 (dd, J = 13.3, 5.1 Hz, 1H), 4.60 (d, J = 6.3 Hz, 2H), 4.44 (d, J = 17.3 Hz, 1H), 4.30 (d, J = 17.3 Hz, 1H), 2.90 (m, 1H), 2.63 - 2.57 (m, 1H), 2.41 - 2.32 (m, 1H), 2.03 - 1.94 (m, 2H).

### Compound 2. 3-(4-Chlorophenyl)-N-((2-(2,6-dioxopiperidin-3-yl)-1-oxoisoindolin-5-yl)methyl)-1H-pyrazole-5-carboxamide

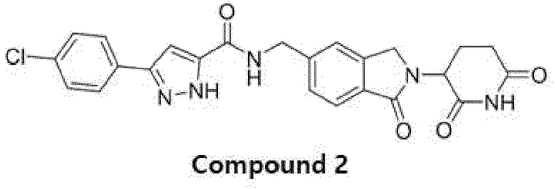

Compound 2 was synthesized in the same manner as in the synthesis method for Compound 1, with the exception of using 3-(4-chlorophenyl)-1H-pyrazole-5-carboxylic acid instead of 6-chloro-1H-indazole-3-carboxylic acid.

¹H NMR (500 MHz, DMSO-d₆) δ 13.75 (s, 1H), 10.98 (s, 1H), 8.99 (s, 1H), 7.82 (d, J = 8.2 Hz, 2H), 7.70 (d, J = 7.8 Hz, 1H), 7.53 (m, 3H), 7.47 (d, J = 7.9 Hz, 1H), 7.20 (s, 1H), 5.10 (dd, J = 13.3, 5.1 Hz, 1H), 4.58 (d, J = 6.1 Hz, 2H), 4.44 (d, J = 17.3 Hz, 1H), 4.31 (d, J = 17.3 Hz, 1H), 2.91 (m, 1H), 2.61 (m, 1H), 2.38 (m, 1H), 2.05 - 1.94 (m, 1H).

### Compound 3. 5-Chloro-N-((2-(2,6-dioxopiperidin-3-yl)-1-oxoisoindolin-5-yl)methyl)-1H-benzo[d]imidazole-2-carboxamide

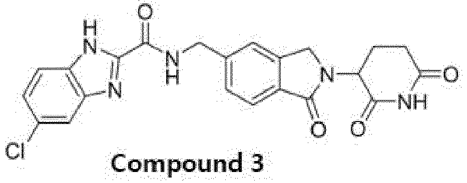

Compound 3 was synthesized in the same manner as in the synthesis method for Compound 1, with the exception of using 5-chloro-1H-benzo[d]imidazole-2-carboxylic acid instead of 6-chloro-1H-indazole-3-carboxylic acid.

¹H NMR (500 MHz, DMSO-d₆) δ 13.46 (s, 1H), 10.98 (s, 1H), 9.71 (t, J = 6.4 Hz, 1H), 7.79 - 7.54 (m, 4H), 7.49 (d, J = 7.9 Hz, 1H), 7.33 (dd, J = 8.7, 2.0 Hz, 1H), 5.10 (dd, J = 13.3, 5.1 Hz, 1H), 4.62 (d, J = 6.4 Hz, 2H), 4.44 (d, J = 17.4 Hz, 1H), 4.31 (d, J = 17.3 Hz, 1H), 2.90 (m, 1H), 2.65 - 2.55 (m, 1H), 2.37 (m, 1H), 1.99 (m, 1H).

### Compound 4. 5,6-Dichloro-N-((2-(2,6-dioxopiperidin-3-yl)-1-oxoisoindolin-5-yl)methyl)-1H-indazole-3-carboxamide

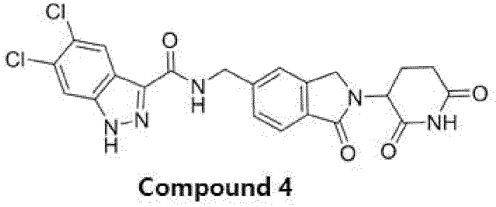

Compound 4 was synthesized in the same manner as in the synthesis method for Compound 1, with the exception of using 5,6-dichloro-1H-indazole-3-carboxylic acid instead of 6-chloro-1H-indazole-3-carboxylic acid.

¹H NMR (500 MHz, DMSO-d₆) δ 10.98 (s, 1H), 9.25 (t, J = 6.3 Hz, 1H), 8.32 (s, 1H), 7.99 (s, 1H), 7.69 (d, J = 7.8 Hz, 1H), 7.56 (s, 1H), 7.50 (d, J = 7.9 Hz, 1H), 5.10 (dd, J = 13.3, 5.1 Hz, 1H), 4.60 (d, J = 6.3 Hz, 2H), 4.44 (d, J = 17.3 Hz, 1H), 4.30 (d, J = 17.3 Hz, 1H), 2.90 (m, 1H), 2.59 (m, 1H), 2.37 (m, 1H), 2.03 - 1.97 (m, 1H).

### Compound 5. 3-(3-Chlorophenyl)-N-((2-(2,6-dioxopiperidin-3-yl)-1-oxoisoindolin-5-yl)methyl)-1H-pyrazole-5-carboxamide

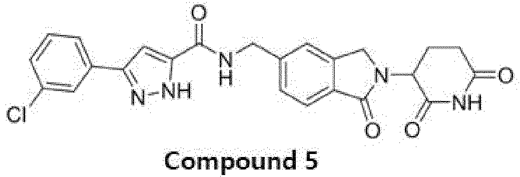

Compound 5 was synthesized in the same manner as in the synthesis method for Compound 1, with the exception of using 5-(3-chlorophenyl)-1H-pyrazole-3-carboxylic acid instead of 6-chloro-1H-indazole-3-carboxylic acid.

¹H NMR (500 MHz, DMSO-d₆) δ 13.80 (s, 1H), 10.98 (s, 1H), 9.01 (s, 1H), 7.87 (s, 1H), 7.76 (d, J = 7.8 Hz, 1H), 7.70 (d, J = 7.8 Hz, 1H), 7.54 (s, 1H), 7.51 - 7.45 (m, 2H), 7.42 (d, J = 9.1 Hz, 1H), 7.26 (s, 1H), 5.10 (dd, J = 13.3, 5.1 Hz, 1H), 4.58 (d, J = 6.2 Hz, 2H), 4.45 (d, J = 17.3 Hz, 1H), 4.31 (d, J = 17.3 Hz, 1H), 2.91 (m, 1H), 2.65 - 2.55 (m, 1H), 2.38 (m, 1H), 2.04 - 1.96 (m, 1H).

### Compound 6. 6-chloro-N-((2-(2,6-dioxopiperidin-3-yl)-1-oxoisoindolin-5-yl)methyl)-1H-indole-3-carboxamide

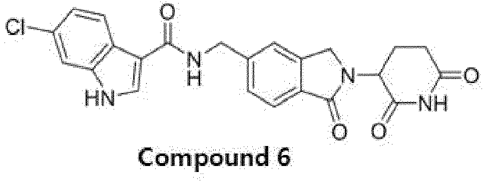

Compound 6 was synthesized in the same manner as in the synthesis method for Compound 1, with the exception of using 6-chloro-1H-indole-3-carboxylic acid instead of 6-chloro-1H-indazole-3-carboxylic acid.

¹H NMR (400 MHz, DMSO-d₆) δ 11.69 (s, 1H), 10.97 (s, 1H), 8.62 (t, J = 6.0 Hz, 1H), 8.18 - 8.08 (m, 2H), 7.69 (d, J = 7.8 Hz, 1H), 7.55 (s, 1H), 7.51 - 7.46 (m, 2H), 7.12 (dd, J = 8.6, 2.0 Hz, 1H), 5.10 (dd, J = 13.3, 5.1 Hz, 1H), 4.59 (d, J = 6.0 Hz, 2H), 4.44 (d, J = 17.3 Hz, 1H), 4.30 (d, J = 17.3 Hz, 1H), 2.88 (m, 1H), 2.68 - 2.55 (m, 1H), 2.42 - 2.29 (m, 1H), 2.02 - 1.90 (m, 1H).

### Compound 7. 5-Chloro-N-((2-(2,6-dioxopiperidin-3-yl)-1-oxoisoindolin-5-yl)methyl)-1H-indole-3-carboxamide

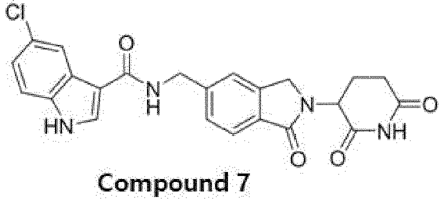

Compound 7 was synthesized in the same manner as in the synthesis method for Compound 1, with the exception of using 5-chloro-1H-indole-3-carboxylic acid instead of 6-chloro-1H-indazole-3-carboxylic acid.

¹H NMR (500 MHz, DMSO-d₆) δ 11.78 (s, 1H), 10.98 (s, 1H), 8.64 (t, J = 6.0 Hz, 1H), 8.18 - 8.12 (m, 2H), 7.69 (d, J = 7.8 Hz, 1H), 7.56 (s, 1H), 7.47 (dd, J = 11.4, 8.4 Hz, 2H), 7.17 (dd, J = 8.6, 2.2 Hz, 1H), 5.10 (dd, J = 13.3, 5.1 Hz, 1H), 4.59 (d, J = 5.9 Hz, 2H), 4.44 (d, J = 17.3 Hz, 1H), 4.31 (d, J = 17.3 Hz, 1H), 2.91 (m, 1H), 2.61 (m, 1H), 2.37 (m, 1H), 2.03 - 1.96 (m, 1H).

### Compound 8. 5-Chloro-N-((2-(2,6-dioxopiperidin-3-yl)-1-oxoisoindolin-5-yl)methyl)-1H-indazole-3-carboxamide

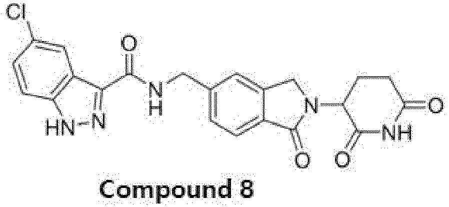

Compound 8 was synthesized in the same manner as in the synthesis method for Compound 1, with the exception of using 5-chloro-1H-indazole-3-carboxylic acid instead of 6-chloro-1H-indazole-3-carboxylic acid.

¹H NMR (500 MHz, DMSO-d₆) δ 13.85 (s, 1H), 10.98 (s, 1H), 9.19 (t, J = 6.3 Hz, 1H), 8.15 (d, J = 2.0 Hz, 1H), 7.68 (dd, J = 8.4, 5.0 Hz, 2H), 7.56 (s, 1H), 7.50 (d, J = 7.8 Hz, 1H), 7.44 (dd, J = 8.9, 2.0 Hz, 1H) , 5.10 (dd, J = 13.3, 5.1 Hz, 1H), 4.61 (d, J = 6.4 Hz, 2H), 4.44 (d, J = 17.3 Hz, 1H), 4.30 (d, J = 17.3 Hz, 1H), 2.90 (m, 1H), 2.59 (m, 1H), 2.37 (m, 1H), 1.99 (m, 1H) .

### Compound 9. 6-chloro-N-((2-(2,6-dioxopiperidin-3-yl)-1-oxoisoindolin-5-yl)methyl)-1H-indole-2-carboxamide

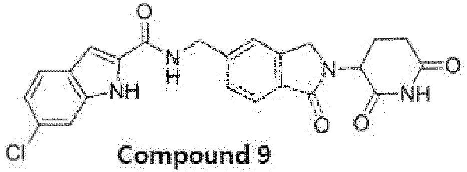

Compound 9 was synthesized in the same manner as in the synthesis method for Compound 1, with the exception of using 6-chloro-1H-indole-2-carboxylic acid instead of 6-chloro-1H-indazole-3-carboxylic acid.

¹H NMR (500 MHz, DMSO-d₆) δ 11.78 (s, 1H), 10.98 (s, 1H), 9.22 (t, J = 6.1 Hz, 1H), 7.71 (d, J = 7.8 Hz, 1H), 7.66 (d, J = 8.5 Hz, 1H), 7.56 (s, 1H), 7.51 - 7.47 (m, 1H), 7.44 (d, J = 1.9 Hz, 1H), 7.22 (s, 1H), 7.06 (dd, J = 8.6, 2.0 Hz, 1H), 5.10 (dd, J = 13.3, 5.1 Hz, 1H), 4.63 (d, J = 6.0 Hz, 2H), 4.45 (d, J = 17.3 Hz, 1H), 4.31 (d, J = 17.3 Hz, 1H), 2.91 (m, 1H), 2.62 - 2.55 (m, 1H), 2.37 (m, 1H), 2.06 - 1.93 (m, 1H).

### Compound 10. 6-Chloro-N-((2-(2,6-dioxopiperidin-3-yl)-1-oxoisoindolin-5-yl)methyl)benzo[d]isoxazole-3-carboxamide

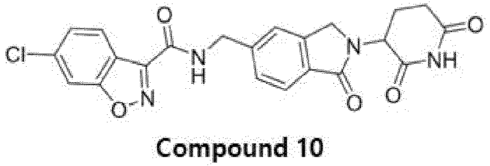

Compound 10 was synthesized in the same manner as in the synthesis method for Compound 1, with the exception of using 5-chlorobenzo[d]isoxazole-3-carboxylic acid instead of 6-chloro-1H-indazole-3-carboxylic acid.

¹H NMR (500 MHz, DMSO-d₆) δ 10.99 (s, 1H), 9.80 (t, J = 6.2 Hz, 1H), 8.15 (d, J = 1.7 Hz, 1H), 8.10 (d, J = 8.5 Hz, 1H), 7.71 (d, J = 7.9 Hz, 1H), 7.59 (s, 1H), 7.57 (dd, J = 8.5, 1.8 Hz, 1H), 7.52 (d, J = 7.9 Hz, 1H), 5.11 (dd, J = 13.3, 5.1 Hz, 1H), 4.64 (d, J = 6.2 Hz, 2H), 4.45 (d, J = 17.4 Hz, 1H), 4.31 (d, J = 17.4 Hz, 1H), 2.96 - 2.89 (m, 1H), 2.61 (m, 1H), 2.38 (dd, J = 13.1, 4.4 Hz, 1H), 1.99 (m, 1H).

### Compound 11. 5-(4-Chlorophenyl)-N-((2-(2,6-dioxopiperidin-3-yl)-1-oxoisoindolin-5-yl)methyl)isoxazole-3-carboxamide

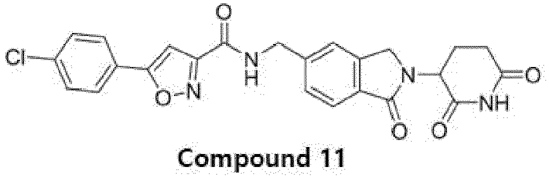

Compound 11 was synthesized in the same manner as in the synthesis method for Compound 1, with the exception of using 5-(4-chlorophenyl)isoxazole-3-carboxylic acid instead of 6-chloro-1H-indazole-3-carboxylic acid.

¹H NMR (500 MHz, DMSO-d₆) δ 10.99 (s, 1H), 9.53 (t, J = 6.2 Hz, 1H), 8.00 - 7.94 (m, 2H), 7.71 (d, J = 7.9 Hz, 1H), 7.67 - 7.62 (m, 2H), 7.56 (s, 1H), 7.48 (d, J = 7.9 Hz, 1H), 7.46 (s, 1H), 5.11 (dd, J = 13.3, 5.1 Hz, 1H), 4.59 (d, J = 6.2 Hz, 2H), 4.45 (d, J = 17.3 Hz, 1H), 4.31 (d, J = 17.3 Hz, 1H), 2.91 (m, 1H), 2.61 (m, 1H), 2.38 (m, 1H), 1.99 (m, 1H).

### Compound 12. N-((2-(2,6-Dioxopiperidin-3-yl)-1-oxoisoindolin-5-yl)methyl)-4-phenylpicolinamide

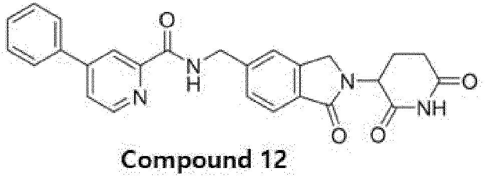

Compound 12 was synthesized in the same manner as in the synthesis method for Compound 1, with the exception of using 4-phenylpicolinic acid instead of 6-chloro-1H-indazole-3-carboxylic acid.

¹H NMR (500 MHz, DMSO-d₆) δ 10.98 (s, 1H), 9.57 (t, J = 6.4 Hz, 1H), 8.73 (d, J = 5.1 Hz, 1H), 8.34 - 8.25 (m, 1H), 7.96 (dd, J = 5.1, 1.9 Hz, 1H), 7.86 (m, 2H), 7.69 (d, J = 7.8 Hz, 1H), 7.59 - 7.47 (m, 5H), 5.10 (dd, J = 13.3, 5.1 Hz, 1H), 4.64 (d, J = 6.4 Hz, 2H), 4.44 (d, J = 17.3 Hz, 1H), 4.30 (d, J = 17.3 Hz, 1H), 2.91 (m, 1H), 2.61 (m, 1H), 2.37 (m, 1H), 2.08 - 1.93 (m, 1H).

### Compound 13. 3-(3,4-Dichlorophenyl)-N-((2-(2,6-dioxopiperidin-3-yl)-1-oxoisoindolin-5-yl)methyl)-1H-pyrazole-5-carboxamide

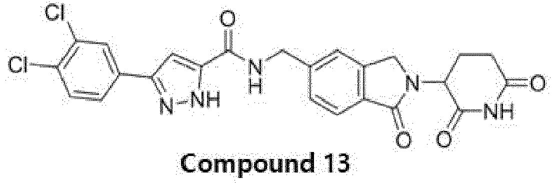

Compound 13 was synthesized in the same manner as in the synthesis method for Compound 1, with the exception of using 3-(3,4-dichlorophenyl)-1H-pyrazole-5-carboxylic acid instead of 6-chloro-1H-indazole-3-carboxylic acid.

¹H NMR (400 MHz, DMSO-d₆) δ 13.85 (m, 1H), 10.98 (s, 1H), 9.05 (m, 1H), 8.05 (m, 1H), 7.88 - 7.66 (m, 3H), 7.61 - 7.44 (m, 2H), 7.31 (m, 1H), 5.10 (dd, J = 13.3, 5.1 Hz, 1H), 4.58 (d, J = 14.5 Hz, 2H), 4.45 (d, J = 17.4 Hz, 1H), 4.31 (d, J = 17.3 Hz, 1H), 2.91 (m, 1H), 2.59 (d, J = 17.0 Hz, 1H), 2.38 (m, 1H), 2.05 - 1.92 (m, 1H) .

### Compound 14. 4-(3-Chlorophenyl)-N-((2-(2,6-dioxopiperidin-3-yl)-1-oxoisoindolin-5-yl)methyl)-1H-pyrrole-2-carboxamide

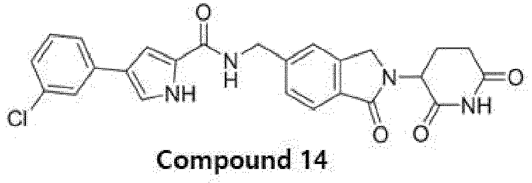

Compound 14 was synthesized in the same manner as in the synthesis method for Compound 1, with the exception of using 4-(3-chlorophenyl)-1H-pyrrole-2-carboxylic acid instead of 6-chloro-1H-indazole-3-carboxylic acid.

¹H NMR (400 MHz, DMSO-d₆) δ 11.80 (s, 1H), 10.98 (s, 1H), 8.82 - 8.65 (m, 1H), 7.70 (dd, J = 7.9, 2.7 Hz, 1H), 7.62 - 7.45 (m, 6H), 7.35 (m, 1H), 7.28 (s, 1H), 7.22 - 7.13 (m, 1H), 5.11 (dd, J = 13.3, 4.9 Hz, 1H), 4.57 (d, J = 5.8 Hz, 2H), 4.45 (d, J = 17.5 Hz, 1H), 4.31 (d, J = 17.0 Hz, 1H), 2.98 - 2.84 (m, 1H), 2.63 (m,1H), 2.40 - 2.32 (m, 1H), 2.04 - 1.96 (m, 1H).

### Compound 15. 1-(3-chlorophenyl)-N-((2-(2,6-dioxopiperidin-3-yl)-1-oxoisoindolin-5-yl)methyl)-1H-1,2,3-triazole-4-carboxamide

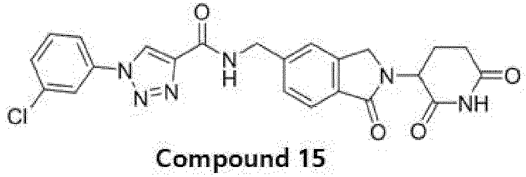

Compound 15 was synthesized in the same manner as in the synthesis method for Compound 1, with the exception of using 1-(3-chlorophenyl)-1H-1,2,3-triazole-4-carboxylic acid instead of 6-chloro-1H-indazole-3-carboxylic acid.

¹H NMR (400 MHz, DMSO-d₆) δ 10.99 (s, 1H), 9.42 (t, J = 6.3 Hz, 1H), 9.39 (s, 1H), 8.13 (t, J = 2.0 Hz, 1H), 7.99 (dt, J = 8.0, 1.6 Hz, 1H), 7.78 - 7.58 (m, 3H), 7.56 (s, 1H), 7.49 (d, J = 8.0 Hz, 1H), 5.11 (dd, J = 13.3, 5.1 Hz, 1H), 4.61 (d, J = 6.2 Hz, 2H), 4.45 (d, J = 17.4 Hz, 1H), 4.31 (d, J = 17.3 Hz, 1H), 2.91 (m, 1H), 2.66 - 2.57 (m, 1H), 2.42 - 2.29 (m, 1H), 2.05 - 1.93 (m, 1H).

### Compound 16. 5,6-dichloro-N-((2-(2,6-dioxopiperidin-3-yl)-1-oxoisoindolin-5-yl)methyl)-1H-benzo[d]imidazole-2-carboxamide

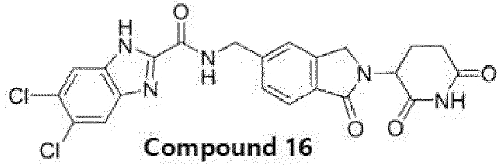

Compound 16 was synthesized in the same manner as in the synthesis method for Compound 1, with the exception of using 5,6-dichloro-1H-benzo[d]imidazole-2-carboxylic acid instead of 6-chloro-1H-indazole-3-carboxylic acid.

¹H NMR (400 MHz, DMSO-d₆) δ 13.62 (s, 1H), 10.99 (s, 1H), 9.78 (t, J = 6.3 Hz, 1H), 8.03 (s, 1H), 7.75 (s, 1H), 7.70 (d, J = 7.8 Hz, 1H), 7.57 (s, 1H), 7.49 (dd, J = 7.9, 1.4 Hz, 1H), 5.10 (dd, J = 13.3, 5.1 Hz, 1H), 4.61 (d, J = 6.3 Hz, 2H), 4.44 (d, J = 17.4 Hz, 1H), 4.30 (d, J = 17.3 Hz, 1H), 2.95 - 2.86 (m, 1H), 2.63 - 2.55 (m, 1H), 2.43 - 2.29 (m, 1H), 2.04 - 1.95 (m, 1H).

### Compound 17. N-((2-(2,6-Dioxopiperidin-3-yl)-1-oxoisoindolin-5-yl)methyl)-4-methyl-5-phenyl-1H-pyrazole-3-carboxamide

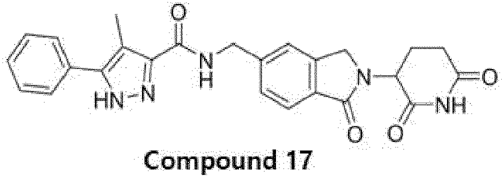

Compound 17 was synthesized in the same manner as in the synthesis method for Compound 1, with the exception of using 5-phenyl-4-methyl-1H-pyrazole-3-carboxylic acid instead of 6-chloro-1H-indazole-3-carboxylic acid.

¹H NMR (400 MHz, DMSO-*d*₆) δ 13.37 (s, 1H), 10.97 (s, 1H), 8.77 (t, J = 6.4 Hz, 1H), 7.69 (d, J = 7.8 Hz, 1H), 7.61 - 7.39 (m, 7H), 5.11 (dd, J = 13.2, 5.1 Hz, 1H), 4.56 (d, J = 6.0 Hz, 2H), 4.46 (d, J = 17.3 Hz, 1H), 4.32 (d, J = 17.3 Hz, 1H), 2.96 - 2.85 (m, 1H), 2.66 - 2.57 (m, 1H), 2.36 (m, 4H), 2.01 (m, 1H).

### Compound 18. 4-(4-chlorophenyl)-N-((2-(2,6-dioxopiperidin-3-yl)-1-oxoisoindolin-5-yl)methyl)picolinamide

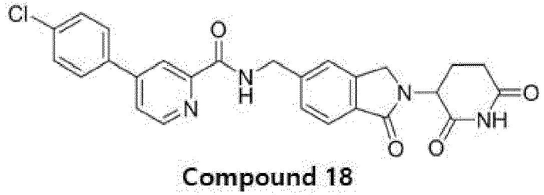

Compound 18 was synthesized in the same manner as in the synthesis method for Compound 1, with the exception of using 4-(4-chlorophenyl)picolinic acid instead of 6-chloro-1H-indazole-3-carboxylic acid.

¹H NMR (400 MHz, DMSO-*d*₆) δ 10.99 (s, 1H), 9.58 (t, J = 6.4 Hz, 1H), 8.75 (d, J = 5.1 Hz, 1H), 8.31 (d, J = 1.9 Hz, 1H), 7.97 (dd, J = 5.1, 1.9 Hz, 1H), 7.94 - 7.89 (m, 2H), 7.69 (d, J = 7.8 Hz, 1H), 7.65 - 7.61 (m, 2H), 7.56 (s, 1H), 7.54 - 7.46 (m, 1H), 5.11 (dd, J = 13.3, 5.1 Hz, 1H), 4.64 (d, J = 6.2 Hz, 2H), 4.44 (d, J = 17.4 Hz, 1H), 4.30 (d, J = 17.4 Hz, 1H), 3.00 - 2.84 (m, 1H), 2.64 - 2.52 (m, 1H), 2.43 - 2.30 (m, 1H) 2.05 - 1.92 (m, 1H).

### Compound 19. 5-(3-Chlorophenyl)-N-((2-(2,6-dioxopiperidin-3-yl)-1-oxoisoindolin-5-yl)methyl)isoxazole-3-carboxamide

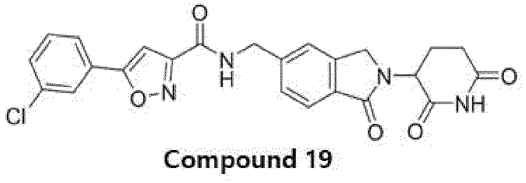

Compound 19 was synthesized in the same manner as in the synthesis method for Compound 1, with the exception of using 5-(3-chlorophenyl)isoxazole-3-carboxylic acid instead of 6-chloro-1H-indazole-3-carboxylic acid.

¹H NMR (400 MHz, DMSO-*d*₆) δ 10.99 (s, 1H), 9.55 (t, J = 6.2 Hz, 1H), 8.05 (d, J = 2.1 Hz, 1H), 7.92 (dt, J = 6.6, 1.9 Hz, 1H), 7.71 (d, J = 7.8 Hz, 1H), 7.64 - 7.59 (m, 2H), 7.56 (s, 1H), 7.54 (s, 1H), 7.49 (d, J = 7.7 Hz, 1H), 5.12 (dd, J = 13.3, 5.1 Hz, 1H), 4.59 (d, J = 6.2 Hz, 2H), 4.46 (d, J = 17.4 Hz, 1H), 4.32 (d, J = 17.2 Hz, 1H), 2.91 (m, 1H), 2.60 (m, 1H), 2.42 - 2.33 (m, 1H), 2.05 - 1.94 (m, 1H).

### Compound 20. 5-(3-Chlorophenyl)-N-((2-(2,6-dioxopiperidin-3-yl)-1-oxoisoindolin-5-yl)methyl)pyrimidine-2-carboxamide

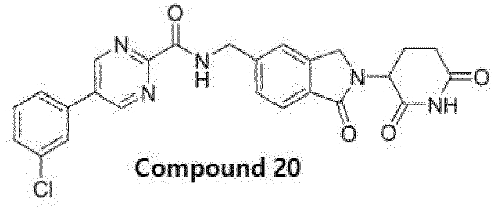

Compound 20 was synthesized in the same manner as in the synthesis method for Compound 1, with the exception of using 5-(3-chlorophenyl)pyrimidine-2-carboxylic acid instead of 6-chloro-1H-indazole-3-carboxylic acid.

¹H NMR (400 MHz, DMSO-*d*₆) δ 10.98 (s, 1H), 9.58 (t, J = 6.4 Hz, 1H), 8.76 (d, J = 5.1 Hz, 1H), 8.32 (d, J = 2.1 Hz, 1H), 8.00 (dd, J = 5.1, 2.0 Hz, 1H), 7.84 (m, 1H), 7.69 (d, J = 7.9 Hz, 1H), 7.64 - 7.54 (m, 3H), 7.50 (d, J = 7.9 Hz, 1H), 5.11 (dd, J = 13.3, 5.1 Hz, 1H), 4.65 (d, J = 6.3 Hz, 2H), 4.44 (d, J = 17.3 Hz, 1H), 4.31 (d, J = 17.4 Hz, 1H), 2.90 (m, 1H), 2.64 - 2.55 (m, 1H), 2.44 - 2.30 (m, 1H), 2.00 (m, 1H).

### Compound 21. 1-Benzyl-5-(3-chlorophenyl)-N-((2-(2,6-dioxopiperidin-3-yl)-1-oxoisoindolin-5-yl)methyl)-4-methyl-1H-pyrazole-3-carboxamide

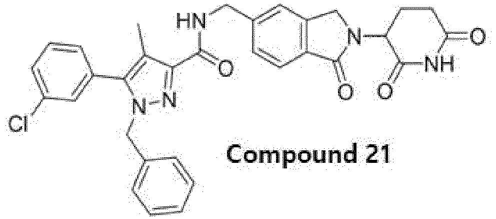

Compound 21 was synthesized in the same manner as in the synthesis method for Compound 1, with the exception of using 1-benzyl-5-(3-chlorophenyl)-4-methyl-1H-pyrazole-3-carboxylic acid instead of 6-chloro-1H-indazole-3-carboxylic acid.

¹H NMR (400 MHz, CDCl₃) δ 8.10 (s, 1H), 7.86 (s, 1H), 7.43 (m, 5H), 7.18 - 6.88 (m, 4H), 5.19 (s, 2H), 4.72 (s, 2H), 4.41 (m, 2H), 2.89 (m, 2H), 2.26 (m, 4H), 1.63 (m, 1H), 1.35 - 1.19 (m, 1H).

### Compound 22. 4-(3-Chlorophenyl)-N-((2-(2,6-dioxopiperidin-3-yl)-1-oxoisoindolin-5-yl)methyl)picolinamide

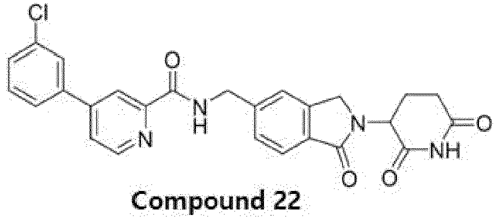

Compound 22 was synthesized in the same manner as in the synthesis method for Compound 1, with the exception of using 4-(3-chlorophenyl)picolinic acid instead of 6-chloro-1H-indazole-3-carboxylic acid.

¹H NMR (400 MHz, DMSO-*d*₆) δ 10.98 (s, 1H), 9.58 (t, J = 6.4 Hz, 1H), 8.76 (d, J = 5.1 Hz, 1H), 8.32 (d, J = 2.1 Hz, 1H), 8.00 (dd, J = 5.1, 2.0 Hz, 1H), 7.84 (m, 1H), 7.69 (d, J = 7.9 Hz, 1H), 7.64 - 7.54 (m, 3H), 7.50 (d, J = 7.9 Hz, 1H), 5.11 (dd, J = 13.3, 5.1 Hz, 1H), 4.65 (d, J = 6.3 Hz, 2H), 4.44 (d, J = 17.3 Hz, 1H), 4.31 (d, J = 17.4 Hz, 1H), 2.90 (m, 1H), 2.64 - 2.55 (m, 1H), 2.44 - 2.30 (m, 1H), 2.00 (m, 1H).

### Compound 23. N-((2-(2,6-Dioxopiperidin-3-yl)-1-oxoisoindolin-5-yl)methyl)-1-methyl-5-phenyl-1H-pyrazole-3-carboxamide

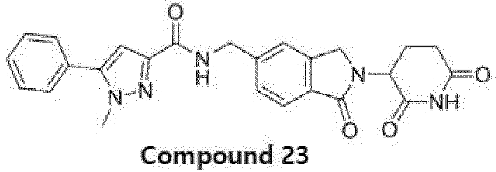

Compound 23 was synthesized in the same manner as in the synthesis method for Compound 1, with the exception of using 1-methyl-5-phenyl-1H-pyrazole-3-carboxylic acid instead of 6-chloro-1H-indazole-3-carboxylic acid.

¹H NMR (400 MHz, DMSO-*d*₆) δ 10.98 (s, 1H), 8.88 (s, 1H), 7.69 (d, J = 7.8 Hz, 1H), 7.60 - 7.55 (m, 3H), 7.54 - 7.45 (m, 4H), 6.80 (s, 1H), 5.11 (dd, J = 13.3, 5.1 Hz, 1H), 4.55 (d, J = 6.3 Hz, 2H), 4.45 (d, J = 17.3 Hz, 1H), 4.31 (d, J = 17.3 Hz, 1H), 3.92 (s, 3jH), 2.98 - 2.82 (m, 1H), 2.64 - 2.54 (m, 1H), 2.44 - 2.32 (m, 1H), 2.00 (m, 1H).

### Compound 24. N-((2-(2,6-Dioxopiperidin-3-yl)-1-oxoisoindolin-5-yl)methyl)-1-methyl-3-phenyl-1H-pyrazole-5-carboxamide

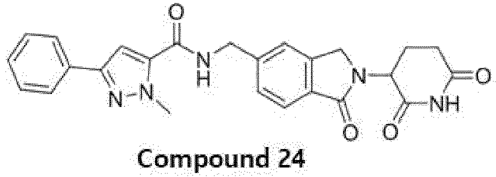

Compound 24 was synthesized in the same manner as in the synthesis method for Compound 1, with the exception of using 1-methyl-3-phenyl-1H-pyrazole-5-carboxylic acid instead of 6-chloro-1H-indazole-3-carboxylic acid.

¹H NMR (400 MHz, DMSO-*d*₆) δ 10.98 (s, 1H), 9.20 (t, J = 6.0 Hz, 1H), 7.80 - 7.75 (m, 2H), 7.72 (d, J = 7.9 Hz, 1H), 7.58 (s, 1H), 7.50 (d, J = 7.6 Hz, 1H), 7.44 (m, 2H), 7.37 - 7.29 (m, 2H), 5.11 (dd, J = 13.3, 5.1 Hz, 1H), 4.59 (d, J = 5.9 Hz, 2H), 4.46 (d, J = 17.4 Hz, 1H), 4.33 (d, J = 17.3 Hz, 1H), 4.12 (s, 3H), 2.97 - 2.86 (m, 1H), 2.65 - 2.56 (m, 1H), 2.37 (m, 1H), 2.06 - 1.97 (m, 1H).

### Compound 25. 5-(2,4-Dichlorophenyl)-N-((2-(2,6-dioxopiperidin-3-yl)-1-oxoisoindolin-5-yl)methyl)-1H-pyrazole-3-carboxamide

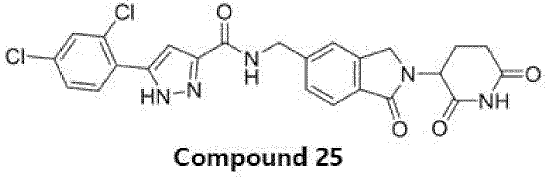

Compound 25 was synthesized in the same manner as in the synthesis method for Compound 1, with the exception of using 5-(2,4-dichlorophenyl)-1H-pyrazole-3-carboxylic acid instead of 6-chloro-1H-indazole-3-carboxylic acid .

¹H NMR (400 MHz, DMSO) δ 13.85 (s, 1H), 10.98 (s, 1H), 9.12 (s, 1H), 7.87 - 7.66 (m, 3H), 7.64 - 7.35 (m, 4H), 5.11 (dd, J = 13.3, 5.1 Hz, 1H), 4.59 (d, J = 5.8 Hz, 2H), 4.46 (d, J = 17.3 Hz, 1H), 4.32 (d, J = 17.3 Hz, 1H), 2.91 (m, 1H), 2.63 - 2.56 (m, 1H), 2.42 - 2.31 (m, 1H), 2.04 - 1.96 (m, 1H).

### Compound 26. 6-Chloro-N-((2-(2,6-dioxopiperidin-3-yl)-1-oxoisoindolin-5-yl)methyl)benzo[d]thiazole-2-carboxamide

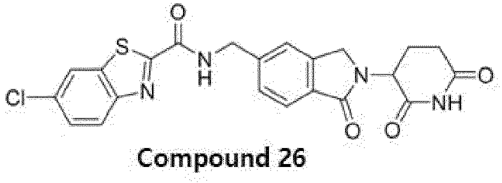

Compound 26 was synthesized in the same manner as in the synthesis method for Compound 1, with the exception of using 6-chlorobenzo[d]thiazole-2-carboxylic acid instead of 6-chloro-1H-indazole-3-carboxylic acid.

¹H NMR (400 MHz, DMSO-*d*₆) δ 10.98 (s, 1H), 9.90 (t, J = 6.3 Hz, 1H), 8.42 (d, J = 2.2 Hz, 1H), 8.15 (d, J = 8.8 Hz, 1H), 7.75 - 7.66 (m, 2H), 7.59 (s, 1H), 7.51 (d, J = 7.8 Hz, 1H), 5.11 (dd, J = 13.3, 5.1 Hz, 1H), 4.62 (d, J = 6.1 Hz, 2H), 4.45 (d, J = 17.3 Hz, 1H), 4.32 (d, J = 17.3 Hz, 1H), 2.93 - 2.85 (m, 1H), 2.60 (m, 1H), 2.40 - 2.32 (m, 1H), 2.03 - 1.95 (m, 1H).

### Compound 27. 4-Bromo-3-(3-chlorophenyl)-N-((2-(2,6-dioxopiperidin-3-yl)-1-oxoisoindolin-5-yl)methyl)-1H-pyrazole-5-carboxamide

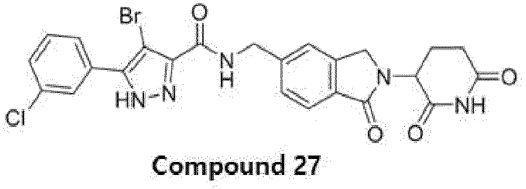

Compound 27 was synthesized in the same manner as in the synthesis method for Compound 1, with the exception of using 4-bromo-3-(3-chlorophenyl)-1H-pyrazole-5-carboxylic acid instead of 6-chloro-1H-indazole-3-carboxylic acid.

¹H NMR (400 MHz, DMSO-*d*₆) δ 14.11 (s, 1H), 10.99 (s, 1H), 8.99 (m, 1H), 7.81 (s, 1H), 7.74 (d, J = 7.0 Hz, 1H), 7.71 (d, J = 7.8 Hz, 1H), 7.62 - 7.55 (m, 3H), 7.49 (d, J = 7.8 Hz, 1H), 5.11 (dd, J = 13.3, 5.1 Hz, 1H), 4.56 (d, J = 6.1 Hz, 2H), 4.46 (d, J = 17.3 Hz, 1H), 4.32 (d, J = 17.3 Hz, 1H), 2.92 (m, 1H), 2.60 (m, 1H), 2.44 - 2.31 (m, 1H), 2.00 (m, 1H).

### Compound 28. 5-(2,5-Dichlorophenyl)-N-((2-(2,6-dioxopiperidin-3-yl)-1-oxoisoindolin-5-yl)methyl)-1H-pyrazole-3-carboxamide

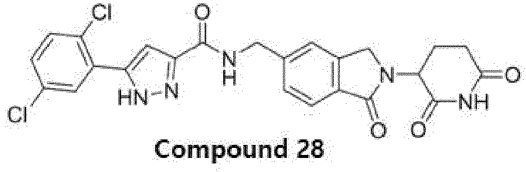

Compound 28 was synthesized in the same manner as in the synthesis method for Compound 1, with the exception of using 5-(2,5-dichlorophenyl)-1H-pyrazole-3-carboxylic acid instead of 6-chloro-1H-indazole-3-carboxylic acid.

¹H NMR (400 MHz, DMSO-*d*₆) δ 14.03 (s, 0.5H), 13.75 (s, 0.4H), 10.99 (s, 1H), 9.27 (s, 0.5H), 8.99 (s, 0.5H), 7.89 - 7.42 (m, 7H), 5.11 (dd, J = 13.3, 5.1 Hz, 1H), 4.60 (m, 2H), 4.46 (d, J = 17.4 Hz, 1H), 4.32 (d, J = 17.3 Hz, 1H), 2.91 (m, 1H), 2.64 - 2.56 (m, 1H), 2.43 - 2.29 (m, 1H), 2.05 - 1.95 (m, 1H).

### Compound 29. 5-(2,4-Dimethylphenyl)-N-((2-(2,6-dioxopiperidin-3-yl)-1-oxoisoindolin-5-yl)methyl)-1H-pyrazole-3-carboxamide

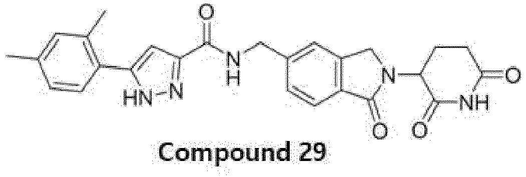

Compound 29 was synthesized in the same manner as in the synthesis method for Compound 1, with the exception of using 5-(2,4-dimethylphenyl)-1H-pyrazole-3-carboxylic acid instead of 6-chloro-1H-indazole-3-carboxylic acid.

¹H NMR (500 MHz, DMSO-d₆) δ 13.40 (s, 1H), 11.00 (s, 1H), 8.92 (s, 1H), 7.70 (d, J = 7.9 Hz, 1H), 7.55 (s, 1H), 7.48 (d, J = 7.8 Hz, 1H), 7.37 (s, 1H), 7.20 - 7.05 (m, 2H), 6.78 (s, 1H), 5.12 (dd, J = 13.3, 5.1 Hz, 1H), 4.58 (d, J = 6.0 Hz, 2H), 4.46 (d, J = 17.3 Hz, 1H), 4.32 (d, J = 17.3 Hz, 1H), 2.92 (m, 1H), 2.65 - 2.56 (m, 1H), 2.43 - 2.35 (m, 4H), 2.32 (s, 3H), 2.03 - 1.96 (m, 1H).

### Compound 30. N-((2-(2,6-Dioxopiperidin-3-yl)-1-oxoisoindolin-5-yl)methyl)-[1,1'-biphenyl]-3-carboxamide

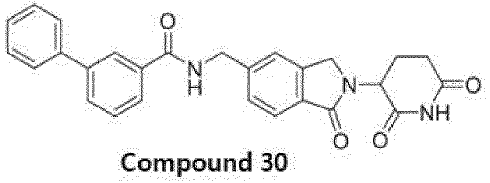

Compound 30 was synthesized in the same manner as in the synthesis method for Compound 1, with the exception of using [1,1'-biphenyl]-3-carboxylic acid instead of 6-chloro-1H-indazole-3-carboxylic acid.

¹H NMR (500 MHz, DMSO-d₆) δ 11.00 (s, 1H), 9.30 (t, J = 6.0 Hz, 1H), 8.21 (t, J = 1.8 Hz, 1H), 7.91 (dt, J = 7.8, 1.3 Hz, 1H), 7.86 (m, 1H), 7.78 - 7.74 (m, 2H), 7.71 (d, J = 7.8 Hz, 1H), 7.62 - 7.57 (m, 2H), 7.51 (m, 3H), 7.45 - 7.36 (m, 1H), 5.11 (dd, J = 13.3, 5.1 Hz, 1H), 4.64 (d, J = 5.9 Hz, 2H), 4.46 (d, J = 17.3 Hz, 1H), 4.32 (d, J = 17.3 Hz, 1H), 2.92 (m, 1H), 2.60 (m, 1H), 2.45 - 2.34 (m, 1H), 2.03 - 1.95 (m, 1H).

### Compound 31. N-((2-(2,6-Dioxopiperidin-3-yl)-1-oxoisoindolin-5-yl)methyl)-4-methylpicolinamide

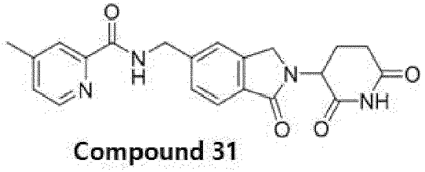

Compound 31 was synthesized in the same manner as in the synthesis method for Compound 1, with the exception of using 4-methylpicolinic acid instead of 6-chloro-1H-indazole-3-carboxylic acid.

¹H NMR (500 MHz, DMSO-d₆) δ 10.99 (s, 1H), 9.46 (t, J = 6.4 Hz, 1H), 8.54 - 8.50 (m, 1H), 7.91 (dd, J = 1.8, 0.9 Hz, 1H), 7.69 (d, J = 7.6 Hz, 1H), 7.54 (s, 1H), 7.50 - 7.43 (m, 2H), 5.11 (dd, J = 13.3, 5.1 Hz, 1H), 4.61 (d, J = 6.4 Hz, 2H), 4.44 (d, J = 17.3 Hz, 1H), 4.30 (d, J = 17.2 Hz, 1H), 2.96 - 2.87 (m, 1H), 2.63 - 2.57 (m, 1H), 2.42 (s, 3H), 2.40 - 2.35 (m, 1H), 1.99 (m, 1H).

### Compound 32. N-((2-(2,6-Dioxopiperidin-3-yl)-1-oxoisoindolin-5-yl)methyl)-4-phenylpicolinamide

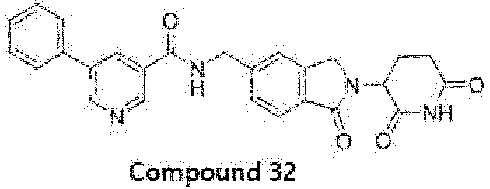

Compound 32 was synthesized in the same manner as in the synthesis method for Compound 1, with the exception of using 4-phenylpicolinic acid instead of 6-chloro-1H-indazole-3-carboxylic acid.

¹H NMR (500 MHz, DMSO-d₆) δ 11.00 (s, 1H), 9.46 (t, J = 5.9 Hz, 1H), 9.06 (dd, J = 4.7, 2.1 Hz, 2H), 8.54 (t, J = 2.2 Hz, 1H), 7.85 - 7.80 (m, 2H), 7.72 (d, J = 7.7 Hz, 1H), 7.60 (s, 1H), 7.57 - 7.51 (m, 3H), 7.49 - 7.45 (m, 1H), 5.12 (dd, J = 13.3, 5.1 Hz, 1H), 4.67 (d, J = 5.9 Hz, 2H), 4.46 (d, J = 17.3 Hz, 1H), 4.32 (d, J = 17.3 Hz, 1H), 2.98 - 2.85 (m, 1H), 2.66 - 2.57 (m, 1H), 2.44 - 2.35 (m, 1H), 2.01 (m, 1H).

### Compound 33. 6-Chloro-N-((2-(2,6-dioxopiperidin-3-yl)-1-oxoisoindolin-5-yl)methyl)benzo[d]oxazole-2-carboxamide

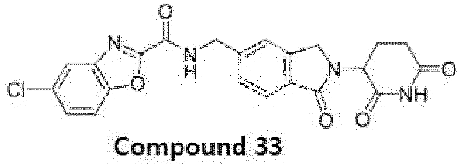

Compound 33 was synthesized in the same manner as in the synthesis method for Compound 1, with the exception of using 5-chlorobenzo[d]oxazole-2-carboxylic acid instead of 6-chloro-1H-indazole-3-carboxylic acid.

¹H NMR (500 MHz, DMSO-d₆) δ 11.00 (s, 1H), 10.01 (t, J = 6.3 Hz, 1H), 8.13 (d, J = 2.0 Hz, 1H), 7.94 (d, J = 8.6 Hz, 1H), 7.71 (d, J = 7.8 Hz, 1H), 7.61 - 7.56 (m, 2H), 7.54 - 7.48 (m, 1H), 5.11 (dd, J = 13.3, 5.1 Hz, 1H), 4.62 (d, J = 6.2 Hz, 2H), 4.45 (d, J = 17.4 Hz, 1H), 4.31 (d, J = 17.3 Hz, 1H), 2.91 (m, 1H), 2.65 - 2.56 (m, 1H), 2.38 (m, 1H), 2.04 - 1.96 (m, 1H).

### Compound 34. N-((2-(2,6-Dioxopiperidin-3-yl)-1-oxoisoindolin-5-yl)methyl)-1H-indazole-3-carboxamide

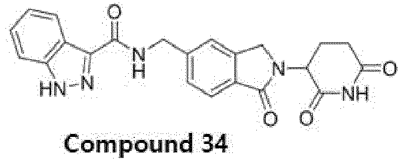

Compound 34 was synthesized in the same manner as in the synthesis method for Compound 1, with the exception of using 1H-indazole-3-carboxylic acid instead of 6-chloro-1H-indazole-3-carboxylic acid.

¹H NMR (500 MHz, DMSO-d₆) δ 13.64 (s, 1H), 10.99 (s, 1H), 9.13 (t, J = 6.3 Hz, 1H), 8.18 (d, J = 8.1 Hz, 1H), 7.70 (d, J = 7.8 Hz, 1H), 7.63 (dd, J = 8.4, 1.0 Hz, 1H), 7.58 (s, 1H), 7.51 (d, J = 7.8 Hz, 1H), 7.43 (m, 1H), 7.25 (m, 1H), 5.11 (dd, J = 13.3, 5.1 Hz, 1H), 4.62 (d, J = 6.3 Hz, 2H), 4.45 (d, J = 17.3 Hz, 1H), 4.31 (d, J = 17.3 Hz, 1H), 2.91 (m, 1H), 2.60 (m, 1H), 2.38 (m, 1H), 1.99 (m, 1H).

### Compound 35. 3-Bromo-N-((2-(2,6-dioxopiperidin-3-yl)-1-oxoisoindolin-5-yl)methyl)-1-methyl-1H-pyrazole-5-carboxamide

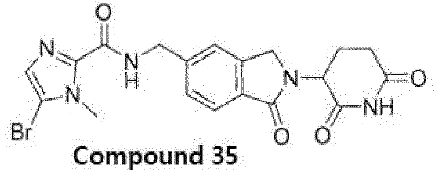

Compound 35 was synthesized in the same manner as in the synthesis method for Compound 1, with the exception of using 5-bromo-1-methyl-1H-imidazole-2-carboxylic acid instead of 6-chloro-1H-indazole-3-carboxylic acid.

¹H NMR (500 MHz, DMSO-d₆) δ 11.00 (s, 1H), 9.22 (t, J = 6.0 Hz, 1H), 7.71 (d, J = 7.8 Hz, 1H), 7.55 (s, 1H), 7.49 - 7.44 (m, 1H), 7.04 (s, 1H), 5.12 (dd, J = 13.3, 5.1 Hz, 1H), 4.56 (d, J = 6.0 Hz, 2H), 4.45 (d, J = 17.3 Hz, 1H), 4.32 (d, J = 17.3 Hz, 1H), 4.05 (s, 3H), 2.97 - 2.86 (m, 1H), 2.66 - 2.57 (m, 1H), 2.42 - 2.33 (m, 1H), 2.03 - 1.96 (m, 1H).

### Compound 36. N-((2-(2,6-Dioxopiperidin-3-yl)-1-oxoisoindolin-5-yl)methyl)-1-methyl-1H-indazole-3-carboxamide

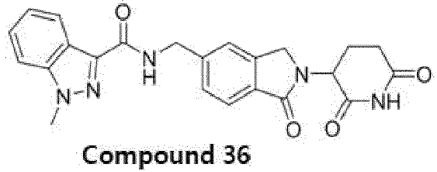

Compound 36 was synthesized in the same manner as in the synthesis method for Compound 1, with the exception of using 1-methyl-1H-indazole-3-carboxylic acid instead of 6-chloro-1H-indazole-3-carboxylic acid.

¹H NMR (500 MHz, DMSO-d₆) δ 10.99 (s, 1H), 9.09 (t, J = 6.3 Hz, 1H), 8.18 (m, 1H), 7.75 (dd, J = 8.5, 0.9 Hz, 1H), 7.70 (d, J = 7.7 Hz, 1H), 7.57 (s, 1H), 7.53 - 7.43 (m, 2H), 7.28 (m, 1H), 5.11 (dd, J = 13.3, 5.1 Hz, 1H), 4.61 (d, J = 6.3 Hz, 2H), 4.45 (d, J = 17.3 Hz, 1H), 4.31 (d, J = 17.3 Hz, 1H), 4.16 (s, 3H), 2.98 - 2.85 (m, 1H), 2.60 (m, 1H), 2.38 (m, 1H), 2.05 - 1.96 (m, 1H).

### Compound 37. 4-Bromo-N-((2-(2,6-dioxopiperidin-3-yl)-1-oxoisoindolin-5-yl)methyl)-1-methyl-1H-imidazole-2-carboxamide

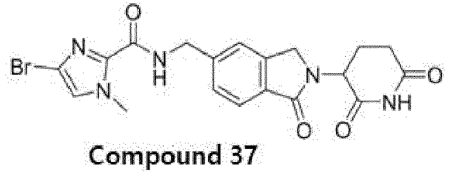

Compound 37 was synthesized in the same manner as in the synthesis method for Compound 1, with the exception of using 1-methyl-1H-indazole-3-carboxylic acid instead of 6-chloro-1H-indazole-3-carboxylic acid.

¹H NMR (500 MHz, MeOD) δ 7.79 - 7.75 (m, 1H), 7.56 (m, 1H), 7.53 - 7.48 (m, 1H), 7.28 (s, 1H), 5.14 (dd, J = 13.4, 5.2 Hz, 1H), 4.63 (s, 2H), 4.50 (d, J = 17.0 Hz, 1H), 4.44 (d, J = 17.1 Hz, 1H), 3.99 (s, 3H), 2.95 - 2.85 (m, 1H), 2.77 (m, 1H), 2.48 (m, 1H), 2.21 - 2.12 (m, 1H).

### Compound 38. 4-Bromo-N-((2-(2,6-dioxopiperidin-3-yl)-1-oxoisoindolin-5-yl)methyl)-1-methyl-1H-pyrrole-2-carboxamide

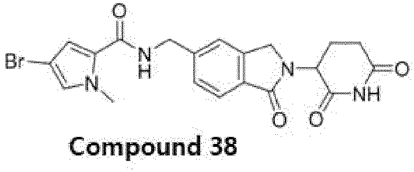

Compound 38 was synthesized in the same manner as in the synthesis method for Compound 1, with the exception of using 4-bromo-1-methyl-1H-pyrrole-2-carboxylic acid instead of 6-chloro-1H-indazole-3-carboxylic acid.

¹H NMR (500 MHz, DMSO-d₆) δ 11.00 (s, 1H), 8.75 (t, J = 6.1 Hz, 1H), 7.69 (dd, J = 7.9, 0.7 Hz, 1H), 7.51 (d, J = 1.4 Hz, 1H), 7.47 - 7.42 (m, 1H), 7.13 (d, J = 1.9 Hz, 1H), 6.94 (d, J = 1.9 Hz, 1H), 5.11 (dd, J = 13.3, 5.1 Hz, 1H), 4.50 (d, J = 6.0 Hz, 2H), 4.45 (d, J = 17.3 Hz, 1H), 4.31 (d, J = 17.3 Hz, 1H), 3.83 (s, 3H), 2.96 - 2.87 (m, 1H), 2.65 - 2.56 (m, 1H), 2.44 - 2.35 (m, 1H), 2.00 (m, 1H).

### Compound 39. N-((2-(2,6-Dioxopiperidin-3-yl)-1-oxoisoindolin-5-yl)methyl)-1-methyl-4-phenyl-1H-pyrrole-2-carboxamide

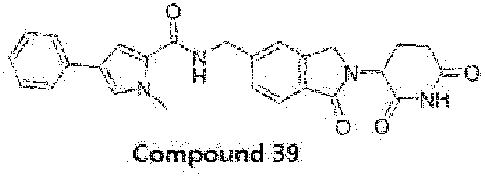

Compound 39 was synthesized in the same manner as in the synthesis method for Compound 1, with the exception of using 1-methyl-4-phenyl-1H-pyrrole-2-carboxylic acid instead of 6-chloro-1H-indazole-3-carboxylic acid.

¹H NMR (500 MHz, DMSO-d₆) δ 11.00 (s, 1H), 8.76 (t, J = 6.1 Hz, 1H), 7.72 - 7.69 (m, 1H), 7.55 (s, 1H), 7.53 - 7.50 (m, 2H), 7.48 (d, J = 7.7 Hz, 0H), 7.44 (d, J = 1.9 Hz, 1H), 7.38 - 7.33 (m, 1H), 7.28 (d, J = 2.0 Hz, 1H), 7.19 - 7.14 (m, 1H), 5.12 (dd, J = 13.3, 5.1 Hz, 1H), 4.54 (d, J = 6.0 Hz, 2H), 4.46 (d, J = 17.4 Hz, 1H), 4.32 (d, J = 17.4 Hz, 1H), 3.89 (s, 3H), 2.90 (m, 1H), 2.66 - 2.56 (m, 1H), 2.44 - 2.33 (m, 1H), 2.04 - 1.97 (m, 1H).

### Compound 40. 4-(3-Chlorophenyl)-N-((2-(2,6-dioxopiperidin-3-yl)-1-oxoisoindolin-5-yl)methyl)-1-methyl-1H-pyrrole-2-carboxamide

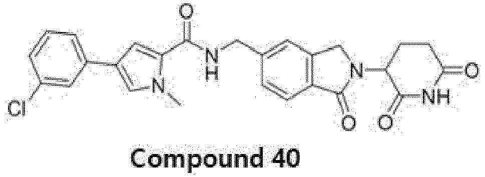

Compound 40 was synthesized in the same manner as in the synthesis method for Compound 1, with the exception of using 4-(3-chlorophenyl)-1-methyl-1H-pyrrole-2-carboxylic acid instead of 6-chloro-1H-indazole-3-carboxylic acid.

¹H NMR (500 MHz, DMSO-d₆) δ 11.00 (s, 1H), 8.74 (t, J = 6.1 Hz, 1H), 7.71 (dd, J = 7.8, 0.7 Hz, 1H), 7.56 (m, 3H), 7.51 - 7.45 (m, 2H), 7.38 (t, J = 7.8 Hz, 1H), 7.33 (d, J = 2.0 Hz, 1H), 7.21 (m, 1H), 5.12 (dd, J = 13.3, 5.1 Hz, 1H), 4.54 (d, J = 6.0 Hz, 2H), 4.46 (d, J = 17.3 Hz, 1H), 4.32 (d, J = 17.3 Hz, 1H), 3.88 (s, 3H), 2.98 - 2.84 (m, 1H), 2.65 - 2.58 (m, 1H), 2.44 - 2.35 (m, 1H), 2.00 (m, 1H).

### Compound 41. 5-(3-Chlorophenyl)-N-((2-(2,6-dioxopiperidin-3-yl)-1-oxoisoindolin-5-yl)methyl)-1-isobutyl-4-methyl-1H-pyrazole-3-carboxamide

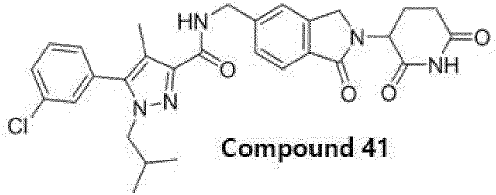

Compound 41 was synthesized in the same manner as in the synthesis method for Compound 1, with the exception of using 5-(3-chlorophenyl)-1-isobutyl-4-methyl-1H-pyrazole-3-carboxylic acid instead of 6-chloro-1H-indazole-3-carboxylic acid.

¹H NMR (500 MHz, DMSO-d₆) δ 11.00 (s, 1H), 8.73 (t, J = 6.3 Hz, 1H), 7.70 (d, J = 7.8 Hz, 1H), 7.62 - 7.46 (m, 5H), 7.38 (m, 1H), 5.12 (dd, J = 13.2, 5.1 Hz, 1H), 4.54 (d, J = 6.3 Hz, 2H), 4.46 (d, J = 17.3 Hz, 1H), 4.32 (d, J = 17.3 Hz, 1H), 3.86 (d, J = 7.3 Hz, 2H), 2.92 (m, 1H), 2.62 (m, 1H), 2.39 (m, 1H), 2.11 (s, 3H), 2.06 - 1.97 (m, 3fH), 0.71 (d, J = 6.7 Hz, 6H).

### Compound 42. 5-(3-chlorophenyl)-N-((2-(2,6-dioxopiperidin-3-yl)-1-oxoisoindolin-5-yl)methyl)-4-methyl-1-phenyl-1H-pyrazole-3-carboxamide

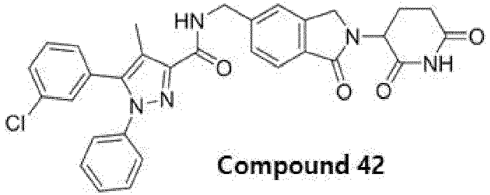

Compound 42 was synthesized in the same manner as in the synthesis method for Compound 1, with the exception of using 5-(3-chlorophenyl)-4-methyl-1-phenyl-1H-pyrazole-3-carboxylic acid instead of 6-chloro-1H-indazole-3-carboxylic acid.

¹H NMR (400 MHz, DMSO-d₆) δ 10.98 (s, 1H), 8.96 (t, J = 6.3 Hz, 1H), 7.70 (d, J = 7.8 Hz, 1H), 7.55 (s, 1H), 7.50 - 7.26 (m, 9H), 7.19 (dt, J = 7.3, 1.5 Hz, 1H), 5.11 (dd, J = 13.3, 5.1 Hz, 1H), 4.57 (d, J = 6.3 Hz, 2H), 4.46 (d, J = 17.3 Hz, 1H), 4.32 (d, J = 17.3 Hz, 1H), 2.99 - 2.84 (m, 1H), 2.64 - 2.58 (m, 1H), 2.37 (m, 1H), 2.24 (s, 3H), 2.00 (m, 1H).

### Compound 43. 5-(3-Chlorophenyl)-N-((2-(2,6-dioxopiperidin-3-yl)-1-oxoisoindolin-5-yl)methyl)-1-(4-fluorophenyl)-4-methyl-1H-pyrazole-3-carboxamide

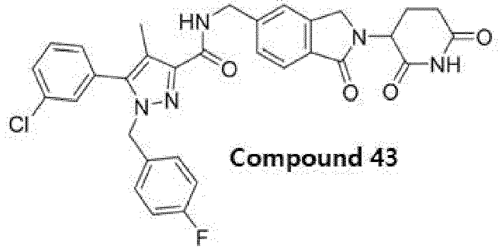

Compound 43 was synthesized in the same manner as in the synthesis method for Compound 1, with the exception of using 5-(3-chlorophenyl)-1-(4-fluorobenzyl)-4-methyl-1H-pyrazole-3-carboxylic acid instead of 6-chloro-1H-indazole-3-carboxylic acid.

¹H NMR (400 MHz, DMSO-d₆) δ 10.99 (s, 1H), 8.81 (t, J = 6.3 Hz, 1H), 7.70 (d, J = 7.8 Hz, 1H), 7.57 - 7.45 (m, 4H), 7.37 (t, J = 2.0 Hz, 1H), 7.27 (dt, J = 7.1, 1.6 Hz, 1H), 7.14 - 7.07 (m, 2H), 7.03 - 6.96 (m, 2H), 5.29 (s, 2H), 5.11 (dd, J = 13.2, 5.1 Hz, 1H), 4.54 (d, J = 6.3 Hz, 2H), 4.45 (d, J = 17.3 Hz, 1H), 4.32 (d, J = 17.3 Hz, 1H), 2.92 (m, 1H), 2.66 - 2.55 (m, 1H), 2.44 - 2.33 (m, 1H), 2.13 (s, 3H), 2.01 (m, 1H).

### Compound 44. 5-(3-Chlorophenyl)-N-((2-(2,6-dioxopiperidin-3-yl)-1-oxoisoindolin-5-yl)methyl)-1-(4-fluorophenyl)-4-methyl-1H -pyrazole-3-carboxamide

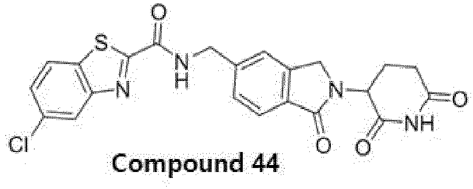

Compound 44 was synthesized in the same manner as in the synthesis method for Compound 1, with the exception of using 5-chlorobenzo[d]thiazole-2-carboxylic acid instead of 6-chloro-1H-indazole-3-carboxylic acid.

¹H NMR (300 MHz, DMSO) δ 10.99 (s, 1H), 9.93 (t, J = 6.3 Hz, 1H), 8.30 (d, J = 8.7 Hz, 1H), 8.20 (d, J = 2.0 Hz, 1H), 7.71 (d, J = 7.8 Hz, 1H), 7.66 (dd, J = 8.7, 2.1 Hz, 1H), 7.59 (s, 1H), 7.51 (d, J = 8.1 Hz, 1H), 5.11 (dd, J = 13.2, 5.1 Hz, 1H), 4.62 (d, J = 6.2 Hz, 2H), 4.46 (d, J = 17.4 Hz, 1H), 4.31 (d, J = 17.4 Hz, 1H), 2.99 - 2.84 (m, 1H), 2.60 (m, 1H), 2.45 - 2.33 (m, 1H), 2.05 - 1.95 (m, 1H).

### Compound 45. N-((2-(2,6-Dioxopiperidin-3-yl)-1-oxoisoindolin-5-yl)methyl)-5-methyl-4-phenylpicolinamide

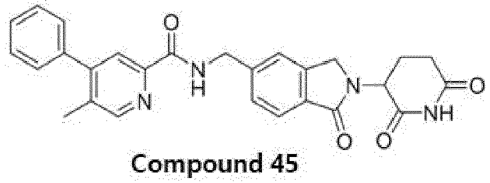

Compound 45 was synthesized in the same manner as in the synthesis method for Compound 1, with the exception of using 5-methyl-4-phenylpicolinic acid instead of 6-chloro-1H-indazole-3-carboxylic acid.

¹H NMR (400 MHz, DMSO) δ 10.98 (s, 1H), 9.50 (t, J = 6.4 Hz, 1H), 8.61 (s, 1H), 7.85 (s, 1H), 7.69 (d, J = 7.9 Hz, 1H), 7.58 - 7.42 (m, 7H), 5.10 (dd, J = 13.3, 5.1 Hz, 1H), 4.62 (d, J = 6.4 Hz, 2H), 4.44 (d, J = 17.3 Hz, 1H), 4.30 (d, J = 17.3 Hz, 1H), 2.91 (m, 1H), 2.65 - 2.56 (m, 1H), 2.45 - 2.37 (m, 1H), 2.35 (s, 3H), 2.05 - 1.92 (m, 1H).

### Compound 46. 5-Chloro-N-((2-(2,6-dioxopiperidin-3-yl)-1-oxoisoindolin-5-yl)methyl)-4-phenylpicolinamide

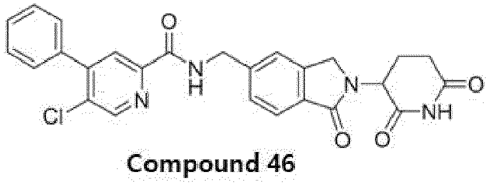

Compound 46 was synthesized in the same manner as in the synthesis method for Compound 1, with the exception of using 5-chloro-4-phenylpicolinic acid instead of 6-chloro-1H-indazole-3-carboxylic acid.

¹H NMR (400 MHz, DMSO-d₆) δ 10.98 (s, 1H), 9.60 (t, J = 6.4 Hz, 1H), 8.84 (s, 1H), 8.01 (s, 1H), 7.69 (d, J = 7.8 Hz, 1H), 7.63 - 7.45 (m, 7H), 5.10 (dd, J = 13.3, 5.1 Hz, 1H), 4.63 (d, J = 6.4 Hz, 2H), 4.44 (d, J = 17.3 Hz, 1H), 4.30 (d, J = 17.4 Hz, 1H), 2.91 (m, 1H), 2.66 - 2.55 (m, 1H), 2.44 - 2.31 (m, 1H) 2.04 - 1.95 (m, 1H).

### Compound 47. N-((2-(2,6-Dioxopiperidin-3-yl)-1-oxoisoindolin-5-yl)methyl)-6-phenylpyrazine-2-carboxamide

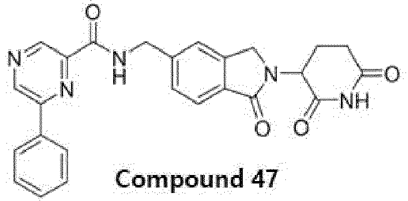

Compound 47 was synthesized in the same manner as in the synthesis method for Compound 1, with the exception of using 6-phenylpyrazine-2-carboxylic acid instead of 6-chloro-1H-indazole-3-carboxylic acid.

¹H NMR (500 MHz, DMSO) δ 10.99 (s, 1H), 9.76 (t, J = 6.4 Hz, 1H), 9.51 (s, 1H), 9.15 (s, 1H), 8.46 - 8.39 (m, 2H), 7.71 (d, J = 7.9 Hz, 1H), 7.62 - 7.50 (m, 5H), 5.10 (dd, J = 13.3, 5.1 Hz, 1H), 4.70 (d, J = 6.3 Hz, 2H), 4.45 (d, J = 17.3 Hz, 1H), 4.31 (d, J = 17.3 Hz, 1H), 2.91 (m, 1H), 2.60 (m, 1H), 2.38 (m, 1H), 2.03 - 1.95 (m, 1H).

### Compound 48. 3-(2-Chloro-6-fluorophenyl)-N-((2-(2,6-dioxopiperidin-3-yl)-1-oxoisoindolin-5-yl)methyl)-5-methylisoxazole-4-carboxamide

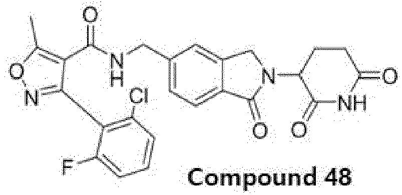

Compound 48 was synthesized in the same manner as in the synthesis method for Compound 1, with the exception of using 3-(2-chloro-6-fluorophenyl)-5-methylisoxazole-4-carboxylic acid instead of 6-chloro-1H-indazole-3-carboxylic acid.

¹H NMR (500 MHz, DMSO-d₆) δ 11.00 (s, 1H), 8.69 (t, J = 6.0 Hz, 1H), 7.66 (d, J = 7.8 Hz, 1H), 7.62-7.54 (m, 1H), 7.47 (d, J = 8.1 Hz, 1H), 7.40 (s, 1H), 7.39 (d, J = 8.7 Hz, 1H), 7.37-7.34 (m, 1H), 5.11 (dd, J = 13.3, 5.1 Hz, 1H), 4.44 (d, J = 6.4 Hz, 2H), 4.40 (s, 1H), 4.29 (d, J = 17.3 Hz, 1H), 2.97-2.86 (m, 1H), 2.67 (s, 3H), 2.64-2.57 (m, 1H), 2.47-2.35 (m, 1H), 2.03-1.97 (m, 1H).

### Compound 49. N-((2-(2,6-Dioxopiperidin-3-yl)-1-oxoisoindolin-5-yl)methyl)-3-phenyl-1H-pyrazole-5-carboxamide

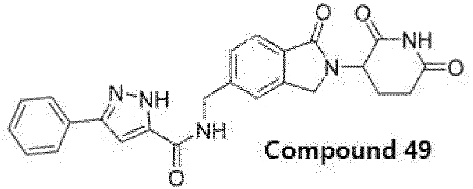

Compound 49 was synthesized in the same manner as in the synthesis method for Compound 1, with the exception of using 3-phenyl-1H-pyrazole-5-carboxylic acid instead of 6-chloro-1H-indazole-3-carboxylic acid.

¹H NMR (300 MHz, DMSO-d₆) δ 13.71 (s, 1H), 10.99 (s, 1H), 8.99 (s, 1H), 7.83-7.75 (m, 2H), 7.70 (d, J = 7.8 Hz, 1H), 7.55 (s, 1H), 7.47 (t, J = 7.5 Hz, 3H), 7.37 (t, J = 7.3 Hz, 1H), 7.15 (s, 1H), 5.10 (dd, J = 13.2, 5.1 Hz, 1H), 4.58 (d, J = 6.1 Hz, 2H), 4.45 (d, J = 17.4 Hz, 1H), 4.31 (d, J = 17.4 Hz, 1H), 2.97-2.83 (m, 1H), 2.63 (s, 1H), 2.44-2.26 (m, 1H), 2.05-1.93 (m, 1H).

### Compound 50. N-((2-(2,6-Dioxopiperidin-3-yl)-1-oxoisoindolin-5-yl)methyl)-1-phenyl-5-(trifluoromethyl)-1H-pyrazole-4-carboxamide

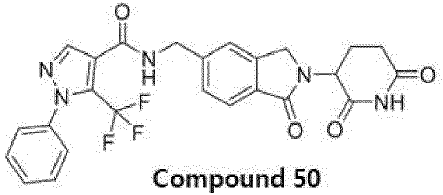

Compound 50 was synthesized in the same manner as in the synthesis method for Compound 1, with the exception of using 1-(cyclohexane -1,5-dien-1-yl)-5-(trifluoromethyl)-1H-pyrazole-4-carboxylic acid instead of 6-chloro-1H-indazole-3-carboxylic acid.

¹H NMR (300 MHz, DMSO) δ 11.00 (s, 1H), 9.23 (t, J = 6.0 Hz, 1H), 8.23 (s, 1H), 7.72 (d, J = 7.8 Hz, 1H), 7.63 - 7.46 (m, 7H), 5.12 (dd, J = 13.2, 5.1 Hz, 1H), 4.58 (d, J = 5.9 Hz, 2H), 4.47 (d, J = 17.3 Hz, 1H), 4.32 (d, J = 17.3 Hz, 1H), 3.03 - 2.82 (m, 1H), 2.60 (m, 1H), 2.40 (m, 1H), 2.01 (m, 1H).

### Compound 51. N-((2-(2,6-Dioxopiperidin-3-yl)-1-oxoisoindolin-5-yl)methyl)-[1,1'-biphenyl]-4-carboxamide

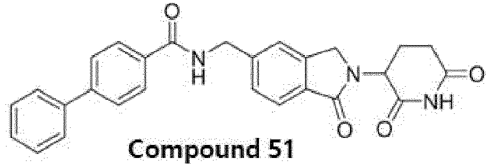

Compound 51 was synthesized in the same manner as in the synthesis method for Compound 1, with the exception of using [1,1'-biphenyl]-4-carboxylic acid instead of 6-chloro-1H-indazole-3-carboxylic acid.

¹H NMR (500 MHz, DMSO) δ 11.00 (s, 1H), 9.23 (t, J = 6.0 Hz, 1H), 8.04 - 8.00 (m, 2H), 7.83 - 7.79 (m, 2H), 7.77 - 7.73 (m, 2H), 7.71 (d, J = 7.8 Hz, 1H), 7.57 (s, 1H), 7.54 - 7.47 (m, 3H), 7.45 - 7.40 (m, 1H), 5.12 (dd, J = 13.4, 5.1 Hz, 1H), 4.63 (d, J = 5.9 Hz, 2H), 4.46 (d, J = 17.4 Hz, 1H), 4.32 (d, J = 17.3 Hz, 1H), 2.92 (m, 1H), 2.67 - 2.57 (m, 1H), 2.39 (m, 1H), 2.07 - 1.94 (m, 1H).

### Compound 52. N-((2-(2,6-Dioxopiperidin-3-yl)-1-oxoisoindolin-5-yl)methyl)-[1,1'-biphenyl]-2-carboxamide

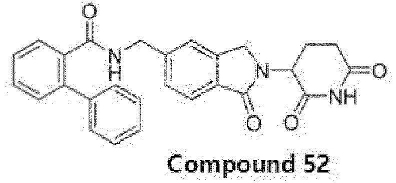

Compound 52 was synthesized in the same manner as in the synthesis method for Compound 1, with the exception of using [1,1'-biphenyl]-2-carboxylic acid instead of 6-chloro-1H-indazole-3-carboxylic acid.

¹H NMR (500 MHz, DMSO-d₆) δ 11.01 (s, 1H), 8.74 (t, J = 6.0 Hz, 1H), 7.59 (d, J = 7.8 Hz, 1H), 7.51 (td, J = 7.4, 1.7 Hz, 1H), 7.47 (dd, J = 7.6, 1.7 Hz, 1H), 7.45-7.41 (m, 1H), 7.39 (dd, J = 7.6, 1.3 Hz, 1H), 7.36-7.29 (m, 5H), 7.18 (d, J = 7.8 Hz, 1H), 7.13 (s, 1H), 5.12 (dd, J = 13.3, 5.1 Hz, 1H), 4.41-4.34 (m, 3H), 4.26 (d, J = 17.2 Hz, 1H), 2.98-2.87 (m, 1H), 2.64-2.57 (m, 1H), 2.48-2.38 (m, 1H), 2.04-1.97 (m, 1H).

### Compound 53. 1-(4-Chlorophenyl)-N-((2-(2,6-dioxopiperidin-3-yl)-1-oxoisoindolin-5-yl)methyl)cyclopentane-1-carboxamide

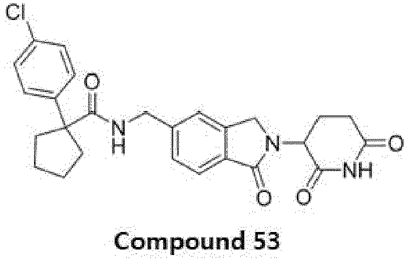

Compound 53 was synthesized in the same manner as in the synthesis method for Compound 1, with the exception of using 1-(4-chlorophenyl)cyclopentane-1-carboxylic acid instead of 6-chloro-1H-indazole-3-carboxylic acid.

¹H NMR (500 MHz, DMSO-d₆) δ 10.99 (s, 1H), 8.21 (t, J = 6.1 Hz, 1H), 7.57 (d, J = 7.8 Hz, 1H), 7.42-7.34 (m, 4H), 7.17 (d, J = 8.0 Hz, 1H), 7.02 (s, 1H), 5.09 (dd, J = 13.4, 5.1 Hz, 1H), 4.32 (d, J = 17.2 Hz, 1H), 4.28 (d, J = 6.0 Hz, 2H), 4.18 (d, J = 17.2 Hz, 1H), 2.95-2.86 (m, 1H), 2.64-2.54 (m, 3H), 2.43-2.30 (m, 1H), 2.03-1.96 (m, 1H), 1.85-1.77 (m, 2H), 1.67-1.54 (m, 4H).

### Compound 54. N-((2-(2,6-Dioxopiperidin-3-yl)-1-oxoisoindolin-5-yl)methyl)-1-phenylcyclopentane-1-carboxamide

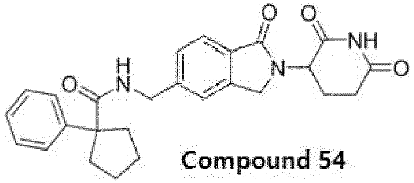

Compound 54 was synthesized in the same manner as in the synthesis method for Compound 1, with the exception of using 1-phenylcyclopentane-1-carboxylic acid instead of 6-chloro-1H-indazole-3-carboxylic acid.

¹H NMR (300 MHz, DMSO-d₆) δ 10.99 (s, 1H), 7.55 (d, J = 7.9 Hz, 1H), 7.40-7.22 (m, 6H), 7.18-7.12 (m, 1H), 7.04 (s, 1H), 5.08 (dd, J = 13.2, 5.1 Hz, 1H), 4.32 (d, J = 17.2 Hz, 3H), 4.18 (d, J = 17.3 Hz, 1H), 2.97-2.84 (m, 1H), 2.60 (d, J = 15.9 Hz, 3H), 2.44-2.26 (m, 1H), 2.06-1.92 (m, 1H), 1.91-1.76 (m, 2H), 1.69-1.55 (m, 4H).

### Compound 55. N-((2-(2,6-Dioxopiperidin-3-yl)-1-oxoisoindolin-5-yl)methyl)-2-oxo-1-phenylpyrrolidine-3-carboxamide

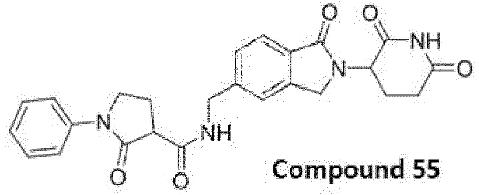

Compound 55 was synthesized in the same manner as in the synthesis method for Compound 1, with the exception of using 2-oxo-1-phenylpyrrolidine-3-carboxylic acid instead of 6-chloro-1H-indazole-3-carboxylic acid.

¹H NMR (300 MHz, DMSO-d₆) δ 10.99 (s, 1H), 8.84 (t, J = 5.9 Hz, 1H), 7.68 (dd, J = 9.8, 7.9 Hz, 3H), 7.55 (s, 1H), 7.47 (d, J = 7.9 Hz, 1H), 7.43-7.36 (m, 2H), 7.20-7.13 (m, 1H), 5.11 (dd, J = 13.2, 5.1 Hz, 1H), 4.57-4.38 (m, 3H), 4.31 (dd, J = 17.4, 2.9 Hz, 1H), 3.95-3.81 (m, 2H), 2.97-2.85 (m, 1H), 2.63 (s, 1H), 2.46-2.28 (m, 3H), 2.05-1.95 (m, 1H).

### Compound 56. N-((2-(2,6-Dioxopiperidin-3-yl)-1-oxoisoindolin-5-yl)methyl)benzofuran-2-carboxamide

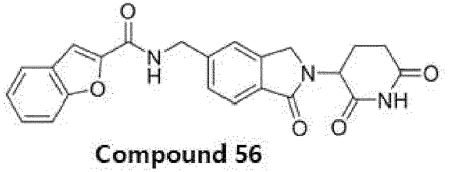

Compound 56 was synthesized in the same manner as in the synthesis method for Compound 1, with the exception of using benzofuran-2-carboxylic acid instead of 6-chloro-1H-indazole-3-carboxylic acid.

¹H NMR (500 MHz, DMSO) δ 11.00 (s, 1H), 9.44 (t, J = 6.2 Hz, 1H), 7.79 (d, J = 7.9 Hz, 1H), 7.69 (dd, J = 17.0, 8.2 Hz, 2H), 7.60 (s, 1H), 7.57 (s, 1H), 7.49 (m, 2H), 7.36 (t, J = 7.6 Hz, 1H), 5.11 (dd, J = 13.3, 4.9 Hz, 1H), 4.61 (d, J = 5.9 Hz, 2H), 4.45 (d, J = 17.4 Hz, 1H), 4.32 (d, J = 17.3 Hz, 1H), 2.97 - 2.85 (m, 1H), 2.60 (m, 1H), 2.39 (m, 1H), 2.01 (m, 1H).

### Compound 57. N-((2-(2,6-Dioxopiperidin-3-yl)-1-oxoisoindolin-5-yl)methyl)-5-methoxy-1H-indole-2-carboxamide

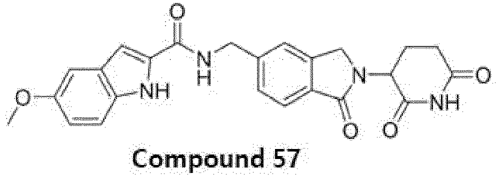

Compound 57 was synthesized in the same manner as in the synthesis method for Compound 1, with the exception of using 5-methoxy-1H-indole-2-carboxylic acid instead of 6-chloro-1H-indazole-3-carboxylic acid.

¹H NMR (400 MHz, DMSO) δ 11.46 (d, J = 2.2 Hz, 1H), 10.99 (s, 1H), 9.09 (t, J = 6.1 Hz, 1H), 7.71 (d, J = 7.8 Hz, 1H), 7.56 (s, 1H), 7.49 (d, J = 7.8 Hz, 1H), 7.32 (d, J = 8.9 Hz, 1H), 7.10 (dd, J = 6.2, 2.2 Hz, 2H), 6.84 (dd, J = 8.9, 2.5 Hz, 1H), 5.11 (dd, J = 13.3, 5.1 Hz, 1H), 4.63 (d, J = 6.0 Hz, 2H), 4.45 (d, J = 17.4 Hz, 1H), 4.31 (d, J = 17.3 Hz, 1H), 3.76 (s, 3H), 2.91 (m, 1H), 2.65 - 2.58 (m, 1H), 2.38 (m, 1H), 2.00 (m, 1H) .

### Compound 58. N-((2-(2,6-Dioxopiperidin-3-yl)-1-oxoisoindolin-5-yl)methyl)benzo[b]thiophene-2-carboxamide

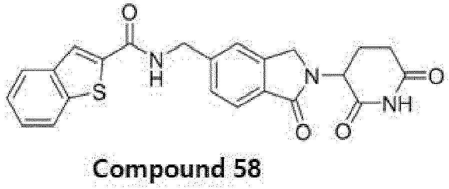

Compound 58 was synthesized in the same manner as in the synthesis method for Compound 1, with the exception of using benzo[b]thiophene-2-carboxylic acid instead of 6-chloro-1H-indazole-3-carboxylic acid.

¹H NMR (400 MHz, DMSO) δ 10.99 (s, 1H), 9.43 (t, J = 6.0 Hz, 1H), 8.16 (s, 1H), 8.05 - 8.02 (m, 1H), 7.96 (m, 1H), 7.72 (d, J = 7.8 Hz, 1H), 7.58 (s, 1H), 7.53 - 7.40 (m, 3H), 5.11 (dd, J = 13.3, 5.1 Hz, 1H), 4.62 (d, J = 5.9 Hz, 2H), 4.46 (d, J = 17.4 Hz, 1H), 4.32 (d, J = 17.3 Hz, 1H), 2.91 (m, 1H), 2.60 (m, 1H), 2.38 (m, 1H), 2.00 (m, 1H).

### Compound 59. 7-chloro-N-((2-(2,6-dioxopiperidin-3-yl)-1-oxoisoindolin-5-yl)methyl)-4-hydroxyquinoline-3-carboxamide

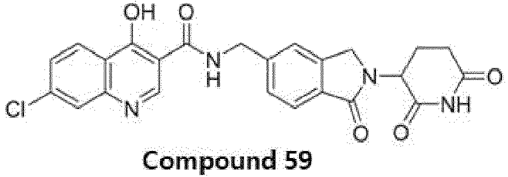

Compound 59 was synthesized in the same manner as in the synthesis method for Compound 1, with the exception of using 7-chloro-4-hydroxyquinoline-3-carboxylic acid instead of 6-chloro-1H-indazole-3-carboxylic acid.

¹H NMR (400 MHz, DMSO) δ 12.75 (s, 1H), 10.98 (s, 1H), 10.50 - 10.29 (m, 1H), 8.82 (s, 0.5H), 8.73 (s, 0.5H), 8.25 (d, J = 8.7 Hz, 1H), 7.77 (d, J = 2.1 Hz, 1H), 7.71 (d, J = 7.8 Hz, 1H), 7.67 - 7.62 (m, 1H), 7.56 (s, 1H), 7.54 - 7.43 (m, 2H), 5.11 (dd, J = 13.3, 5.1 Hz, 1H), 4.68 (t, J = 6.3 Hz, 2H), 4.45 (d, J = 17.3 Hz, 1H), 4.31 (d, J = 17.2 Hz, 1H), 3.00 - 2.87 (m, 1H), 2.70 - 2.57 (m, 1H), 2.43 - 2.26 (m, 1H), 2.04 - 1.94 (m, 1H).

### Compound 60. N-((2-(2,6-Dioxopiperidin-3-yl)-1-oxoisoindolin-5-yl)methyl)-2-oxo-2H-cromene-3-carboxamide

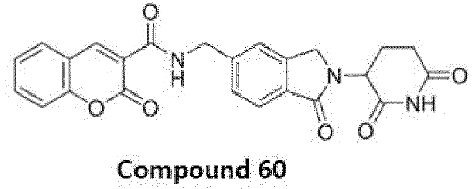

Compound 60 was synthesized in the same manner as in the synthesis method for Compound 1, with the exception of using 2-oxo-2H-cromene-3-carboxylic acid instead of 6-chloro-1H-indazole-3-carboxylic acid.

¹H NMR (400 MHz, DMSO) δ 10.99 (s, 1H), 9.26 (t, J = 6.1 Hz, 1H), 8.89 (s, 1H), 8.00 (dd, J = 7.8, 1.6 Hz, 1H), 7.80 - 7.74 (m, 1H), 7.70 (d, J = 7.8 Hz, 1H), 7.58 (s, 1H), 7.52 (t, J = 7.9 Hz, 2H),7.45 (t, J = 7.5 Hz, 1H), 5.11 (dd, J = 13.3, 5.1 Hz, 1H), 4.67 (d, J = 6.1 Hz, 2H), 4.45 (d, J = 17.3 Hz, 1H), 4.31 (d, J = 17.3 Hz, 1H), 2.92 (m, 1H), 2.64 - 2.57 (m, 1H), 2.38 (m, 1H), 2.06 - 1.95 (m, 1H).

### Compound 61. N-((2-(2,6-Dioxopiperidin-3-yl)-1-oxoisoindolin-5-yl)methyl)cinnoline-4-carboxamide

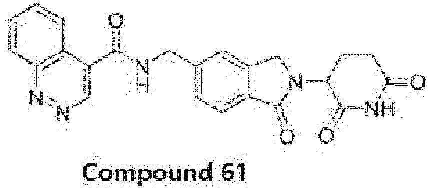

Compound 61 was synthesized in the same manner as in the synthesis method for Compound 1, with the exception of using cinnoline-4-carboxylic acid instead of 6-chloro-1H-indazole-3-carboxylic acid.

¹H NMR (400 MHz, DMSO-d₆) δ 11.00 (s, 1H), 9.66 (t, J = 5.9 Hz, 1H), 9.53 (s, 1H), 8.58 (d, J = 8.4 Hz, 1H), 8.28 (d, J = 8.3 Hz, 1H), 8.08 - 8.02 (m, 1H), 8.00 - 7.94 (m, 1H), 7.75 (d, J = 7.8 Hz, 1H), 7.67 (s, 1H), 7.58 (d, J = 7.8 Hz, 1H), 5.13 (dd, J = 13.3, 5.1 Hz, 1H), 4.72 (d, J = 5.9 Hz, 2H), 4.49 (d, J = 17.3 Hz, 1H), 4.36 (d, J = 17.3 Hz, 1H), 2.93 (m, 1H), 2.67 - 2.56 (m, 1H), 2.43 - 2.29 (m, 1H), 2.18 - 1.86 (m, 1H).

### Compound 62. 5-(4-Chlorophenyl)-N-((2-(2,6-dioxopiperidin-3-yl)-1-oxoisoindolin-5-yl)methyl)-1-isobutyl-4-methyl-1H-pyrazole-3-carboxamide

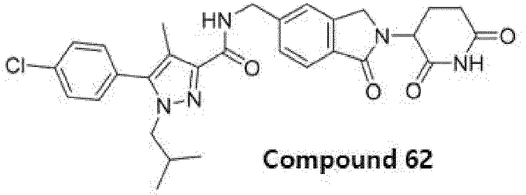

Compound 62 was synthesized in the same manner as in the synthesis method for Compound 1, with the exception of using 5-(4-chlorophenyl)-1-isobutyl-4-methyl-1H-pyrazole-3-carboxylic acid instead of 6-chloro-1H-indazole-3-carboxylic acid.

¹H NMR (400 MHz, DMSO) δ 10.99 (s, 1H), 8.71 (t, J = 6.3 Hz, 1H), 7.70 (d, J = 7.8 Hz, 1H), 7.64 - 7.59 (m, 2H), 7.54 (s, 1H), 7.47 (d, J = 7.8 Hz, 1H), 7.46 - 7.40 (m, 2H), 5.11 (dd, J = 13.3, 5.1 Hz, 1H), 4.54 (d, J = 6.2 Hz, 2H), 4.45 (d, J = 17.3 Hz, 1H), 4.31 (d, J = 17.3 Hz, 1H), 3.85 (d, J = 7.3 Hz, 2H), 2.92 (m, 1H), 2.60 (m, 1H), 2.39 (m, 1H), 2.10 (s, 3H), 2.02 (m, 2H), 0.70 (d, J = 6.7 Hz, 6H).

### Compound 63. 5-(3-Chlorophenyl)-1-cyclopropyl-N-((2-(2,6-dioxopiperidin-3-yl)-1-oxoisoindolin-5-yl)methyl)-4-methyl-1H-pyrazole-3-carboxamide

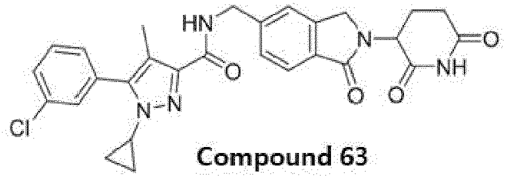

Compound 63 was synthesized in the same manner as in the synthesis method for Compound 1, with the exception of using 5-(3-chlorophenyl)-1-cyclopropyl-4-methyl-1H-pyrazole-3-carboxylic acid instead of 6-chloro-1H-indazole-3-carboxylic acid.

¹H NMR (400 MHz, DMSO) δ 10.99 (s, 1H), 8.72 (t, J = 6.4 Hz, 1H), 7.70 (d, J = 7.8 Hz, 1H), 7.63 - 7.56 (m, 3H), 7.53 (s, 1H), 7.49 - 7.42 (m, 2H), 5.11 (dd, J = 13.3, 5.0 Hz, 1H), 4.53 (d, J = 6.3 Hz, 2H), 4.45 (d, J = 17.3 Hz, 1H), 4.31 (d, J = 17.3 Hz, 1H), 3.66 (m, 1H), 2.92 (m, 1H), 2.60 (m, 1H), 2.44 - 2.29 (m, 1H), 2.13 (s, 3H), 2.00 (m, 1H), 1.01 (m, 2H), 0.87 (m, 2H).

### Compound 64. 5-(3-Chlorophenyl)-N-((2-(2,6-dioxopiperidin-3-yl)-1-oxoisoindolin-5-yl)methyl)-4-methyl-1-(2,2,2-trifluoroethyl)-1H-pyrazole-3-carboxamide

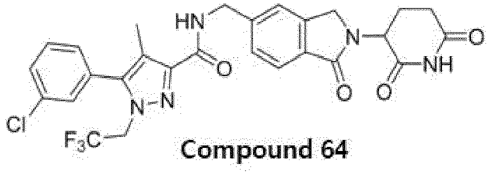

Compound 64 was synthesized in the same manner as in the synthesis method for Compound 1, with the exception of using 5-(3-chlorophenyl)-4-methyl-1-(2,2,2-trifluoroethyl)-1H-pyrazole-3-carboxylic acid instead of 6-chloro-1H-indazole-3-carboxylic acid.

¹H NMR (400 MHz, DMSO) δ 10.99 (s, 1H), 8.80 (t, J = 6.2 Hz, 1H), 7.70 (d, J = 7.8 Hz, 1H), 7.64 - 7.58 (m, 2H), 7.54 (m, 2H), 7.47 (d, J = 7.9 Hz, 1H), 7.39 (m, 1H), 5.11 (dd, J = 13.3, 5.1 Hz, 1H), 4.97 (q, J = 8.9 Hz, 2H), 4.56 (d, J = 6.3 Hz, 2H), 4.46 (d, J = 17.4 Hz, 1H), 4.32 (d, J = 17.3 Hz, 1H), 2.92 (m, 1H), 2.64 - 2.56 (m, 1H), 2.38 (m, 1H), 2.11 (s, 3H), 2.04 - 1.99 (m, 1H).

### Compound 65. N-((2-(2,6-Dioxopiperidin-3-yl)-1-oxoisoindolin-5-yl)methyl)-5-methoxybenzo[b]thiophene-2-carboxamide

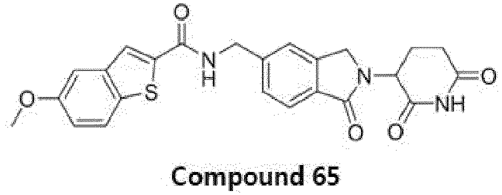

Compound 65 was synthesized in the same manner as in the synthesis method for Compound 1, with the exception of using 5-methoxybenzo[b]thiophene-2-carboxylic acid instead of 6-chloro-1H-indazole-3-carboxylic acid.

¹H NMR (400 MHz, DMSO-d₆) δ 10.97 (s, 1H), 9.39 (t, J = 6.3 Hz, 1H), 8.05 (s, 1H), 7.89 (d, J = 8.9 Hz, 1H), 7.71 (d, J = 7.8 Hz, 1H), 7.57 (s, 1H), 7.49 (d, J = 7.9 Hz, 1H), 7.44 (s, 1H), 7.11 (d, J = 2.3 Hz, 1H), 5.10 (dd, J = 13.3, 5.0 Hz, 1H), 4.60 (d, J = 5.9 Hz, 2H), 4.50 - 4.41 (m, 1H), 4.36 - 4.27 (m, 1H), 3.83 (s, 3H), 2.97 - 2.84 (m, 1H), 2.64 - 2.55 (m, 1H), 2.45 - 2.33 (m, 1H), 2.04 - 1.95 (m, 1H).

### Compound 66. N-((2-(2,6-Dioxopiperidin-3-yl)-1-oxoisoindolin-5-yl)methyl)-6-methylbenzo[b]thiophene-2-carboxamide

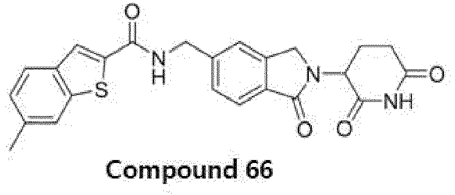

Compound 66 was synthesized in the same manner as in the synthesis method for Compound 1, with the exception of using 6-methylbenzo[b]thiophene-2-carboxylic acid instead of 6-chloro-1H-indazole-3-carboxylic acid.

¹H NMR (400 MHz, DMSO-d₆) δ 10.98 (s, 1H), 9.36 (t, J = 6.0 Hz, 1H), 8.09 (s, 1H), 7.83 (d, J = 9.2 Hz, 2H), 7.71 (d, J = 7.9 Hz, 1H), 7.56 (s, 1H), 7.49 (d, J = 7.9 Hz, 1H), 7.27 (d, J = 8.2 Hz, 1H), 5.10 (dd, J = 13.4, 4.9 Hz, 1H), 4.60 (d, J = 5.8 Hz, 2H), 4.50 - 4.41 (m, 1H), 4.36 - 4.27 (m, 1H), 2.97 - 2.84 (m, 1H), 2.64 - 2.55 (m, 1H), 2.44 (s, 3H), 2.42 - 2.31 (m, 1H), 2.03 - 1.95 (m, 1H).

### Compound 67. 3-Chloro-N-((2-(2,6-dioxopiperidin-3-yl)-1-oxoisoindolin-5-yl)methyl)-6-methylbenzo[b]thiophene-2-carboxamide

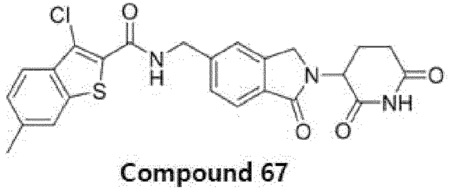

Compound 67 was synthesized in the same manner as in the synthesis method for Compound 1, with the exception of using 3-chloro-6-methylbenzo[b]thiophene-2-carboxylic acid instead of 6-chloro-1H-indazole-3-carboxylic acid.

¹H NMR (400 MHz, DMSO-d₆) δ 10.99 (s, 1H), 9.05 (t, J = 6.5 Hz, 1H), 7.91 (s, 1H), 7.80 (d, J = 8.3 Hz, 1H), 7.73 (d, J = 7.8 Hz, 1H), 7.60 (s, 1H), 7.53 (d, J = 7.9 Hz, 1H), 7.43 (d, J = 8.4 Hz, 1H), 5.12 (dd, J = 13.0, 4.8 Hz, 1H), 4.64 (d, J = 6.0 Hz, 2H), 4.52 - 4.43 (m, 1H), 4.38 - 4.29 (m, 1H), 2.98 - 2.86 (m, 1H), 2.65 - 2.56 (m, 1H), 2.48 (s, 3H), 2.44 - 2.36 (m, 1H), 2.05 - 1.97 (m, 1H).

### Compound 68. 3-Chloro-N-((2-(2,6-dioxopiperidin-3-yl)-1-oxoisoindolin-5-yl)methyl)-6-methoxybenzo[b]thiophene-2-carboxamide

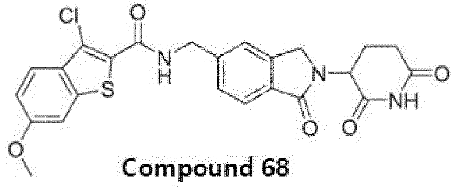

Compound 68 was synthesized in the same manner as in the synthesis method for Compound 1, with the exception of using 3-chloro-6-methoxybenzo[b]thiophene-2-carboxylic acid instead of 6-chloro-1H-indazole-3-carboxylic acid.

¹H NMR (400 MHz, DMSO-d₆) δ 10.98 (s, 1H), 8.94 (t, J = 6.1 Hz, 1H), 7.78 (d, J = 9.0 Hz, 1H), 7.72 (d, J = 7.8 Hz, 1H), 7.70-7.66 (m, 1H), 7.59 (s, 1H), 7.55-7.48 (m, 1H), 7.19 (dd, J = 8.9, 2.4 Hz, 1H), 5.15-5.06 (m, 1H), 4.63 (d, J = 5.9 Hz, 2H), 4.51-4.42 (m, 1H), 4.37-4.28 (m, 1H), 3.86 (s, 3H), 2.97-2.85 (m, 1H), 2.64-2.55 (m, 1H), 2.46-2.31 (m, 1H), 2.04-1.96 (m, 1H).

### Compound 69. N-((2-(2,6-Dioxopiperidin-3-yl)-1-oxoisoindolin-5-yl)methyl)-5,6-dimethoxybenzo[b]thiophene-2-carboxamide

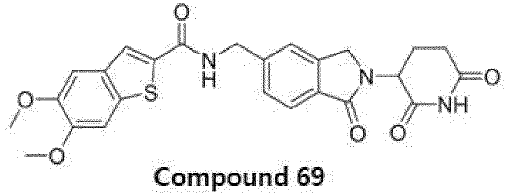

Compound 69 was synthesized in the same manner as in the synthesis method for Compound 1, with the exception of using 5,6-dimethoxybenzo[b]thiophene-2-carboxylic acid instead of 6-chloro-1H-indazole-3-carboxylic acid.

¹H NMR (400 MHz, DMSO-d₆) δ 10.98 (s, 1H), 9.27 (t, J = 6.5 Hz, 1H), 7.98 (s, 1H), 7.71 (d, J = 7.9 Hz, 1H), 7.57 (s, 2H), 7.49 (d, J = 8.0 Hz, 1H), 7.40 (s, 1H), 5.11 (dd, J = 13.4, 5.0 Hz, 1H), 4.59 (d, J = 5.9 Hz, 2H), 4.50-4.41 (m, 1H), 4.36-4.28 (m, 1H), 3.84 (s, 3H), 3.83 (s, 3H), 2.97-2.85 (m, 1H), 2.64-2.55 (m, 1H), 2.45-2.35 (m, 1H), 2.04-1.96 (m, 1H).

### Compound 70. N-((2-(2,6-Dioxopiperidin-3-yl)-1-oxoisoindolin-5-yl)methyl)-4-(trifluoromethyl)benzo[b]thiophene-2-carboxamide

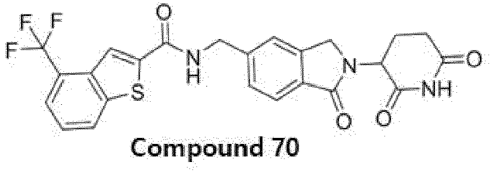

Compound 70 was synthesized in the same manner as in the synthesis method for Compound 1, with the exception of using 4-(trifluoromethyl)benzo[b]thiophene-2-carboxylic acid instead of 6-chloro-1H-indazole-3-carboxylic acid.

¹H NMR (400 MHz, DMSO) δ 10.99 (s, 1H), 9.70 (m, 1H), 8.41 (m, 2H), 7.86 (d, J = 7.5 Hz, 1H), 7.73 (d, J = 7.9 Hz, 1H), 7.65 (t, J = 7.9 Hz, 1H), 7.59 (s, 1H), 7.51 (d, J = 7.9 Hz, 1H), 5.11 (dd, J = 13.3, 4.9 Hz, 1H), 4.64 (d, J = 5.8 Hz, 2H), 4.47 (d, J = 17.3 Hz, 1H), 4.33 (d, J = 17.4 Hz, 1H), 2.91 (m, 1H), 2.60 (m, 1H), 2.43 - 2.27 (m, 1H), 2.01 (m, 1H).

### Compound 71. N-((2-(2,6-Dioxopiperidin-3-yl)-1-oxoisoindolin-5-yl)methyl)-7-(trifluoromethyl)benzo[b]thiophene-2-carboxamide

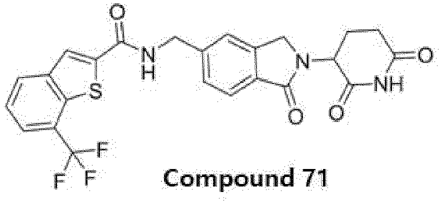

Compound 71 was synthesized in the same manner as in the synthesis method for Compound 1, with the exception of using 7-(trifluoromethyl)benzo[b]thiophene-2-carboxylic acid instead of 6-chloro-1H-indazole-3-carboxylic acid.

¹H NMR (400 MHz, DMSO) δ 10.99 (s, 1H), 9.60 (m, 1H), 8.31 (m, 1H), 7.92 (d, J = 7.5 kjHz, 1H), 7.77 - 7.63 (m, 2H), 7.59 (s, 1H), 7.51 (d, J = 7.9 Hz, 1H), 5.11 (dd, J = 13.2, 4.9 Hz, 1H), 4.64 (d, J = 5.8 Hz, 2H), 4.46 (d, J = 17.3 Hz, 1H), 4.34 (d, J = 16.0, 1H), 2.91 (m, 1H), 2.60 (m, 1H), 2.42 - 2.32 (m, 1H), 2.00 (m, 1H).

### Compound 72. 3-chloro-N-((2-(2,6-dioxopiperidin-3-yl)-1-oxoisoindolin-5-yl)methyl)-6-(trifluoromethyl)benzo[b]thiophene-2-carboxamide

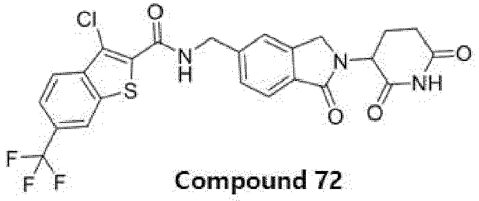

Compound 72 was synthesized in the same manner as in the synthesis method for Compound 1, with the exception of using 3-chloro-6-(trifluoromethyl)benzo[b]thiophene-2-carboxylic acid instead of 6-chloro-1H-indazole-3-carboxylic acid.

¹H NMR (400 MHz, DMSO) δ 11.00 (s, 1H), 9.26 (m, 1H), 8.71 (s, 1H), 8.12 (d, J = 8.5 Hz, 1H), 7.90 (d, J = 8.5 Hz, 1H), 7.74 (d, J = 7.9 Hz, 1H), 7.61 (s, 1H), 7.54 (d, J = 7.9 Hz, 1H), 5.12 (dd, J = 13.3, 4.9 Hz, 1H), 4.66 (d, J = 5.4 Hz, 2H), 4.48 (d, J = 17.2 Hz, 1H), 4.34 (d, J = 17.4 Hz, 1H), 3.00 - 2.83 (m, 1H), 2.65 - 2.58 (m, 1H), 2.40 (m, 1H), 2.08 - 1.94 (m, 1H).

### Compound 73. N-((2-(2,6-Dioxopiperidin-3-yl)-1-oxoisoindolin-5-yl)methyl)-5-(trifluoromethyl)benzo[b]thiophene-2-carboxamide

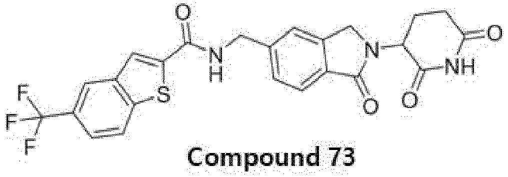

Compound 73 was synthesized in the same manner as in the synthesis method for Compound 1, with the exception of using 5-(trifluoromethyl)benzo[b]thiophene-2-carboxylic acid instead of 6-chloro-1H-indazole-3-carboxylic acid.

¹H NMR (400 MHz, DMSO) δ 10.99 (s, 1H), 9.60 (m, 1H), 8.41 (s, 1H), 8.34 - 8.25 (m, 2H), 7.75 (dd, J = 17.5, 8.2 Hz, 2H), 7.59 (s, 1H), 7.51 (d, J = 7.9 Hz, 1H), 5.11 (dd, J = 13.3, 4.8 Hz, 1H), 4.63 (d, J = 5.8 Hz, 2H), 4.46 (d, J = 17.3 Hz, 1H), 4.32 (d, J = 17.3 Hz, 1H), 2.90 (m, 1H), 2.60 (m, 1H), 2.39 (m, 1H), 2.00 (m, 1H).

### Compound 74. N-((2-(2,6-Dioxopiperidin-3-yl)-1-oxoisoindolin-5-yl)methyl)-4-methoxybenzo[b]thiophene-2-carboxamide

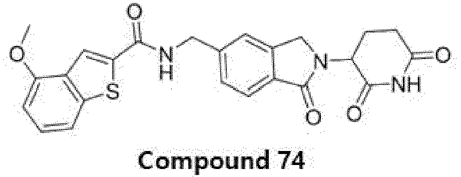

Compound 74 was synthesized in the same manner as in the synthesis method for Compound 1, with the exception of using 4-methoxybenzo[b]thiophene-2-carboxylic acid instead of 6-chloro-1H-indazole-3-carboxylic acid.

¹H NMR (400 MHz, DMSO) δ 10.99 (s, 1H), 9.58 - 9.36 (m, 1H), 8.28 (s, 1H), 7.71 (d, J = 7.8 Hz, 1H), 7.56 (d, J = z6.9 Hz, 2H), 7.49 (d, J = 7.9 Hz, 1H), 7.42 (t, J = 8.1 Hz, 1H), 6.95 (d, J = 7.9 Hz, 1H), 5.11 (dd, J = 13.3, 4.9 Hz, 1H), 4.59 (d, J = 5.8 Hz, 2H), 4.46 (d, J = 17.3 Hz, 1H), 4.32 (d, J = 17.4 Hz, 1H), 3.95 (s, 3H), 3.01 - 2.83 (m, 1H), 2.60 (m, 1H), 2.44 - 2.32 (m, 1H), 2.07 - 1.92 (m, 1H).

### Compound 75. 5-Chloro-N-((2-(2,6-dioxopiperidin-3-yl)-1-oxoisoindolin-5-yl)methyl)-3-methylbenzo[b]thiophene-2-carboxamide

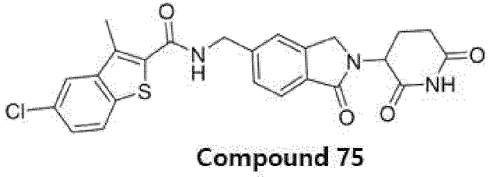

Compound 75 was synthesized in the same manner as in the synthesis method for Compound 1, with the exception of using 5-chloro-3-methylbenzo[b]thiophene-2-carboxylic acid instead of 6-chloro-1H-indazole-3-carboxylic acid.

¹H NMR (400 MHz, DMSO) δ 10.99 (s, 1H), 9.10 - 8.90 (m, 1H), 8.06 (d, J = 8.6 Hz, 1H), 7.98 (m, 1H), 7.73 (d, J = 7.9 Hz, 1H), 7.58 (s, 1H), 7.54 - 7.45 (m, 2H), 5.12 (dd, J = 13.5, 4.9 Hz, 1H), 4.60 (d, J = 5.8 Hz, 2H), 4.47 (d, J = 17.4 Hz, 1H), 4.33 (d, J = 17.3 Hz, 1H), 2.98 - 2.86 (m, 1H), 2.60 (m, 4H), 2.47 - 2.32 (m, 1H), 1.99 (m, 1H).

### Compound 76. N-((2-(2,6-Dioxopiperidin-3-yl)-1-oxoisoindolin-5-yl)methyl)-3-methylbenzo[b]thiophene-2-carboxamide

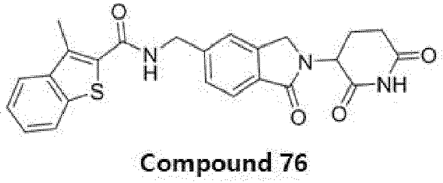

Compound 76 was synthesized in the same manner as in the synthesis method for Compound 1, with the exception of using 3-methylbenzo[b]thiophene-2-carboxylic acid instead of 6-chloro-1H-indazole-3-carboxylic acid.

¹H NMR (400 MHz, DMSO) δ 10.99 (s, 1H), 8.93 (t, J = 6.0 Hz, 1H), 8.07 - 7.95 (m, 1H), 7.94 - 7.84 (m, 1H), 7.73 (d, J = 7.9 Hz, 1H), 7.58 (s, 1H), 7.54 - 7.46 (m, 3H), 5.12 (dd, J = 13.5, 4.9 Hz, 1H), 4.60 (d, J = 5.9 Hz, 2H), 4.47 (d, J = 17.3 Hz, 1H), 4.33 (d, J = 17.3 Hz, 1H), 3.00 - 2.83 (m, 1H), 2.62 (m, 4H), 2.44 - 2.30 (m, 1H), 2.06 - 1.96 (m, 1H).

### Compound 77. 4-Chloro-N-((2-(2,6-dioxopiperidin-3-yl)-1-oxoisoindolin-5-yl)methyl)-3-methylbenzo[b]thiophene-2-carboxamide

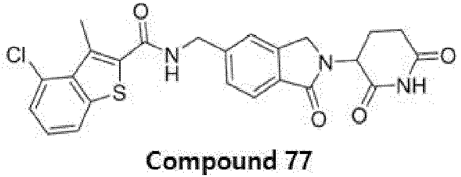

Compound 77 was synthesized in the same manner as in the synthesis method for Compound 1, with the exception of using 4-chloro-3-methylbenzo[b]thiophene-2-carboxylic acid instead of 6-chloro-1H-indazole-3-carboxylic acid.

¹H NMR (400 MHz, DMSO) δ 11.00 (s, 1H), 9.14 (m, 1H), 8.08 - 7.96 (m, 1H), 7.73 (d, J = 7.9 Hz, 1H), 7.58 (s, 1H), 7.52 - 7.47 (m, 2H), 7.43 (t, J = 7.9 Hz, 1H), 5.12 (dd, J = 13.4, 4.9 Hz, 1H), 4.60 (d, J = 5.8 Hz, 2H), 4.48 (d, J = 17.2 Hz, 1H), 4.34 (d, J = 17.4 Hz, 1H), 2.98 - 2.87 (m, 1H), 2.82 (s, 3H), 2.61 (m, 1H), 2.40 (m, 1H), 2.06 - 1.96 (m, 1H).

### Compound 78. 7-Chloro-N-((2-(2,6-dioxopiperidin-3-yl)-1-oxoisoindolin-5-yl)methyl)-3-methylbenzo[b]thiophene-2-carboxamide

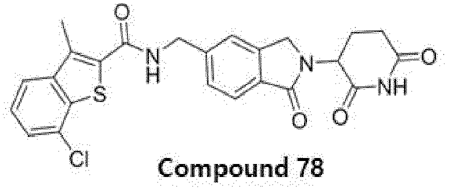

Compound 78 was synthesized in the same manner as in the synthesis method for Compound 1, with the exception of using 7-chloro-3-methylbenzo[b]thiophene-2-carboxylic acid instead of 6-chloro-1H-indazole-3-carboxylic acid.

¹H NMR (400 MHz, DMSO) δ 11.00 (s, 1H), 9.06 (m, 1H), 7.93 (d, J = 8.0 Hz, 1H), 7.73 (d, J = 7.9 Hz, 1H), 7.64 (d, J = 7.6 Hz, 1H), 7.58 (s, 1H), 7.56 - 7.48 (m, 2H), 5.12 (dd, J = 13.4, 5.0 Hz, 1H), 4.60 (d, J = 5.9 Hz, 2H), 4.48 (d, J = 17.4 Hz, 1H), 4.33 (d, J = 17.4 Hz, 1H), 3.02 - 2.86 (m, 1H), 2.64 (m, 4H), 2.43 - 2.31 (m, 1H), 2.01 (m, 1H).

### Compound 79. N-((2-(2,6-Dioxopiperidin-3-yl)-1-oxoisoindolin-5-yl)methyl)-4-fluoro-3-methylbenzo[b]thiophene-2-carboxamide

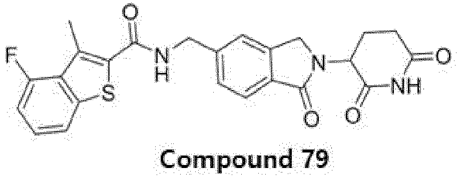

Compound 79 was synthesized in the same manner as in the synthesis method for Compound 1, with the exception of using 4-fluoro-3-methylbenzo[b]thiophene-2-carboxylic acid instead of 6-chloro-1H-indazole-3-carboxylic acid.

¹H NMR (400 MHz, DMSO) δ 11.00 (s, 1H), 9.06 (m, 1H), 7.84 (d, J = 8.2 Hz, 1H), 7.73 (d, J = 7.8 Hz, 1H), 7.58 (s, 1H), 7.49 (m, 2H), 7.23 (dd, J = 12.2, 8.1 Hz, 1H), 5.12 (dd, J = 13.4, 4.9 Hz, 1H), 4.59 (d, J = 5.8 Hz, 2H), 4.47 (d, J = 17.3 Hz, 1H), 4.33 (d, J = 17.3 Hz, 1H), 2.99 - 2.85 (m, 2H), 2.72 (s, 3H), 2.61 (m, 1H), 2.46 - 2.35 (m, 1H), 2.01 (m, 1H).

### Compound 80. N-((2-(2,6-Dioxopiperidin-3-yl)-1-oxoisoindolin-5-yl)methyl)-3-methylbenzofuran-2-carboxamide

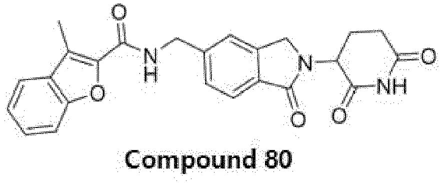

Compound 80 was synthesized in the same manner as in the synthesis method for Compound 1, with the exception of using 3-methylbenzofuran-2-carboxylic acid instead of 6-chloro-1H-indazole-3-carboxylic acid.

¹H NMR (400 MHz, DMSO) δ 10.99 (s, 1H), 9.28 - 9.20 (m, 1H), 7.76 (d, J = 7.9 Hz, 1H), 7.70 (d, J = 7.8 Hz, 1H), 7.63 - 7.56 (m, 2H), 7.49 (m, 2H), 7.36 (t, J = 7.6 Hz, 1H), 5.11 (dd, J = 13.2, 5.0 Hz, 1H), 4.58 (d, J = 6.1 Hz, 2H), 4.45 (d, J = 17.3 Hz, 1H), 4.31 (d, J = 17.2 Hz, 1H), 2.98 - 2.82 (m, 1H), 2.55 (s, 4H), 2.38 (m, 1H), 2.00 (m, 1H).

### Compound 81. N-((2-(2,6-Dioxopiperidin-3-yl)-1-oxoisoindolin-5-yl)methyl)-5-methoxybenzofuran-2-carboxamide

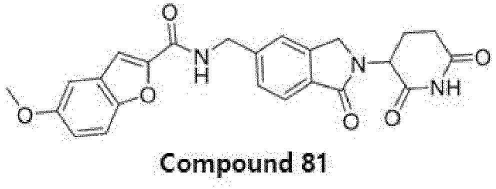

Compound 81 was synthesized in the same manner as in the synthesis method for Compound 1, with the exception of using 5-methoxybenzofuran-2-carboxylic acid instead of 6-chloro-1H-indazole-3-carboxylic acid.

¹H NMR (400 MHz, DMSO) δ 10.99 (s, 1H), 9.36 (m, 1H), 7.70 (d, J = 7.9 Hz, 1H), 7.60 - 7.44 (m, 4H), 7.28 (m, 1H), 7.06 (m, 1H), 5.11 (dd, J = 13.5, 4.9 Hz, 1H), 4.59 (d, J = 6.0 Hz, 2H), 4.45 (d, J = 17.4 Hz, 1H), 4.31 (d, J = 17.4 Hz, 1H), 3.81 (s, 3H), 2.98 - 2.84 (m, 1H), 2.60 (m, 1H), 2.38 (m, 1H), 2.06 - 1.95 (m, 1H).

### Compound 82. N-((2-(2,6-Dioxopiperidin-3-yl)-1-oxoisoindolin-5-yl)methyl)-7-methoxybenzofuran-2-carboxamide

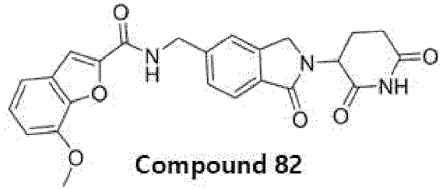

Compound 82 was synthesized in the same manner as in the synthesis method for Compound 1, with the exception of using 7-methoxybenzofuran-2-carboxylic acid instead of 6-chloro-1H-indazole-3-carboxylic acid.

¹H NMR (400 MHz, DMSO) δ 10.99 (s, 1H), 9.25 (m, 1H), 7.70 (d, J = 7.8 Hz, 1H), 7.65 (d, J = 8.7 Hz, 1H), 7.56 (s, 1H), 7.52 (s, 1H), 7.48 (d, J = 7.8 Hz, 1H), 7.21 (s, 1H), 6.98 (dd, J = 8.6, 2.1 Hz, 1H), 5.11 (dd, J = 13.2, 4.9 Hz, 1H), 4.59 (d, J = 6.0 Hz, 2H), 4.45 (d, J = 17.3 Hz, 1H), 4.31 (d, J = 17.3 Hz, 1H), 3.84 (s, 3H), 2.91 (m, 1H), 2.60 (m, 1H), 2.38 (m, 1H), 2.04 - 1.93 (m, 1H).

### Compound 83. N-((2-(2,6-Dioxopiperidin-3-yl)-1-oxoisoindolin-5-yl)methyl)-6-methoxybenzofuran-2-carboxamide

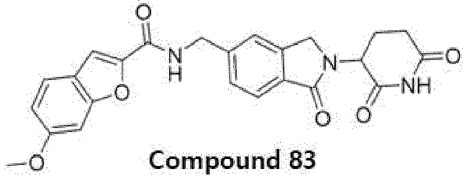

Compound 83 was synthesized in the same manner as in the synthesis method for Compound 1, with the exception of using 6-methoxybenzofuran-2-carboxylic acid instead of 6-chloro-1H-indazole-3-carboxylic acid.

¹H NMR (400 MHz, DMSO) δ 10.99 (s, 1H), 9.46 - 9.25 (m, 1H), 7.70 (d, J = 7.9 Hz, 1H), 7.57 (m, 2H), 7.49 (d, J = 7.9 Hz, 1H), 7.32 (d, J = 7.8 Hz, 1H), 7.26 (t, J = 7.9 Hz, 1H), 7.08 (d, J = 7.8 Hz, 1H), 5.11 (dd, J = 13.4, 4.9 Hz, 1H), 4.59 (d, J = 6.0 Hz, 2H), 4.45 (d, J = 17.4 Hz, 1H), 4.31 (d, J = 17.4 Hz, 1H), 3.97 (s, 3H), 3.02 - 2.82 (m, 1H), 2.60 (m, 1H), 2.36 (m, 1H), 2.00 (m, 1H).

### Compound 84. N-((2-(2,6-Dioxopiperidin-3-yl)-1-oxoisoindolin-5-yl)methyl)-3,6-dimethylbenzofuran-2-carboxamide

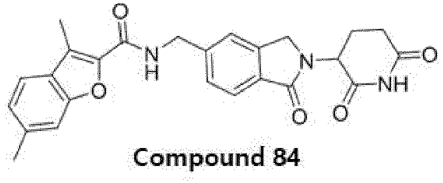

Compound 84 was synthesized in the same manner as in the synthesis method for Compound 1, with the exception of using 3,6-dimethylbenzofuran-2-carboxylic acid instead of 6-chloro-1H-indazole-3-carboxylic acid.

¹H NMR (400 MHz, DMSO) δ 10.99 (s, 1H), 9.17 (t, J = 6.2 Hz, 1H), 7.70 (d, J = 7.8 Hz, 1H), 7.62 (d, J = 8.0 Hz, 1H), 7.56 (s, 1H), 7.49 (d, J = 7.9 Hz, 1H), 7.39 (s, 1H), 7.18 (d, J = 8.0 Hz, 1H), 5.11 (dd, J = 13.3, 4.9 Hz, 1H), 4.57 (d, J = 6.1 Hz, 2H), 4.45 (d, J = 17.3 Hz, 1H), 4.31 (d, J = 17.4 Hz, 1H), 2.99 - 2.85 (m, 1H), 2.60 (m, 1H), 2.51 (s, 3H), 2.47 (s, 3H), 2.41 - 2.32 (m, 1H), 2.07 - 1.94 (m, 1H).

### Compound 85. 5-bromo-N-((2-(2,6-dioxopiperidin-3-yl)-1-oxoisoindolin-5-yl)methyl)-3-methylbenzofuran-2-carboxamide

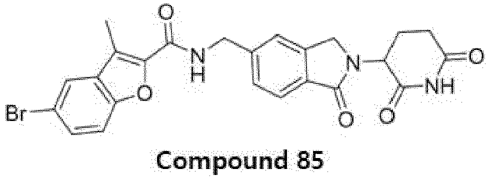

Compound 85 was synthesized in the same manner as in the synthesis method for Compound 1, with the exception of using 5-bromo-3-methylbenzofuran-2-carboxylic acid instead of 6-chloro-1H-indazole-3-carboxylic acid.

¹H NMR (400 MHz, DMSO) δ 10.99 (s, 1H), 9.38 - 9.27 (m, 1H), 8.01 (m, 1H), 7.70 (d, J = 7.8 Hz, 1H), 7.66 - 7.54 (m, 3H), 7.49 (d, J = 7.8 Hz, 1H), 5.11 (dd, J = 13.4, 4.9 Hz, 1H), 4.57 (d, J = 6.1 Hz, 2H), 4.45 (d, J = 17.2 Hz, 1H), 4.31 (d, J = 17.3 Hz, 1H), 2.90 (m, 1H), 2.60 (m, 1H), 2.51 (s, 3H), 2.37 (m, 1H), 2.10 - 1.94 (m, 1H).

### Compound 86. N-((2-(2,6-Dioxopiperidin-3-yl)-1-oxoisoindolin-5-yl)methyl)naphtho[2,1-b]furan-2-carboxamide

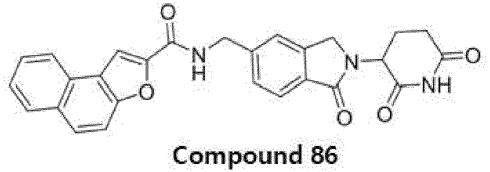

Compound 86 was synthesized in the same manner as in the synthesis method for Compound 1, with the exception of using naphtho[2,1-b]furan-2-carboxylic acid instead of 6-chloro-1H-indazole-3-carboxylic acid.

¹H NMR (400 MHz, DMSO) δ 10.99 (s, 1H), 9.45 (t, J = 6.2 Hz, 1H), 8.38 (d, J = 8.0 Hz, 1H), 8.27 (s, 1H), 8.09 (d, J = 8.1 Hz, 1H), 8.01 (d, J = 9.1 Hz, 1H), 7.84 (d, J = 9.1 Hz, 1H), 7.70 (m, 2H), 7.60 (m, 2H), 7.52 (d, J = 7.9 Hz, 1H), 5.11 (dd, J = 13.4, 4.9 Hz, 1H), 4.64 (d, J = 6.0 Hz, 2H), 4.46 (d, J = 17.3 Hz, 1H), 4.32 (d, J = 17.3 Hz, 1H), 2.98 - 2.83 (m, 1H), 2.60 (m, 1H), 2.42 - 2.29 (m, 1H), 2.06 - 1.92 (m, 1H).

### COMPARATIVE EXAMPLE 1. Synthesis and Physicochemical Characterization of Comparative Isoindolinone Compound Having Glutarimide Scaffold

Physicochemical properties of Comparative Compounds 1 to 3 are as follows.

### Comparative Compound 1. N-((2-(2,6-Dioxopiperidin-3-yl)-1-oxoisoindolin-5-yl)methyl)isoquinoline-3-carboxamide

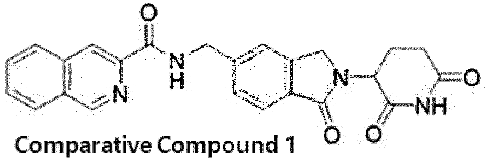

Comparative Compound 1 was synthesized in the same manner as in the synthesis method for Compound 1, with the exception of using isoquinoline-3-carboxylic acid instead of 6-chloro-1H-indazole-3-carboxylic acid.

¹H NMR (500 MHz, DMSO-d₆) δ 10.98 (s, 1H), 9.62 (t, J = 6.4 Hz, 1H), 9.41 (s, 1H), 8.59 (s, 1H), 8.27 (d, J = 8.1 Hz, 1H), 8.20 (d, J = 8.2 Hz, 1H), 7.89 (t, J = 7.5 Hz, 1H), 7.85 - 7.79 (m, 1H), 7.69 (d, J = 7.8 Hz, 1H), 7.58 (s, 1H), 7.51 (d, J = 7.9 Hz, 1H), 5.10 (dd, J = 13.3, 5.1 Hz, 1H), 4.68 (d, J = 6.4 Hz, 2H), 4.44 (d, J = 17.3 Hz, 1H), 4.30 (d, J = 17.3 Hz, 1H), 2.98 - 2.84 (m, 1H), 2.59 (m, 1H), 2.37 (m, 1H), 2.03 - 1.92 (m, 1H).

### Comparative Compound 2. N-((2-(2,6-Dioxopiperidin-3-yl)-1-oxoisoindolin-5-yl)methyl)quinoxaline-2-carboxamide

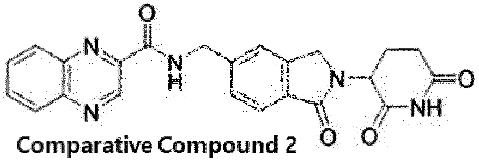

Comparative Compound 2 was synthesized in the same manner as in the synthesis method for Compound 1, with the exception of using quinoxaline-2-carboxylic acid instead of 6-chloro-1H-indazole-3-carboxylic acid.

¹H NMR (500 MHz, DMSO-d₆) δ 10.98 (s, 1H), 9.77 (t, J = 6.4 Hz, 1H), 9.50 (s, 1H), 8.22 (m, 2H), 8.04 - 7.98 (m, 2H), 7.70 (d, J = 7.8 Hz, 1H), 7.61 (s, 1H), 7.54 (d, J = 7.9 Hz, 1H), 5.10 (dd, J = 13.3, 5.1 Hz, 1H), 4.69 (d, J = 6.4 Hz, 2H), 4.44 (d, J = 17.4 Hz, 1H), 4.31 (d, J = 17.3 Hz, 1H), 2.91 (m, 1H), 2.59 (m, 1H), 2.43 - 2.32 (m, 1H), 2.06 - 1.95 (m, 1H).

### Comparative Compound 3. 6-Chloro-N-((2-(2,6-dioxopiperidin-3-yl)-1-oxoisoindolin-5-yl)methyl)quinoline-2-carboxamide

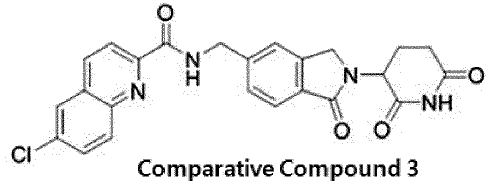

Comparative Compound 3 was synthesized in the same manner as in the synthesis method for Compound 1, with the exception of using 6-chloroquinoline-2-carboxylic acid instead of 6-chloro-1H-indazole-3-carboxylic acid.

¹H NMR (300 MHz, DMSO) δ 10.98 (s, 1H), 9.65 (t, J = 6.4 Hz, 1H), 8.57 (d, J = 8.5 Hz, 1H), 8.27 (d, J = 2.4 Hz, 1H), 8.23 (d, J = 8.6 Hz, 1H), 8.16 (d, J = 9.1 Hz, 1H), 7.90 (dd, J = 9.0, 2.4 Hz, 1H), 7.71 (d, J = 7.8 Hz, 1H), 7.60 (s, 1H), 7.53 (dd, J = 7.8, 1.4 Hz, 1H), 5.11 (dd, J = 13.2, 5.1 Hz, 1H), 4.69 (d, J = 6.3 Hz, 2H), 4.45 (d, J = 17.4 Hz, 1H), 4.31 (d, J = 17.4 Hz, 1H), 2.99 - 2.84 (m, 1H), 2.59 (m, 1H), 2.35 (m, 1H), 2.06 - 1.94 (m, 1H).

### EXPERIMENTAL EXAMPLE 1: Evaluation for Substrate Protein Degradation Activity of CRBN

Examination was made to determine whether the compounds of the present disclosure bind to cereblon (CRBN) and inhibit the function of CRBN. In this regard, the compounds were investigated for degradation activity for Ikaros (IKZF1) protein (Chamberlain, P. P. et al., 2014) or GSPT1 protein (Matyskiela, M. E. et al., 2016) which CRBN protein degrades when binding to thalidomide and analogs thereof.

In order to assay the compounds according to the present disclosure for degradation activity for Ikaros (IKZF1) protein or GSPT1 protein, experiments were carried out as follows.

KG-1 cells were seeded at a density of 5×10⁵ cells per well into 12-well plates, and each compound was added at predetermined concentration to the wells. After 6 hours, cell lysates were collected using TBSN buffer. The degradation activity of Ikaros protein was assessed using an antibody against Ikaros protein, and the degradation activity of GSPT1 protein was evaluated using an antibody against GSPT1 protein through Western blot analysis. In this regard, equal amounts of protein were loaded into each well of a 4-15% gradient gel, and after electrophoresis, proteins were transferred to a PVDF membrane. Each protein was then bound with primary antibodies, followed by the attachment of HRP-conjugated secondary antibodies, and developed using an HRP substrate.

As shown in FIG. 1, when applied to KG-1 cell lines for 6 hours, Compound 10 of the present disclosure was observed to selectively promote the degradation of GSPT1 protein.

### EXPERIMENTAL EXAMPLE 2: Cytotoxicity Assay

To evaluate the effect of the compounds of the present disclosure on cancer cells, a cytotoxicity assay was conducted as follows.

Cancer cells (KG-1) were seeded at a concentration of 10,000 cells per well into a 96-well plate. Then, the cells were treated with predetermined concentrations of compounds of the Examples and comparative substances (comparative compounds 1, 2, 3). After 72 hours, the cells were incubated with WST-1 reagent for one hour. Then, absorbance was measured at 450 nm using a spectramax spectrophotometer to assess the degree of cancer cell apoptosis. The IC₅₀ (µM) values were calculated using the measured values with the GraphPad Prism program and the measurements are summarized in Table 1.

The survival rate of NCI-H1155 (lung cancer cells) was measured using the CytoX cell viability assay kit (LPS solution, #CYT3000). NCI-H1155 cells were inoculated into 12-well plates. Afterwards, the cells were treated with each compound of the present disclosure and a comparative substance (CC-90009) at concentrations of 10 nM and 500 nM for 72 hours. Then, CytoX solution was added to the cells, incubated for one hour, and the absorbance of each well was read at 450 nm using a microplate reader to calculate the % viability, and the measurements are summarized in Table 2

**TABLE 1**

| **Compound No.** | **IC**₅₀ **(µM)** | **Compound No.** | **IC**₅₀ **(µM)** |
|---|---|---|---|
| | **KG-1** | | **KG-1** |
| Compound 1 | ++++ | Compound 28 | ++++ |
| Compound 2 | ++++ | Compound 29 | ++++ |
| Compound 4 | ++++ | Compound 30 | + |
| Compound 5 | ++++ | Compound 31 | + |
| Compound 6 | +++ | Compound 34 | +++ |
| Compound 7 | +++ | Compound 35 | + |
| Compound 8 | +++ | Compound 38 | ++ |
| Compound 9 | +++ | Compound 39 | ++++ |
| Compound 10 | ++++ | Compound 40 | ++++ |
| Compound 11 | ++++ | Compound 41 | ++++ |
| Compound 13 | +++ | Compound 42 | ++++ |
| Compound 14 | ++++ | Compound 43 | ++++ |
| Compound 15 | ++++ | Compound 44 | +++ |
| Compound 16 | +++ | Compound 45 | ++++ |
| Compound 17 | ++++ | Compound 46 | ++++ |
| Compound 18 | ++++ | Compound 52 | + |
| Compound 19 | ++++ | Compound 53 | + |
| Compound 20 | ++++ | Compound 54 | + |
| Compound 21 | ++++ | Compound 55 | ++ |
| Compound 23 | ++++ | Comparative Compound 1 | + |
| Compound 25 | ++++ | Comparative Compound 2 | + |
| Compound 26 | +++ | Comparative Compound 3 | + |
| Compound 27 | ++++ | 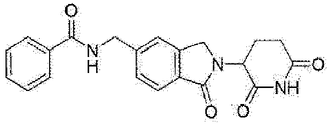 Comparative substance | + |
| 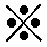 IC₅₀ value | | | |
| ++++: 0.0001∼0.01µM, +++: 0.01∼1µM, ++: 1∼10µM, +: 10∼50µM | | | |

**TABLE 2**

| **Compound No.** | **NCI-H1155 (% viability)** | |
|---|---|---|
| | **at 10 nM** | **at 500 nM** |
| Compound 56 | 29.82 | 5.94 |
| Compound 57 | 83.78 | 21.25 |
| Compound 58 | 4.31 | 2.96 |
| Compound 59 | 102.33 | 40.08 |
| Compound 60 | 82 | 22.27 |
| Compound 61 | 96.79 | 87.17 |
| Compound 62 | 18.95 | 9.56 |
| Compound 65 | 31.7 | 23.42 |
| Compound 66 | 32.13 | 26.11 |
| Compound 67 | 26.15 | 24.38 |
| Compound 68 | 29.87 | 26.41 |
| Compound 71 | 24.2 | 24.44 |
| Compound 72 | 33.03 | 24.55 |
| Compound 73 | 40.36 | 26.01 |
| Compound 74 | 26.56 | 25.36 |
| Compound 75 | 32.13 | 24.53 |
| Compound 76 | 28.02 | 26.33 |
| Compound 77 | 23.98 | 24.01 |
| Compound 78 | 15.67 | 15.64 |
| Compound 79 | 16.25 | 15.20 |
| Compound 82 | 38.98 | 17.61 |
| Compound 83 | 26.10 | 15.93 |
| Compound 84 | 28.85 | 18.23 |
| Compound 85 | 36.62 | 18.59 |
| Compound 86 | 14.68 | 14.65 |
| CC-90009 (Eragidomide) | 85.0 | 23.5 |

As shown in Tables 1 and 2, the heteroaryl amide structure-possessing compounds of the Examples of the present disclosure exhibited excellent cytotoxicity against cancer cells. Particularly, comparison of structure-activity between the compounds of the present disclosure revealed that more potent cytotoxic activity against KG-1 and NCI-H1155 cancer cells was obtained when R₁ is a bicyclic heteroaryl (Compounds 3, 7, and 10) compared to when R₁ is a monocyclic aryl (comparative substance) or heteroaryl (Compounds 37 and 38).

Among the compounds where R₁ is a (6+5) bicyclic heteroaryl, compounds with R₁ as indole, indazole, benzimidazole, benzoisoxazole, benzoxazole, benzothiazole, benzofuran, and benzothiophene (Compounds 3, 4, 6-10, 26, 33, 34, 36, 44, 56, 57, and 65-84) showed superior cytotoxic activity against KG-1 and cancer cells, compared to compounds with quinoline, isoquinoline, and quinoxaline, which are (6+6) bicyclic heteroaryls, (comparative compounds 1-3).

It was observed that the cytotoxic activity significantly improved when R₁ is a monocyclic heteroaryl with R2 existing as an aryl substituent. Among compounds with similar overall structures, compounds where R₁ is a monocyclic heteroaryl and R₂ exists as an aryl substituent (Compounds 2, 5, 11, 13-15, 17-21, 23, 25, 29, 32, 39, 40, 45, and 46) showed more superior cytotoxic activity against cancer cells compared to those without such substituents (Compounds 30 and 38).

Additionally, among compounds with similar overall structures, compounds with monocyclic heteroaryl for R₁ and aryl for R₂ (Compound 17, 23, 39, and 40) exhibited significantly more potent activity, compared to those with R₁ being a bicyclic heteroaryl (Compounds 8, 9, and 26).

Consistent with the data in Table 1, the data for H1155 cells (% viability) in Table 2 demonstrates that compounds with R₁ being a (6+6) bicyclic heteroaryl (Compounds 59, 60, and 61) have less cytotoxic activity against cancer cells, compared to those where R₁ is a (6+5) bicyclic heteroaryl (Compounds 56-58 and 62-70).

### <FORMULATION EXAMPLE 1. Preparation of Powders>

The powders were prepared by mixing 1.2 g of the compound of the present disclosure and 1 g of lactose and loading the mixture into an airtight bag.

### <FORMULATION EXAMPLE 2. Preparation of Tablet>

A tablet was prepared by mixing 100 mg of Compound 1 of the present disclosure, 100 mg of microcrystalline cellulose, 60 mg of lactose hydrate, 20 mg of low-substituted hydroxypropyl cellulose, and 2 mg of magnesium stearate, and tableting the mixture according to conventional methods for manufacturing tablets.

### <FORMULATION EXAMPLE 3. Preparation of Capsule>

A capsule was prepared by mixing 100 mg of Compound 1 of the present disclosure, 100 mg of microcrystalline cellulose, 60 mg of lactose hydrate, 20 mg of low-substituted hydroxypropyl cellulose, and 2 mg of magnesium stearate and loading the mixture into a gelatin capsule according to a common capsule preparation method.

### <FORMULATION EXAMPLE 4. Preparation of Pill>

Together with a small amount of anti-hygroscopic additives including dextrin, maltodextrin, corn starch, and microcrystalline cellulose (MCC), 90 mg of Compound 1 of the present disclosure, 5 mg of glutinous rice starch, and 5 mg of purified water were mixed and prepared into 100 mg of a pill according to a typical method.

### <FORMULATION EXAMPLE 5. Preparation of Injection>

10 mg of Compound 1 of the present disclosure was mixed with a suitable amount of sterile distilled water for injection, and a suitable amount of a pH adjuster and the mixture was put into an ample (2 ml) according to a common injection preparation method.

## Claims

1. A compound represented by the following Chemical Formula 1, an optical isomer thereof, or a pharmaceutically acceptable salt thereof: 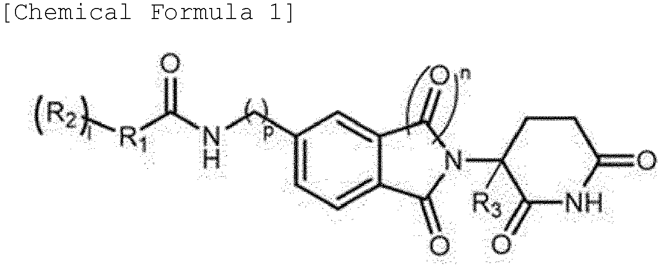 wherein,
l is an integer of 0 to 3;
n is an integer of 0 or 1;
p is an integer of 1 or 2;
R₁ is a C₅₋₁₃ heteroaryl, a C₆₋₁₀ aryl, a C₄₋₁₀ cycloalkyl, or a C₄₋₁₀ heterocycloalkyl;
R₂ is independently at least one substituent selected from the group consisting of a hydrogen atom, halogen, ketone, a C₁₋₅ alkyl, a C₁₋₃ alkoxy, a halo C₁₋₃ alkyl, a halo C₁₋₃ alkoxy, a C₃₋₆ cycloalkyl, a substituted or unsubstituted phenyl, and a substituted or unsubstituted benzyl,
the substituted phenyl or benzyl having as a substituent at least one selected from the group consisting of a halogen or a C₁₋₅ alkyl; and
R₃ is a hydrogen atom or a deuterium atom (D).

2. The compound, optical isomer, or pharmaceutically acceptable salt of claim 1 wherein, in Chemical Formula 1, R₁ is at least one substituent selected from the group consisting of pyrrolyl, pyrazolyl, imidazolyl, isoxazolyl, triazolyl, pyridinyl, pyrazinyl, pyrimidinyl, indolyl, indazolyl, benzoimidazolyl, benzofuranyl, benzopyridinyl, benzopyranyl, benzopyridazinyl, benzoisoxazolyl, benzoxazolyl, benzothiazolyl, benzothiophenyl, and naphthofuranyl.

3. The compound, optical isomer, or pharmaceutically acceptable salt of claim 1 wherein, in Chemical Formula 1,
R₁ is C₅₋₁₃ heteroaryl;
R₂ is at least one substituent selected from the group consisting of a substituted or unsubstituted phenyl and a substituted or unsubstituted benzyl,
the substituted phenyl or benzyl having as a substituent at least one selected from the group consisting of a halogen or a C₁₋₅ alkyl.

4. The compound, optical isomer, or pharmaceutically acceptable salt of claim 3 wherein, in Chemical Formula 1,
R₂ is a substituent selected from the group consisting of pyrrolyl, pyrazolyl, imidazolyl, isoxazolyl, triazolyl, pyridinyl, pyrazinyl, pyrimidinyl, indolyl, indazolyl, benzoimidazolyl, benzofuranyl, benzopyridinyl, benzopyranyl, benzopyridazinyl, benzoisoxazolyl, benzoxazolyl, benzothiazolyl, benzothiophenyl, and naphthofuranyl.

5. The compound, optical isomer, or pharmaceutically acceptable salt of claim 1 or 2, wherein the compound of Chemical Formula 1 is selected from the group consisting of:
6-chloro-N-((2-(2,6-dioxopiperidin-3-yl)-1-oxoisoindolin-5-yl)methyl)-1H-indazole-3-carboxamide (Compound 1);
3-(4-chlorophenyl)-N-((2-(2,6-dioxopiperidin-3-yl)-1-oxoisoindolin-5-yl)methyl)-1H-pyrazole-5-carboxamide (Compound 2);
5-chloro-N-((2-(2,6-dioxopiperidin-3-yl)-1-oxoisoindolin-5-yl)methyl)-1H-benzo[d]imidazole-2-carboxamide (Compound 3);
5,6-dichloro-N-((2-(2,6-dioxopiperidin-3-yl)-1-oxoisoindolin-5-yl)methyl)-1H-indazole-3-carboxamide (Compound 4);
3-(3-chlorophenyl)-N-((2-(2,6-dioxopiperidin-3-yl)-1-oxoisoindolin-5-yl)methyl)-1H-pyrazole-5-carboxamide (Compound 5);
6-chloro-N-((2-(2,6-dioxopiperidin-3-yl)-1-oxoisoindolin-5-yl)methyl)-1H-indole-3-carboxamide (Compound 6);
5-chloro-N-((2-(2,6-dioxopiperidin-3-yl)-1-oxoisoindolin-5-yl)methyl)-1H-indole-3-carboxamide (Compound 7);
5-chloro-N-((2-(2,6-dioxopiperidin-3-yl)-1-oxoisoindolin-5-yl)methyl)-1H-indazole-3-carboxamide (Compound 8);
6-chloro-N-((2-(2,6-dioxopiperidin-3-yl)-1-oxoisoindolin-5-yl)methyl)-1H-indole-2-carboxamide (Compound 9);
6-chloro-N-((2-(2,6-dioxopiperidin-3-yl)-1-oxoisoindolin-5-yl)methyl)benzo[d]isoxazole-3-carboxamide (Compound 10);
5-(4-chlorophenyl)-N-((2-(2,6-dioxopiperidin-3-yl)-1-oxoisoindolin-5-yl)methyl)isoxazole-3-carboxamide (Compound 11);
N-((2-(2,6-dioxopiperidin-3-yl)-1-oxoisoindolin-5-yl)methyl)-4-phenylpicolinamide (Compound 12);
3-(3,4-dichlorophenyl)-N-((2-(2,6-dioxopiperidin-3-yl)-1-oxoisoindolin-5-yl)methyl)-1H-pyrazole-5-carboxamide (Compound 13);
4-(3-chlorophenyl)-N-((2-(2,6-dioxopiperidin-3-yl)-1-oxoisoindolin-5-yl)methyl)-1H-pyrrole-2-carboxamide (Compound 14);
1-(3-chlorophenyl)-N-((2-(2,6-dioxopiperidin-3-yl)-1-oxoisoindolin-5-yl)methyl)-1H-1,2,3-triazole-4-carboxamide (Compound 15);
5,6-dichloro-N-((2-(2,6-dioxopiperidin-3-yl)-1-oxoisoindolin-5-yl)methyl)-1H-benzo[d]imidazole-2-carboxamide (Compound 16);
N-((2-(2,6-dioxopiperidin-3-yl)-1-oxoisoindolin-5-yl)methyl)-4-methyl-5-phenyl-1H-pyrazole-3-carboxamide (Compound 17);
4-(4-chlorophenyl)-N-((2-(2,6-dioxopiperidin-3-yl)-1-oxoisoindolin-5-yl)methyl)picolinamide (Compound 18);
5-(3-chlorophenyl)-N-((2-(2,6-dioxopiperidin-3-yl)-1-oxoisoindolin-5-yl)methyl)isoxazole-3-carboxamide (Compound 19);
5-(3-chlorophenyl)-N-((2-(2,6-dioxopiperidin-3-yl)-1-oxoisoindolin-5-yl)methyl)pyrimidine-2-carboxamide (Compound 20);
1-benzyl-5-(3-chlorophenyl)-N-((2-(2,6-dioxopiperidin-3-yl)-1-oxoisoindolin-5-yl)methyl)-4-methyl-1H-pyrazole-3-carboxamide (Compound 21);
4-(3-chlorophenyl)-N-((2-(2,6-dioxopiperidin-3-yl)-1-oxoisoindolin-5-yl)methyl)picolinamide (Compound 22);
N-((2-(2,6-dioxopiperidin-3-yl)-1-oxoisoindolin-5-yl)methyl)-1-methyl-5-phenyl-1H-pyrazole-3-carboxamide (Compound 23);
N-((2-(2,6-dioxopiperidin-3-yl)-1-oxoisoindolin-5-yl)methyl)-1-methyl-3-phenyl-1H-pyrazole-5-carboxamide (Compound 24);
5-(2,4-dichlorophenyl)-N-((2-(2,6-dioxopiperidin-3-yl)-1-oxoisoindolin-5-yl)methyl)-1H-pyrazole-3-carboxamide (Compound 25);
6-chloro-N-((2-(2,6-dioxopiperidin-3-yl)-1-oxoisoindolin-5-yl)methyl)benzo[d]thiazole-2-carboxamide (Compound 2 6);
4-bromo-3-(3-chlorophenyl)-N-((2-(2,6-dioxopiperidin-3-yl)-1-oxoisoindolin-5-yl)methyl)-1H-pyrazole-5-carboxamide (Compound 27);
5-(2,5-dichlorophenyl)-N-((2-(2,6-dioxopiperidin-3-yl)-1-oxoisoindolin-5-yl)methyl)-1H-pyrazole-3-carboxamide (Compound 28);
5-(2,4-dimethylphenyl)-N-((2-(2,6-dioxopiperidin-3-yl)-1-oxoisoindolin-5-yl)methyl)-1H-pyrazole-3-carboxamide (Compound 29);
N-((2-(2,6-dioxopiperidin-3-yl)-1-oxoisoindolin-5-yl)methyl)-[1,1'-biphenyl]-3-carboxamide (Compound 30);
N-((2-(2,6-dioxopiperidin-3-yl)-1-oxoisoindolin-5-yl)methyl)-4-methylpicolinamide (Compound 31);
N-((2-(2,6-dioxopiperidin-3-yl)-1-oxoisoindolin-5-yl)methyl)-4-phenylpicolinamide (Compound 32);
6-chloro-N-((2-(2,6-dioxopiperidin-3-yl)-1-oxoisoindolin-5-yl)methyl)benzo[d]oxazole-2-carboxamide (Compound 33);
N-((2-(2,6-dioxopiperidin-3-yl)-1-oxoisoindolin-5-yl)methyl)-1H-indazole-3-carboxamide (Compound 34);
3-bromo-N-((2-(2,6-dioxopiperidin-3-yl)-1-oxoisoindolin-5-yl)methyl)-1-methyl-1H-pyrazole-5-carboxamide (Compound 35);
N-((2-(2,6-dioxopiperidin-3-yl)-1-oxoisoindolin-5-yl)methyl)-1-methyl-1H-indazole-3-carboxamide (Compound 36);
4-bromo-N-((2-(2,6-dioxopiperidin-3-yl)-1-oxoisoindolin-5-yl)methyl)-1-methyl-1H-imidazole-2-carboxamide (Compound 37);
4-bromo-N-((2-(2,6-dioxopiperidin-3-yl)-1-oxoisoindolin-5-yl)methyl)-1-methyl-1H-pyrrole-2-carboxamide (Compound 38);
N-((2-(2,6-dioxopiperidin-3-yl)-1-oxoisoindolin-5-yl)methyl)-1-methyl-4-phenyl-1H-pyrrole-2-carboxamide (Compound 39);
4-(3-chlorophenyl)-N-((2-(2,6-dioxopiperidin-3-yl)-1-oxoisoindolin-5-yl)methyl)-1-methyl-1H-pyrrole-2-carboxamide (Compound 40);
5-(3-chlorophenyl)-N-((2-(2,6-dioxopiperidin-3-yl)-1-oxoisoindolin-5-yl)methyl)-1-isobutyl-4-methyl-1H-pyrazole-3 -carboxamide (Compound 41);
5-(3-chlorophenyl)-N-((2-(2,6-dioxopiperidin-3-yl)-1-oxoisoindolin-5-yl)methyl)-4-methyl-1-phenyl-1H-pyrazole-3-carboxamide (Compound 42);
5-(3-chlorophenyl)-N-((2-(2,6-dioxopiperidin-3-yl)-1-oxoisoindolin-5-yl)methyl)-1-(4-fluorophenyl)-4-methyl-1H-pyrazole-3-carboxamide (Compound 43);
5-chloro-N-((2-(2,6-dioxopiperidin-3-yl)-1-oxoisoindolin-5-yl)methyl)benzo[d]thiazole-2-carboxamide (Compound 44);
N-((2-(2,6-dioxopiperidin-3-yl)-1-oxoisoindolin-5-yl)methyl)-5-methyl-4-phenylpicolinamide (Compound 45);
5-chloro-N-((2-(2,6-dioxopiperidin-3-yl)-1-oxoisoindolin-5-yl)methyl)-4-phenylpicolinamide (Compound 46);
N-((2-(2,6-dioxopiperidin-3-yl)-1-oxoisoindolin-5-yl)methyl)-6-phenylpyrazine-2-carboxamide (Compound 47);
3-(2-chloro-6-fluorophenyl)-N-((2-(2,6-dioxopiperidin-3-yl)-1-oxoisoindolin-5-yl)methyl)-5-methylisoxazole-4-carboxamide (Compound 48);
N-((2-(2,6-dioxopiperidin-3-yl)-1-oxoisoindolin-5-yl)methyl)-3-phenyl-1H-pyrazole-5-carboxamide (Compound 49);
N-((2-(2,6-dioxopiperidin-3-yl)-1-oxoisoindolin-5-yl)methyl)-1-phenyl-5-(trifluoromethyl)-1H-pyrazole-4-carboxamide (Compound 50);
N-((2-(2,6-dioxopiperidin-3-yl)-1-oxoisoindolin-5-yl)methyl)-[1,1'-biphenyl]-4-carboxamide (Compound 51);
N-((2-(2,6-dioxopiperidin-3-yl)-1-oxoisoindolin-5-yl)methyl)-[1,1'-biphenyl]-2-carboxamide (Compound 52);
1-(4-chlorophenyl)-N-((2-(2,6-dioxopiperidin-3-yl)-1-oxoisoindolin-5-yl)methyl)cyclopentane-1-carboxamide (Compound 53);
N-((2-(2,6-dioxopiperidin-3-yl)-1-oxoisoindolin-5-yl)methyl)-1-phenylcyclopentane-1-carboxamide (Compound 54);
N-((2-(2,6-dioxopiperidin-3-yl)-1-oxoisoindolin-5-yl)methyl)-2-oxo-1-phenylpyrrolidine-3-carboxamide (Compound 55);
N-((2-(2,6-dioxopiperidin-3-yl)-1-oxoisoindolin-5-yl)methyl)benzofuran-2-carboxamide (Compound 56);
N-((2-(2,6-dioxopiperidin-3-yl)-1-oxoisoindolin-5-yl)methyl)-5-methoxy-1H-indole-2-carboxamide (Compound 57);
N-((2-(2,6-dioxopiperidin-3-yl)-1-oxoisoindolin-5-yl)methyl)benzo[b]thiophene-2-carboxamide (Compound 58);
7-chloro-N-((2-(2,6-dioxopiperidin-3-yl)-1-oxoisoindolin-5-yl)methyl)-4-hydroxyquinoline-3-carboxamide (Compound 59);
N-((2-(2,6-dioxopiperidin-3-yl)-1-oxoisoindolin-5-yl)methyl)-2-oxo-2H-cromene-3-carboxamide (Compound 60);
N-((2-(2,6-dioxopiperidin-3-yl)-1-oxoisoindolin-5-yl)methyl)cinnoline-4-carboxamide (Compound 61);
5-(4-chlorophenyl)-N-((2-(2,6-dioxopiperidin-3-yl)-1-oxoisoindolin-5-yl)methyl)-1-isobutyl-4-methyl-1H-pyrazole-3 -carboxamide (Compound 62);
5-(3-chlorophenyl)-1-cyclopropyl-N-((2-(2,6-dioxopiperidin-3-yl)-1-oxoisoindolin-5-yl)methyl)-4-methyl-1H-pyrazole-3 -carboxamide (Compound 63);
5-(3-chlorophenyl)-N-((2-(2,6-dioxopiperidin-3-yl)-1-oxoisoindolin-5-yl)methyl)-4-methyl-1-(2,2,2-trifluoroethyl)-1H-pyrazole-3-carboxamide (Compound 64);
N-((2-(2,6-dioxopiperidin-3-yl)-1-oxoisoindolin-5-yl)methyl)-5-methoxybenzo[b]thiophene-2-carboxamide (Compound 65);
N-((2-(2,6-dioxopiperidin-3-yl)-1-oxoisoindolin-5-yl)methyl)-6-methylbenzo[b]thiophene-2-carboxamide (Compound 66);
3-chloro-N-((2-(2,6-dioxopiperidin-3-yl)-1-oxoisoindolin-5-yl)methyl)-6-methylbenzo[b]thiophene-2-carboxamide (Compound 67);
3-chloro-N-((2-(2,6-dioxopiperidin-3-yl)-1-oxoisoindolin-5-yl)methyl)-6-methoxybenzo[b]thiophene-2-carboxamide (Compound 68);
N-((2-(2,6-dioxopiperidin-3-yl)-1-oxoisoindolin-5-yl)methyl)-5,6-dimethoxybenzo[b]thiophene-2-carboxamide (Compound 69);
N-((2-(2,6-dioxopiperidin-3-yl)-1-oxoisoindolin-5-yl)methyl)-4-(trifluoromethyl)benzo[b]thiophene-2-carboxamide (Compound 70);
N-((2-(2,6-dioxopiperidin-3-yl)-1-oxoisoindolin-5-yl)methyl)-7-(trifluoromethyl)benzo[b]thiophene-2-carboxamide (Compound 71);
3-chloro-N-((2-(2,6-dioxopiperidin-3-yl)-1-oxoisoindolin-5-yl)methyl)-6-(trifluoromethyl)benzo[b]thiophene-2-carboxamide (Compound 72);
N-((2-(2,6-dioxopiperidin-3-yl)-1-oxoisoindolin-5-yl)methyl)-5-(trifluoromethyl)benzo[b]thiophene-2-carboxamide (Compound 73);
N-((2-(2,6-dioxopiperidin-3-yl)-1-oxoisoindolin-5-yl)methyl)-4-methoxybenzo[b]thiophene-2-carboxamide (Compound 74);
5-chloro-N-((2-(2,6-dioxopiperidin-3-yl)-1-oxoisoindolin-5-yl)methyl)-3-methylbenzo[b]thiophene-2-carboxamide (Compound 75);
N-((2-(2,6-dioxopiperidin-3-yl)-1-oxoisoindolin-5-yl)methyl)-3-methylbenzo[b]thiophene-2-carboxamide (Compound 76);
4-chloro-N-((2-(2,6-dioxopiperidin-3-yl)-1-oxoisoindolin-5-yl)methyl)-3-methylbenzo[b]thiophene-2-carboxamide (Compound 77);
7-chloro-N-((2-(2,6-dioxopiperidin-3-yl)-1-oxoisoindolin-5-yl)methyl)-3-methylbenzo[b]thiophene-2-carboxamide (Compound 78);
N-((2-(2,6-dioxopiperidin-3-yl)-1-oxoisoindolin-5-yl)methyl)-4-fluoro-3-methylbenzo[b]thiophene-2-carboxamide (Compound 79);
N-((2-(2,6-dioxopiperidin-3-yl)-1-oxoisoindolin-5-yl)methyl)-3-methylbenzofuran-2-carboxamide (Compound 80) ;
N-((2-(2,6-dioxopiperidin-3-yl)-1-oxoisoindolin-5-yl)methyl)-5-methoxybenzofuran-2-carboxamide (Compound 81) ;
N-((2-(2,6-dioxopiperidin-3-yl)-1-oxoisoindolin-5-yl)methyl)-7-methoxybenzofuran-2-carboxamide (Compound 82) ;
N-((2-(2,6-dioxopiperidin-3-yl)-1-oxoisoindolin-5-yl)methyl)-6-methoxybenzofuran-2-carboxamide (Compound 83) ;
N-((2-(2,6-dioxopiperidin-3-yl)-1-oxoisoindolin-5-yl)methyl)-3,6-dimethylbenzofuran-2-carboxamide (Compound 84);
5-bromo-N-((2-(2,6-dioxopiperidin-3-yl)-1-oxoisoindolin-5-yl)methyl)-3-methylbenzofuran-2-carboxamide (Compound 85); and
N-((2-(2,6-dioxopiperidin-3-yl)-1-oxoisoindolin-5-yl)methyl)naphtho[2,1-b]furan-2-carboxamide (Compound 86) .

6. A pharmaceutical composition comprising the compound represented by Chemical Formula 1 or pharmaceutically acceptable salt of claim 1 or 2 as an active ingredient for prevention or treatment of leprosy, chronic graft-versus-host disease, inflammatory diseases, or cancer.

7. The pharmaceutical composition of claim 6, wherein the cancer is selected from the group consisting of breast cancer, colon cancer, lung cancer, small cell lung cancer, stomach cancer, liver cancer, blood cancer, bone cancer, pancreatic cancer, skin cancer, head or neck cancer, cutaneous or intraocular melanoma, uterine cancer, ovarian cancer, rectal cancer, perianal cancer, colorectal cancer, breast cancer, fallopian tube carcinoma, endometrial carcinoma, cervical cancer, vaginal cancer, vulvar carcinoma, Hodgkin's disease, esophageal cancer, small intestine cancer, endocrine gland cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvis carcinoma, CNS tumors, primary CNS lymphoma, spinal tumors, brainstem gliomas, and pituitary adenomas.
